# EUROPEAN PATENT SPECIFICATION

(11) **EP 1 633 714 B1**
(45) Date of publication and mention of the grant of the patent: **23.01.2008**
(21) Application number: 04734320.7
(22) Date of filing: 21.05.2004
(51) Int. Cl.: C07D 213/73, A61K 31/44, A61P 11/00

(54) **2-AMINO-PYRIDINE DERIVATIVES AS BETA-2 ADRENORECEPTOR AGONISTS**
2-AMINO-PYRIDIN-DERIVATE ALS BETA-2 ADRENORECEPTOR AGONISTEN
DERIVES DE 2-AMINO-PYRIDINE UTILES COMME AGONISTES DE RECEPTEURS BETA-2 ADRENERGIQUES

(30) Priority: 04.06.2003 GB 0312840; 04.06.2003 GB 0312842
(43) Date of publication of application: 15.03.2006
(73) Proprietor: Pfizer Limited, Sandwich, Kent CT13 9NJ (GB); PFIZER INC., New York, N.Y. 10017 (US)
(72) Inventor: Brown, Alan Daniel, Sandwich, Kent CT13 9NJ (GB); Bryans, Justin Stephen, Sandwich, Kent CT13 9NJ (GB); Lane, Charlotte Alice Louise, Sandwich, Kent CT13 9NJ (GB); Mantell, Simon John, Sandwich, Kent CT13 9NJ (GB)
(74) Representative: Pringot, Thomas
(86) International application number: PCT/IB2004/001735
(87) International publication number: WO 2004/108675

(56) References cited:
- WO-A-03/024439
- US-A- 5 541 197
- US-A- 5 714 506

## Description

This invention relates to β2 agonists of general formula (1): 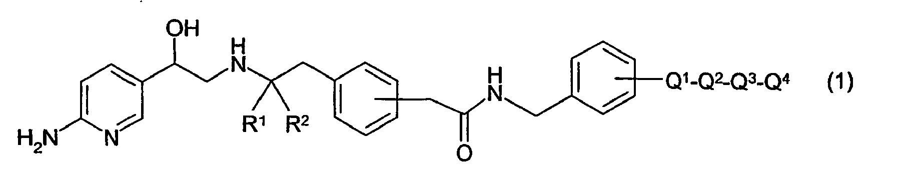 in which R¹, R², Q¹, Q², Q³ and Q⁴ have the meanings indicated below,
and to processes for the preparation of, intermediates used in the preparation of, compositions containing and the uses of, such derivatives.

Adrenoceptors are members of the large G-protein coupled receptor super-family. The adrenoceptor subfamily is itself divided into the α and β subfamilies with the β sub-family being composed of at least 3 receptor subtypes: β1, β2 and β3. These receptors exhibit differential expression patterns in tissues of various systems and organs of mammals. β2 adrenergic (β2) receptors are mainly expressed in smooth muscle cells (e.g. vascular, bronchial, uterine or intestinal smooth muscles), whereas β3 adrenergic receptors are mainly expressed in fat tissues (therefore β3 agonists could potentially be useful in the treatment of obesity and diabetes) and β1 adrenergic receptors are mainly expressed in cardiac tissues (therefore β1 agonists are mainly used as cardiac stimulants).

The pathophysiology and treatments of airway diseases have been extensively reviewed in the literature (for reference see Barnes, P.J. Chest, 1997, 111:2, pp 17S-26S and Bryan, S.A. et al, Expert Opinion on investigational drugs, 2000, 9:1, pp25-42) and therefore only a brief summary will be included here to provide some background information.

Glucocorticosteroids, anti-leukotrienes, theophylline, cromones, anticholinergics and β2 agonists constitute drug classes that are currently used to treat allergic and non-allergic airways diseases such as asthma and chronic obstructive airways disease (COPD). Treatment guidelines for these diseases include both short and long acting inhaled β2 agonists. Short acting, rapid onset β2 agonists are used for "rescue" bronchodilation, whereas, long-acting forms provide sustained relief and are used as maintenance therapy.

Bronchodilation is mediated via agonism of the β2 adrenoceptor expressed on airway smooth muscle cells, which results in relaxation and hence bronchodilation. Thus, as functional antagonists, β2 agonists can prevent and reverse the effects of all bronchoconstrictor substances, including leukotriene D4 (LTD4), acetylcholine, bradykinin, prostaglandins, histamine and endothelins. Because β2 receptors are so widely distributed in the airway, β2 agonists may also affect other types of cells that play a role in asthma. For example, it has been reported that β2 agonists may stabilize mast cells. The inhibition of the release of bronchoconstrictor substances may be how β2 agonists block the bronchoconstriction induced by allergens, exercise and cold air. Furthermore, β2 agonists inhibit cholinergic neurotransmission in the human airway, which can result in reduced cholinergic-reflex bronchoconstriction.

In addition to the airways, it has also been established that β2 adrenoceptors are also expressed in other organs and tissues and thus β2 agonists, such as those described in the present invention, may have application in the treatment of other diseases such as, but not limited to those of the nervous system, premature labor, congestive heart failure, depression, inflammatory and allergic skin diseases, psoriasis, proliferative skin diseases, glaucoma and in conditions where there is an advantage in lowering gastric acidity, particularly in gastric and peptic ulceration.

However, numerous β2 agonists are limited in their use due to their low selectivity or adverse side-effects driven by high systemic exposure and mainly mediated through action at β2 adrenoreceptors expressed outside the airways (muscle tremor, tachycardia, palpitations, restlessness). Therefore there is a need for improved agents in this class.

Accordingly, there is still a need for novel β2 agonists that would have an appropriate pharmacological profile, for example in terms of potency, selectivity, duration of action and/or pharmacodynamic properties. In this context, the present invention relates to novel β2 agonists.

Various 2-amino-pyridine derivatives have already been synthesised. For example, the US patent US 4,358,455 discloses compounds having a random activity either as beta-adrenergic stimulants or as beta-adrenergic blockers, of formula : 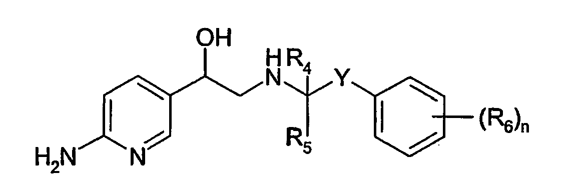 wherein R₄ and R₅ are independently selected from H and (C₁-C₃)alkyl; Y may namely be a methylene group, R₆ is selected from H, OH, alkoxy, methylenedioxy, halo or alkyl; and n is equal to 1 or 2.

Another example concerns the patent application WO 95/29259 that discloses selective β3 agonists (with little β1 and β2 activity) of formula : 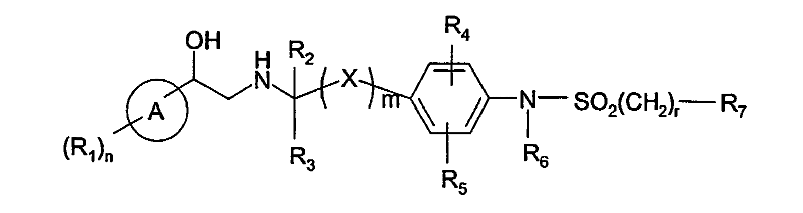 wherein A may be a 6-membered heterocyclic ring with from 1 to 4 heteroatoms selected from O, S and N; R₁ may be amino and n may be equal to 1; R₂, R₃ may be independently H or (C₁-C₁₀)alkyl; m is 0 or 1, and X may be a methylene group; R₄, R₅ may be H; R₆ is H or (C₁-C₁₀)alkyl; r is 0 to 3 and R₇ may be a phenyl 0 to 5 times substituted by numerous substituents (OH, oxo, Hal, CN etc...).

Other 2-amino-pyridine derivatives are also disclosed in US 5,714,506 as selective β3 agonists They are more specifically of formula : 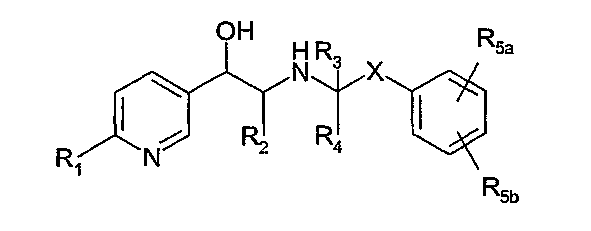 wherein R₁ may be amino; R₂ is H or (C₁-C₆)alkyl; R₃ and R₄ may be independently H or (C₁-C₁₂)alkyl; X may be a -(CH₂)ₙ- group with n selected from 1, 2 and 3; and R₅ₐ and R_{5b} may be chosen from -CONR₂R₂, -O-CH₂-CONR₂R₂, aryl, -CH₂-alkoxy, -CH₂-CONR₂R₂ wherein R₂ is H or (C₁-C₆)alkyl.

However, none of the 2-amino-pyridine derivatives synthesised so far have shown a selective β2 agonist activity, allowing them to be used as efficient drugs in the treatment of the β2-mediated diseases and/or conditions, in particular allergic and non-allergic airways diseases or other diseases such as those previously cited.

The invention relates to the compounds of general formula (1): 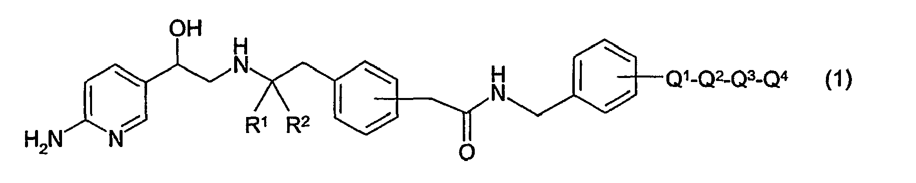 wherein the CH₂-C(=O)NH-benzyl-Q¹-Q²-Q³-Q⁴ group is in the meta or para position, and
- R¹ and R² are independently selected from H and C₁-C₄ alkyl;
- Q¹ is (CH₂)ₙ wherein n is an integer selected from 0 and 1;
- Q² is a group selected from NH, -C(=O)NH-, -NHC(=O)-, NH-C(O)-NH and-SO₂NH-;
- Q³ is a single bond, a C₁-C₄ alkylene optionally substituted with OH or a group (CH₂)ₘ-O-(CH₂)ₚ wherein m and p are integers independently selected from 1, 2 or 3;
- Q⁴ is selected from 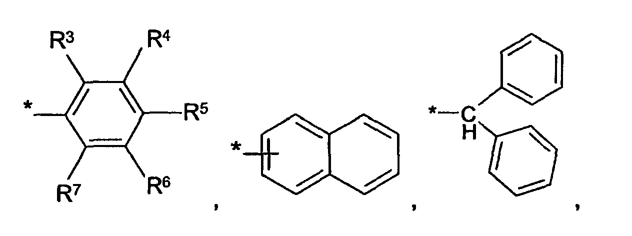
wherein * represents the attachment point to Q³ and R³, R⁴, R⁵, R⁶ and R⁷ are independently selected from H, phenoxy, C₁-C₄ alkyl, OR⁹, SR⁹, halo, CF₃, OCF₃, COOR⁹, SO₂NR⁸R⁹, CONR⁸R⁹, NHR⁸R⁹, NHCOR⁹, CH₂-NHC(=O)NH-R⁹ provided at least 2 of R³ to R⁷ are equal to H;
wherein R⁸ is selected from H or C₁-C₄ alkyl and R⁹ is selected from H, C₁-C₄ alkyl, benzyl optionally substituted with 1, 2, 3 or 4 C₁-C₄ alkoxy, and a group 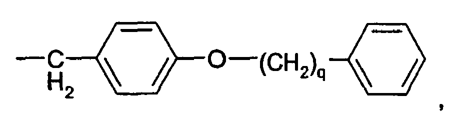 wherein q is an integer selected from 0, 1, 2 and 3;
or, if appropriate, their pharmaceutically acceptable salts and/or isomers, tautomers, solvates or isotopic variations thereof.

The compounds of formula (1) are agonists of the β2 receptors, that are particularly useful for the treatment of β2-mediated diseases and/or conditions, and show good potency, in particular when administered via the inhalation route.

Preferably, the term "selective" means that the compounds of formula (1) show an agonist potency for the β2 receptor, which is at least about 100-fold higher as for the β3 receptor and at least about 500-fold higher as for the β1 receptor.

Preferably, the compounds of formula (1) show an agonist potency for the β2 receptor, which is less than 10 nM as measured by the cell-based assay described herein.

In the here above general formula (1), C₁-C₄ alkyl and C₁-C₄ alkylene denote a straight-chain or branched group containing 1, 2, 3 or 4 carbon atoms. This also applies if they carry substituents or occur as substituents of other radicals, for example in O-(C₁-C₄)alkyl radicals, S-(C₁-C₄)alkyl radicals etc.... Examples of suitable (C₁-C₄)alkyl radicals are methyl, ethyl, *n*-propyl, *iso*-propyl, *n*-butyl, *iso*-butyl, *sec*-butyl, *tert*-butyl.... Examples of suitable (C₁-C₄)alkoxy radicals are methoxy, ethoxy, *n*-propyloxy, *iso*-propyloxy, *n*-butyloxy, *iso*-butyloxy, *sec*-butyloxy and *tert*-butyloxy....

Finally, halo denotes a halogen atom selected from the group consisting of fluoro, chloro, bromo and iodo in particular fluoro or chloro.

The 2-amino-pyridine derivatives of the formula (1) can be prepared using conventional procedures such as by the following illustrative methods in which R¹, R², Q¹, Q², Q³ and Q⁴ are as previously defined for the 2-amino-pyridine derivatives of the formula (1) unless otherwise stated.

The 2-amino-pyridine derivatives of the formula (1) may be prepared by removal of the protecting group(s) "Prot" from the compound of formula (2): 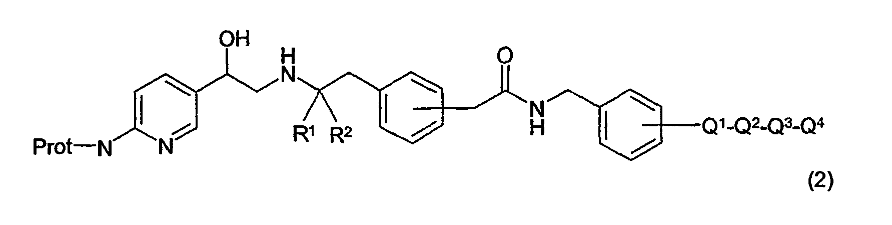 wherein R¹, R², Q¹, Q², Q³ and Q⁴ are as previously described for the 2-amino-pyridine derivatives of formula (1) and Prot is a suitable protecting group (or 2 suitable protecting groups) for the amino-pyridine, which include but is not limited to *tert*-butoxycarbonyl, acyl or 2,5-dimethyl pyrrole,
by methods well known to those skilled in the art such as standard methodology for cleaving nitrogen protecting groups as found in textbooks (e.g. T.W. GREENE, Protective Groups in Organic Synthesis, A. Wiley-Interscience Publication, 1981).

The compound of formula (2) may be prepared by coupling an acid of formula (3) : 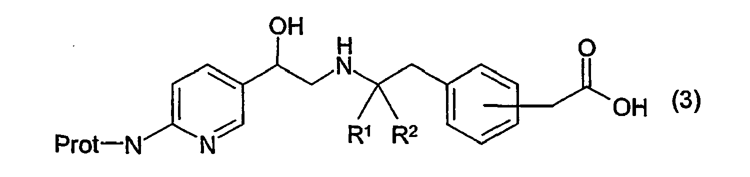 wherein the CH₂-C(=O)OH is in the meta or para position and Prot, R¹ and R² are as previously defined,
with an amine of formula (4) : 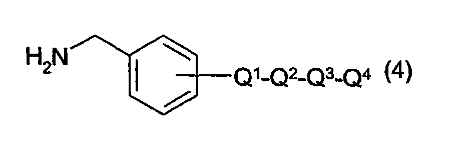 wherein Q¹, Q², Q³ and Q⁴ are as previously defined.

The coupling of the acid (3) to the amine (4) is generally carried out in an excess of said amine, with a conventional coupling agent (e.g. 1-(3-dimethylaminopropyl)-3-ethylcarbodiimide hydrochloride or *N,N'-*dicyclohexylcarbodiimide), optionally in the presence of a catalyst (e.g. 1-hydroxybenzotriazole hydrate or 1-hydroxy-7-azabenzotriazole), and optionally in the presence of a tertiary amine base (e.g. *N*-methylmorpholine, triethylamine or *N,N-*diisopropylethylamine). The reaction may be undertaken in a suitable solvent such as pyridine, *N,N*-dimethylformamide, tetrahydrofuran, dimethylsulfoxide, dichloromethane or ethyl acetate, and at temperature comprised between 10°C and 40°C (room temperature).

The amine (4) wherein Q² is -NHC(=O)NH- may be prepared according to scheme 1 as follows : 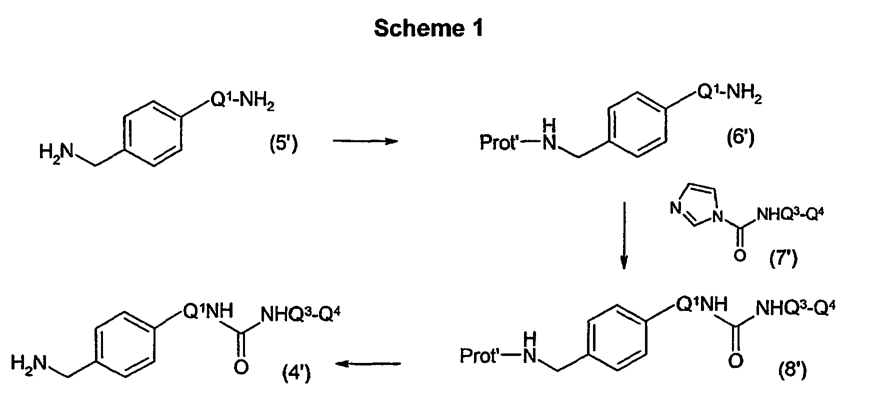 wherein Q¹, Q³ and Q⁴ are as previously defined, and Prot' represents a suitable protecting group for the amine, which includes but is not limited to *tert-*butoxycarbonyl.

In a typical procedure the amine (5') is treated with di-*tert-*butyl dicarbonate in a suitable solvent (e.g. dichloromethane) at a temperature between 0°C and 40°C for a period of 0.5 to 4 hours to give the monoprotected amine (6'). The amine (6') is then reacted with an excess of imidazocarbamate (7') in a suitable solvent (e.g. toluene or dichloromethane) at a temperature between 20°C and 100°C, for a period of 1 to 40 hours to give the urea of formula (8'). The urea (8') is then deprotected using methods well known to those skilled in the art, without modifying the rest of the molecule to give the amine of formula (4'.

The imidazocarbamate of formula (7') is prepared by treatment of an amine of formula H₂NQ₃Q₄ with 1,1-carbonyldiimidazole in a suitable solvent (e.g. dichloromethane or tetrahydrofuran) at a temperature between 0°C and 50°C, for a period of 1 to 40 hours.

Alternatively, the compound of formula (2) may be prepared by coupling the acid (3) with an amine of formula (5) : 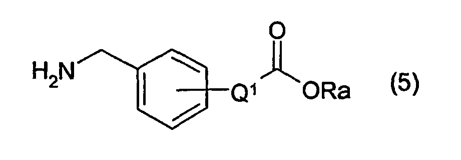 wherein Q¹ is as previously defined and Ra is a suitable acid protecting group, preferably a (C₁-C₄)alkyl group, which includes, but is not limited to, methyl and ethyl. The resulting ester is hydrolysed to give the acid, which is then subjected to further coupling with an amine of formula HNQ³Q⁴ (selected from a range of commercially available amines, or prepared according to standard methodology well known to the man skilled in the art), to give the compound of formula (2) where Q² represents the group of formula -C(=O)NH-.

In a typical procedure, the hydrolysis of the ester to give an acid is undertaken according to any method well known to the man skilled in the art to prepare an acid from an ester, without modifying the rest of the molecule. For example, the ester may be hydrolysed by treatment with aqueous acid or base (e.g. hydrochloric acid, potassium hydroxide, sodium hydroxide or lithium hydroxide), optionally in the presence of a co-solvent (e.g. tetrahydrofuran/1,4-dioxan), at a temperature comprised between 20°C and 100°C, for a period of 1 to 40 hours.

Said amine (4) is either commercially available or may be prepared by conventional methods well-known to the one skilled in the art (e.g. acylation, sulfonylation, reduction, oxidation, alkylation, protection, deprotection etc...) from commercially available materials.

The acid of formula (3) may be prepared from the corresponding ester of formula (6) : 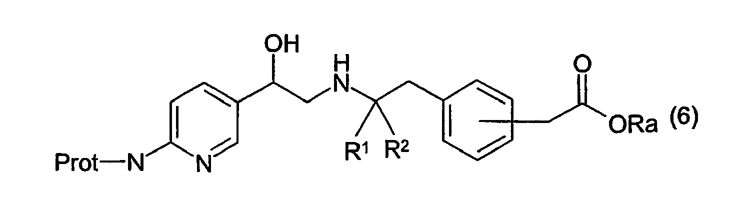 wherein Prot, R¹, R² and Ra are as previously defined according to any method well-known to the one skilled in the art to prepare an acid from an ester, without modifying the rest of the molecule, as previously described.

In a typical procedure, the hydrolysis of the ester to give an acid is undertaken according to any method well known to the man skilled in the art to prepare an acid from an ester, without modifying the rest of the molecule. For example, the ester may be hydrolysed by treatment with aqueous acid or base (e.g. hydrochloric acid, potassium hydroxide, sodium hydroxide or lithium hydroxide), optionally in the presence of a co-solvent (e.g. tetrahydrofuran/1,4-dioxan), at a temperature comprised between 20°C and 100°C, for a period of 1 to 40 hours.

The ester of formula (6) may be prepared according to different routes depending on the choice of R¹ and R².

If R¹ is hydrogen and R² is (C₁-C₄)alkyl the ester of formula (6) may be prepared by reaction of an amine of formula (7) : 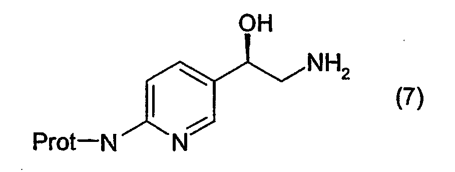 with an excess of a ketone of formula (8) : 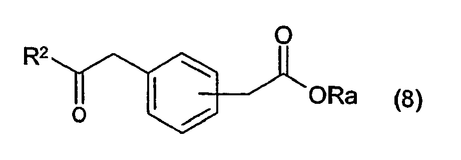 wherein Prot, R² and Ra are as previously defined, to form an intermediate compound, which is reduced by a suitable reducing agent (e.g. sodium cyanoborohydride of formula NaCN(BH)₃ or sodium triacetoxyborohydride of formula Na(OAC)₃BH), optionally in the presence of sodium acetate or acetic acid. The reaction is generally done in a suitable solvent such as tetrahydrofuran or dichloromethane, at temperature comprised between 20°C and 80°C for 3 to 72 hours, giving the compound of formula (6) as a mixture of diastereomers. According to another alternative, the reduction may be carried out in the presence of a drying agent such as molecular sieves or magnesium sulfate.

The amine of formula (7) may be prepared starting from a 2-amino-5-bromo-pyridine as described in EP 1 078 924 or WO 99/32475.

Alternatively, if R¹ is hydrogen and R² is (C₁-C₄)alkyl the ester of formula (6) may be prepared according to scheme 2 as follows : 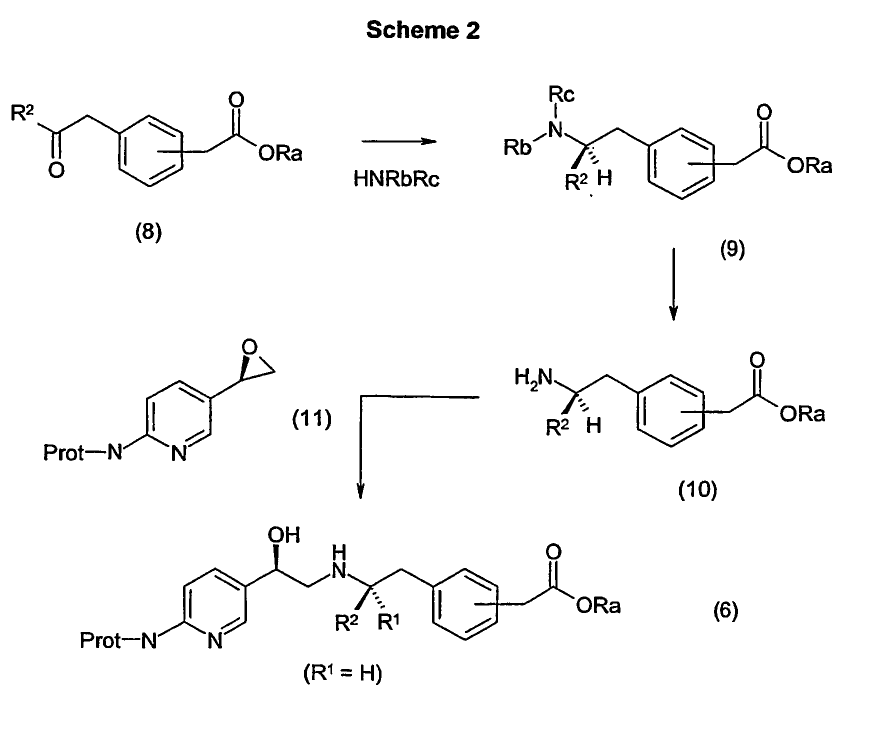 wherein Ra and Prot are as previously defined, and Rb and Rc represent any suitable substituents so that HNRbRc is a chiral amine (for example, Rb may be hydrogen and Rc may be a α-methylbenzyl group) and the bonds between N and Rb and N and Rc can be easily cleaved to give the free amine of formula (10).

In a typical procedure, the ketone of formula (8) is reacted with a suitable chiral non-racemic amine HNRbRc (e.g. α-methylbenzylamine or any other commercially available chiral non-racemic amine) to form a chiral intermediate, which is reduced by a suitable reducing agent (e.g. sodium cyanoborohydride of formula NaCN(BH)₃ or sodium triacetatoxyborohydride of formula Na(OAc)₃BH) optionally in the presence of acetic acid, and also optionally in the presence of a drying agent (e.g. molecular sieves, magnesium sulfate) as previously described. The resulting product is then converted to the hydrochloride salt and selectively crystallised from a suitable solvent or mixture of solvents (e.g. isopropanol, ethanol, methanol, diisopropyl ether or diisopropyl ether/methanol) to give the diastereomerically pure product of formula (9), or its enantiomer, if the opposite enantiomer of the amine HNRbRc is used.

The protected amine of formula (9) is then cleaved to give the corresponding free amine of formula (10) using standard methodology for cleaving nitrogen protecting groups, such as that found in the text book (see for example T.W. Greene, Protective Groups in Organic Synthesis, A. Wiley-Interscience Publication, 1981). When a α-methylbenzylamine is used, then the cleavage may be done using ammonium formate and palladium hydroxide on carbon of formula Pd(OH)₂/C as catalyst.

Said amine of formula (10) is then reacted with an epoxide of formula (11) in a suitable solvent or mixture of solvents (e.g. dimethyl sulfoxide/toluene), at a temperature comprised between 20°C and 80°C and optionally in the presence of a catalyst for 8 to 40 hours, to give the ester of formula (6).

The epoxide of formula (11) may be prepared starting from starting from a 2-amino-5-bromo-pyridine as described in EP 1 078 924 or WO 99/32475.

The ketone of formula (8) described above may be prepared by enolate arylation of an aryl halide of formula (12) : 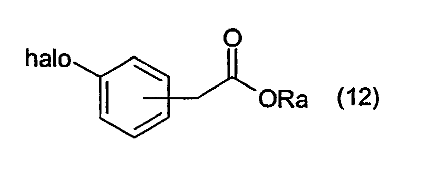 wherein Ra is as previously defined and halo represents an halogen atom, which includes, but is not limited to, fluoro, chloro and bromo.

In a typical procedure, the aryl halide of formula (12) is reacted with a tin enolate generated *in situ* by treatment of a vinyl acetate (e.g. isoprenyl acetate with tri-n-butyltin methoxide of formula BU₃SnOMe) in the presence of a suitable palladium catalyst (palladium acetate/ tri-*ortho*-tolylphosphine of formula Pd(OAc)₂/P(o-Tol)₃) in a non-polar solvent (e.g. toluene, benzene, hexane). Preferably, the reaction is carried out at a temperature comprised between 80°C and 110°C for 6 to 16 hours.

The aryl halide of formula (12) may be prepared by esterification of the corresponding acid of formula (13) : 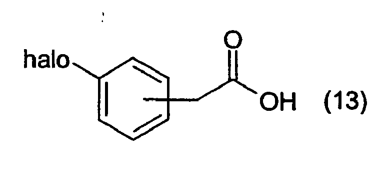 wherein halo is as previously defined,
according to any method well-known to the one skilled in the art to prepare an ester from an acid, without modifying the rest of the molecule.

In a typical procedure, the acid of formula (13) is reacted with an alcoholic solvent of formula RaOH, wherein Ra is as previously defined, in the presence of an acid, such as hydrochloric acid, at a temperature between 10°C and 40°C (room temperature) for 8 to 16 hours.

According to another alternative, the acid of formula (13) is reacted with a bromoalkyl of formula RaBr in the presence of a suitable base such as cesium carbonate, in a suitable organic solvent (e.g. *N,N*-dimethylformamide, tetrahydrofuran) at a temperature and for a time as mentioned above.

The acid of formula (13) is either a commercial product or it may be prepared by conventional procedures well-known to the man skilled in the art.

If R¹ and R² are both different from hydrogen, then the ester of formula (6) may be prepared according to scheme 3 as follows: 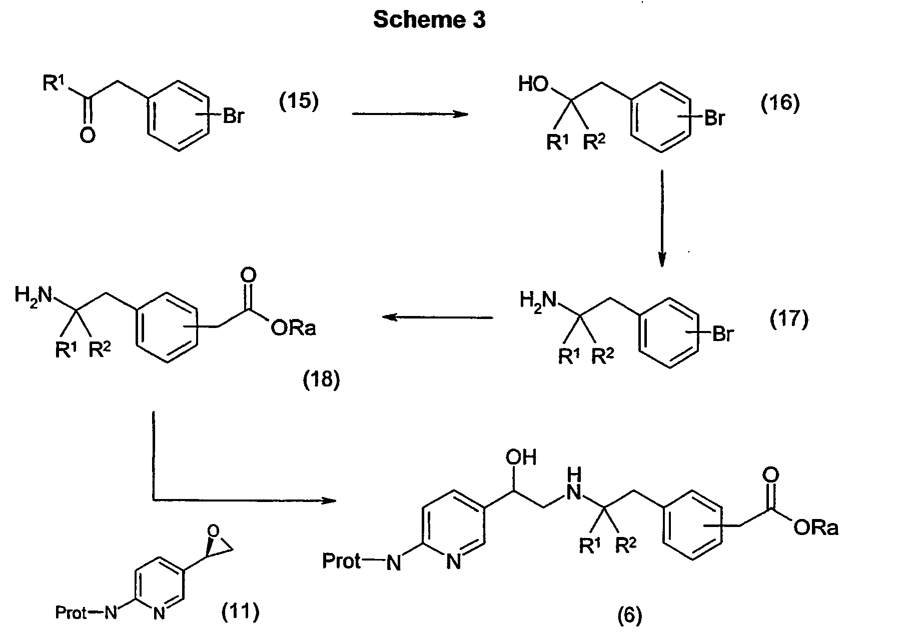 wherein R¹, R², Ra and Prot' are as previously defined.

In a typical procedure, the halo ketone of formula (15) is reacted with an "activated" alkyl (organometallic alkyl such as R²MgBr, R²MgCl or R²Li) to give the corresponding tertiary alcohol of formula (16). This organometallic addition is generally undertaken in a suitable solvent such as tetrahydrofuran, ether, cyclohexane or 1,4-dioxane, at a temperature comprised between 10°C and 40°C (room temperature) for 1 to 24 hours

Said tertiary alcohol of formula (16) is then treated with an alkyl nitrile (e.g. acetonitrile, chloroacetonitrile) in the presence of an acid (e.g. sulfuric acid, acetic acid) to give a protected intermediate which in turn is cleaved using standard methodology for cleaving nitrogen protecting group such as those mentioned in textbooks, to give the amine of formula (17).

The amine of formula (17) is then converted to the boronic acid ester by treatment with a suitable boron source (e.g. pinacolborane, bis(pinacolato)diboron) in the presence of a suitable palladium catalyst (e.g. palladium(II)acetate/ tri-*ortho*-tolylphosphine of formula Pd(OAc)₂/P(o-tol)₃ or (diphenylphosphino)ferrocenyl palladium(II)chloride of formula dppfPdCl₂). The reaction is generally undertaken in a suitable solvent such as dimethyl sulfoxide or toluene, optionally in the presence of a base (e.g. potassium acetate), at a temperature comprised between 60°C and 110°C for a period of 4 to 24 hours. The intermediate boronic acid ester is then coupled with ethyl bromoacetate in the presence of a suitable palladium catalyst (e.g. tetrakis(triphenylphosphine)palladium(0) of formula Pd(PPh₃)₄, palladium acetate/tri-ortho-tolylphosphine of formula Pd(OAc)₂/P(o-tol)₃ or (diphenylphosphino)ferrocenyl palladium chloride of formula dppfPdCl₂) to give the compound of formula (18).

The compound of formula (6) is finally obtained by reaction of the compound of formula (18) with the epoxide of formula (11) as previously described.

The compound of formula (15) is either commercial or it may be easily prepared from commercial compounds by conventional procedures well known to the one skilled in the art.

The amine of formula (10), where R¹ and R² are both H, may be prepared according to the following scheme: 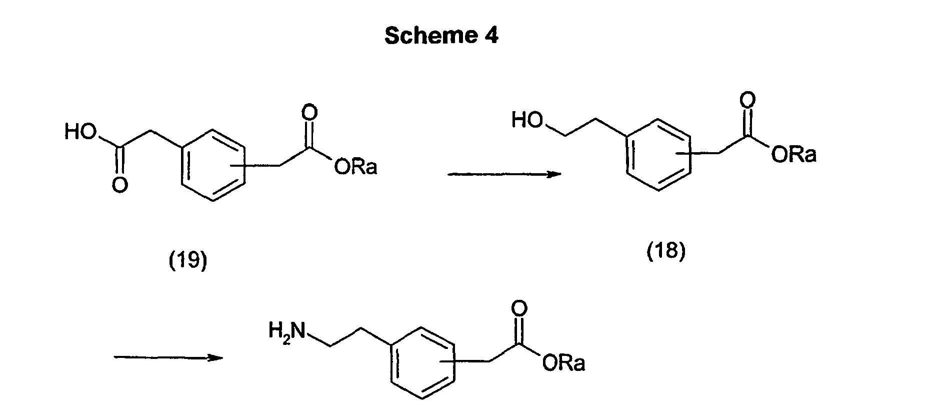 wherein R¹, R² and Ra are as previously defined.

In a typical procedure, the acid of formula (19) is preferentially reduced to the corresponding alcohol (18) in the presence of the ester. This may be performed by formation of the acyl imidazole or mixed anhydride and subsequent reduction with sodium borohydride or another suitable reducing agent.

Said primary alcohol of formula (18) is then converted into a leaving group such as mesylate, tosylate, bromide or iodide and displaced with appropriate amine nucleophile. The preferred nucleophile is azide ion which can then be reduced to the primary amine via hydrogenation or triphenylphosphine. Alternative nucleophiles could include ammonia or alkylamines such as benzylamine or allylamine and subsequent cleavage of the alkyl group to furnish the amine.

All of the above reactions and the preparations of novel starting materials used in the preceding methods are conventional and appropriate reagents and reaction conditions for their performance or preparation as well as procedures for isolating the desired products will be well-known to those skilled in the art with reference to literature precedents and the examples and preparations hereto.

For some of the steps of the here above described process of preparation of the 2-amino-pyridine derivatives of formula (1), it can be necessary to protect the potential reactive functions that are not wished to react, and to cleave said protecting groups in consequence. In such a case, any compatible protecting radical can be used. In particular methods of protection and deprotection such as those described by T.W. GREENE (Protective Groups in Organic Synthesis, A. Wiley-Interscience Publication, 1981) or by McOMIE (Protective Groups in Organic Chemistry, Plenum Press, 1973), can be used.

Also, the 2-amino-pyridine derivatives of formula (1) as well as intermediate for the preparation thereof can be purified according to various well-known methods, such as for example crystallization or chromatography.

In the above compounds of formula (1), the following substitutions are preferred:
R¹ is H and R² is C₁-C₄ alkyl, more preferably CH₃, or R¹ and R² are the same or different and are selected from C₁-C₄ alkyl, more preferably R¹ and R² are both CH₃ and/or,
n is 0 and Q² is -C(=O)NH- or SO₂NH-, or n is 1 and Q² is -NH-C(=O)- or NHC(=O)NH- and/or,
Q³ is selected from single bond, CH₂, CH(-CH₃)CH₂OH-, CH(CH₃)-,-CH(CH₂OH)CH₂-, -(CH₂)₂-, -(CH₂)₂-OCH₂- and -(CH₂)₂-O-(CH₂)₃- and/or,
Q⁴ is selected from 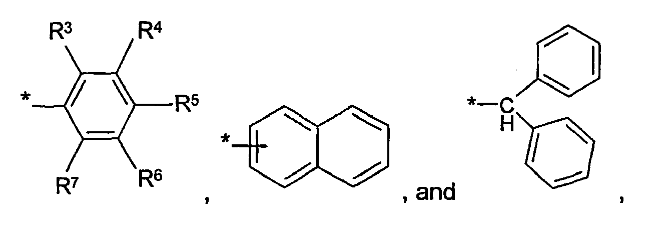
wherein * represents the attachment point to Q³ and R³, R⁴, R⁵, R⁶ and R⁷ are independently selected from H,
- OR⁹, provided at least 2 of R³ to R⁷ are equal to H, or
- SO₂NR⁸R⁹ or CH₂-NHC(=O)NH-R⁹ provided at least 4 of R³ to R⁷ are equal to H,
wherein R⁸ is selected from H or C₁-C₄ alkyl and R⁹ is selected from H, C₁-C₄ alkyl, benzyl optionally substituted with 1, 2, 3 or 4 C₁-C₄ alkoxy, and a group 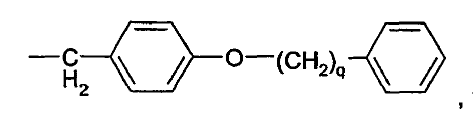 wherein q is an integer selected from 0, 1,2 and 3.

Preferably, the group Q¹-Q³-Q³-Q⁴ is in meta or para position.

Compound of particular interest with respect to the present invention are those wherein n is 0 and Q² is -C(=O)NH-.

Compound of particular interest with respect to the present invention are compounds of formula (1a) 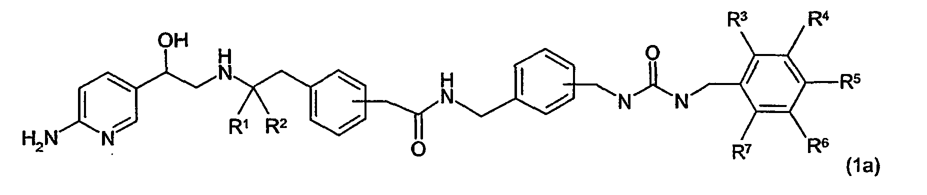 wherein the CH₂-C(=O)NH-benzyl and the CH₂-NHC(=O)NH-benzyl-Q¹-Q²-Q³-Q⁴ groups are independently in para or meta position and,
wherein, R³, R⁴, R⁵, R⁶ and R⁷ are independently selected from H, C₁-C₄ alkyl, OR⁹, phenoxy, SR⁹, halo, CF₃, OCF₃, COOR⁹, SO₂NR⁸R⁹, CONR⁸R⁹, NHR⁸R⁹ and NHCOR⁹ provided at least 2 of R³ to R⁷ are equal to H;
wherein R⁸ is selected from H or C₁-C₄ alkyl and R⁹ is selected from H, C₁-C₄ alkyl and benzyl optionally substituted with 1, 2, 3 or 4 C₁-C₄ alkoxy.

Preferred compounds of formula (1a) are those in which,
R¹ is CH₃ and R² is H, and/or,
R³ R⁴, R⁵, R⁶ and R⁷ are independently selected from H,
- OR⁹, phenoxy provided at least 2 of R³ to R⁷ are equal to H, or
- SO₂NR⁸R⁹ provided at least 4 of R³ to R⁷ are equal to H,
wherein R⁸ is selected from H or C₁-C₄ alkyl and R⁹ is selected from H, C₁-C₄ alkyl, phenyl, benzyl optionally substituted with 1, 2, 3 or 4 C₁-C₄ alkoxy.

Particularly preferred are the compounds of the formula (1) as described in the Examples section hereafter, i.e. :
4-{[2-(3-{2-[(2R)-2-(6-Amino-pyridin-3-yl)-2-hydroxy-ethylamino]-propyl}-phenyl)-acetylamino]-methyl}-*N*-(3,4-dimethoxy-benzyl)-benzamide,
4-{[2-(3-{2-[(2*R*)-2-(6-Amino-pyridin-3-yl)-2-hydroxy-ethylamino]-propyl}-phenyl)-acetylamino]-methyl}-*N*-(4-sulfamoyl-benzyl)-benzamide,
4-{[2-(3-{2-[(2*R*)-2-(6-Amino-pyridin-3-yl)-2-hydroxy-ethylamino]-propyl}-phenyl)-acetylamino]-methyl}-*N-*(2-ethoxy-benzyl)-benzamide,
4-{[2-(3-{2-[(2*R*)-2-(6-Amino-pyridin-3-yl)-2-hydroxy-ethylamino]-propyl}-phenyl)-acetylamino]-methyl}-*N-*[(1*R*)-1-(4-methoxy-phenyl)-ethyl]-benzamide,
4-([2-(3-{2-[(2*R*)-2-(6-Amino-pyridin-3-yl)-2-hydroxy-ethylamino]-propyl)-phenyl)-acetylamino]-methyl)-*N*-((1*R*)-1-hydroxymethyl-2-phenyl-ethyl)-benzamide,
4-{[2-(3-{2-[(2*R*)-2-(6-Amino-py(idin-3-yl)-2-hydroxy-ethylamino]-propyl}-phenyl)-acetylamino]-methyl}-M-((1*R*, 2*S*)-2-hydroxy-1-methyl-2-phenyl-ethyl)-benzamide,
4-{[2-(3-{2-[(2*R*)-2-(6-Amino-pyridin-3-yl)-2-hydroxy-ethylamino]-propyl}-phenyl)-acetylamino]-methyl}-*N*-((1*S*, 2*R*)-2-hydroxy-1-methyl-2-phenyl-ethyl)-benzamide,
4-{[2-(3-{2-[(2*R*)-2-(6-Amino-pyridin-3-yl)-2-hydroxy-ethylamino]-propyl}-phenyl)-acetylamino]-methyl}-*N*-[2-(3-methoxy-phenyl)-ethyl]-benzamide,
4-{[2-(3-{(2*R*)-2-[(2*R*)-2-(6-Amino-pyridin-3-yl)-2-hydroxy-ethylamino]-propyl}-phenyl)-acetylamino]-methyl}-*N*-(3,4-dimethoxy-benzyl)-benzamide,
4-{[2-(3-{(2*R*)-2-[(2*R*)-2-(6-Amino-pyridin-3-yl)-2-hydroxy-ethylamino]-propyl}-phenyl)-acetylamino]-methyl}-*N*-(2-ethoxy-benzyl)-benzamide,
4-{[2-(3-{(2*R*)-2-[(2*R*)-2-(6-Amino-pyridin-3-yl)-2-hydroxy-ethylamino]-propyl}-phenyl)-acetylamino]-methyl}-*N*-(4-hydroxy-3-methoxy-benzyl)-benzamide,
4-{[2-(3-{(2*R*)-2-[(2*R*)-2-(6-Amino-pyridin-3-yl)-2-hydroxy-ethylamino]-propyl}-phenyl)-acetylamino]-methyl}-*N-*(4-sulfamoyl-benzyl)-benzamide,
4-{[2-(3-{(2*R*)-2-[(2*R*)-2-(6-Amino-pyridin-3-yl)-2-hydroxy-ethylamino]-propyl}-phenyl)-acetylamino]-methyl}-*N*-((1*R*, 2*S*)-2-hydroxy-1-methyl-2-phenyl-ethyl)-benzamide,
N-(4-{[2-(3-{(2*R*)-2-[(2*R*)-2-(6-Amino-pyridin-3-yl)-2-hydroxy-ethylamino]-propyl}-phenyl)-acetylamino]-methyl}-benzyl)-2,2-diphenyl-acetamide,
2-(3-{(2*R*)-2-[(2*R*)-2-(6-Amino-pyridin-3-yl)-2-hydroxy-ethylamino]-propyl}-phenyl)-*N-*{4-[(benzhydryl-amino)-methyl]-benzyl}-acetamide,
4-{[2-(3-{(2*R*)-2-[(2*R*)-2-(6-Amino-pyridin-3-yl)-2-hydroxy-ethylamino]-propyl}-phenyl)-acetylamino]-methyl}-*N*-(2-benzyloxy-ethyl)-benzamide,
4-{[2-(3-{(2*R*)-2-[(2*R*)-2-(6-Amino-pyridin-3-yl)-2-hydroxy-ethylamino]-propyl}-phenyl)-acetylamino]-methyl}-*N*-[2-(3-phenyl-propoxy)-ethyl]-benzamide,
4-{[2-(3-{(2*R*)-2-[(2*R*)-2-(6-Amino-pyridin-3-yl)-2-hydroxy-ethylamino]-propyl}-phenyl)-acetylamino]-methyl}-*N*-[2-(naphthalen-1-ylmethoxy)-ethyl]-benzamide,
4-{[2-(3-{(2*R*)-2-[(2*R*)-2-(6-Amino-pyrid in-3-yl)-2-hydroxy-ethylamino]-propyl}-phenyl)-acetylamino]-methyl}-*N*-(3-benzylsulfamoyl-benzyl)-benzamide,
4-{[2-(3-{(2*R*)-2-[(2*R*)-2-(6-Amino-pyridin-3-yl)-2-hydroxy-ethylamino]-propyl}-phenyl)-acetylamino]-methyl}-*N*-(4-methylsulfamoyl-benzyl)-benzamide,
4-{[2-(3-{(2*R*)-2-[(2*R*)-2-(6-Amino-pyridin-3-yl)-2-hydroxy-ethylamino]-propyl}-phenyl)-acetylamino]-methyl}-*N*-(4-ethylsulfamoyl-benzyl)-benzamide,
4-{[2-(3-{(2*R*)-2-[(2*R*)-2-(6-Amino-pyridin-3-yl)-2-hydroxy-ethylamino]-propyl}-phenyl)-acetylamino]-methyl}-*N*-(3-benzylsulfamoyl-benzyl)-benzamide,
4-{[2-(3-{(2*R*)-2-[(2*R*)-2-(6-Amino-pyridin-3-yl)-2-hydroxy-ethylamino]-propyl}-phenyl)-acetylamino]-methyl}-N-(3-methylsulfamoyl-benzyl)-benzamide,
4-{[2-(3-{(2*R*}-2-[(2*R*)-2-(6-Amino-pyridin-3-yl)-2-hydroxy-ethylamino]-propyl}-phenyl)-acetylamino]-methyl}-*N*-(3-ethylsulfamoyl-benzyl)-benzamide,
2-(3-{(2*R*)-2-[(2*R*)-2-(6-Amino-pyridin-3-yl)-2-hydroxy-ethylamino]-propyl}-phenyl)-*N*-(4-benzylsulfamoyl-benzyl)-acetamide hydrochloride.
4-{[2-(3-{(2*R*)-2-[(2*R*)-2-(6-Amino-pyridin-3-yl)-2-hydroxy-ethylamino]-propyl}-phenyl)-acetylamino]-methyl}-*N*-[4-(3-benzyl-ureidomethyl)-benzyl]-benzamide
4-{[2-(3-{(2*R*)-2-[(2*R*)-2-(6-Amino-pyridin-3-yl)-2-hydroxy-ethylamino]-propyl}-phenyl)-acetylamino]-methyl}-*N*-{4-[3-(3,4-dimethoxy-benzyl)-ureidomethyl]-benzyl}-benzamide
4-{[2-(3-{(2*R*)-2-[(2*R*)-2-(6-Amino-pyridin-3-yl)-2-hydroxy-ethylamino]-propyl}-phenyl)-acetylamino]-methyl}-*N*-{4-[3-(4-phenoxy-benzyl)-ureidomethyl]-benzyl}-benzamide,
4-{[2-(3-{(2*R*)-2-[(2*R*)-2-(6-Amino-pyridin-3-yl)-2-hydroxy-ethylamino]-propyl}-phenyl)-acetylamino]-methyl}-*N*-{4-[3-(4-benzyloxy-benzyl)-ureidomethyl]-benzyl}-benzamide,
2-(3-{(2*R*)-2-[(2*R*)-2-(6-Amino-pyridin-3-yl)-2-hydroxy-ethylamino]-propyl}-phenyl)-*N-*{4-[3-(3-phenoxy-benzyl)-ureidomethyl]-benzyl}-acetamide,
2-(3-{(2*R*)-2-[(2*R*)-2-(6-Amino-pyridin-3-yl)-2-hydroxy-ethylamino]-propyl}-phenyl)-*N-*{4-[3-(3,4-dimethoxy-benzyl)-ureidomethyl]-benzyl}-acetamide, and,
2-(3-{(2*R*)-2-[(2*R*)-2-(6-Amino-pyridin-3-yl)-2-hydroxy-ethylamino]-propyl}-phenyl)-*N*-{4-[3-(4-phenoxy-benzyl)-ureidomethyl]-benzyl}-acetamide.

According to one aspect of the present invention, the compounds of formula (1) wherein the CH₂-C(=O)NH-benzyl-Q¹-Q²-Q³-Q⁴ group is in position meta are generally preferred.

The compounds of formula (1) may also be optionally transformed into pharmaceutically acceptable salts. In particular, these pharmaceutically acceptable salts of the compounds of the formula (1) include the acid addition and the base salts (including disalts) thereof.

Suitable acid addition salts are formed from acids which form non-toxic salts. Examples include the acetate, aspartate, benzoate, besylate, bicarbonate/carbonate, bisulphate/sulphate, borate, camsylate, citrate, edisylate, esylate, formate, fumarate, gluceptate, gluconate, glucuronate, hexafluorophosphate, hibenzate, hydrochloride/chloride, hydrobromide/bromide, hydroiodide/iodide, hydrogen phosphate, isethionate, D- and L-lactate, malate, maleate, malonate, mesylate, methylsulphate, 2-napsylate, nicotinate, nitrate, orotate, oxalate, palmitate, pamoate, phosphate/hydrogen, phosphate/phosphate dihydrogen" saccharate, stearate, succinate, D- and L-tartrate, 1-hydroxy-2-naphthoate and tosylate salts.

Suitable base salts are formed from bases which form non-toxic salts. Examples include the aluminium, arginine, benzathine, calcium, choline, diethylamine, diolamine, glycine, lysine, magnesium, meglumine, olamine, potassium, sodium, tromethamine and zinc salts.

Hemisalts of acids and bases may also be formed, for example, hemisulphate and hemicalcium salts.

For a review on suitable salts, see Stahl and Wermuth, Handbook of Pharmaceutical Salts: Properties, Selection, and Use, Wiley-VCH, Weinheim, Germany (2002).

Pharmaceutically acceptable salts of compounds of formula (1) may be prepared by one or more of three methods:
(i) by reacting the compound of formula (1) with the desired acid or base;
(ii) by removing an acid- or base-labile protecting group from a suitable precursor of the compound of formula (1) or by ring-opening a suitable cyclic precursor, for example, a lactone or lactam, using the desired acid or base; or
(iii) by converting one salt of the compound of formula (1) to another by reaction with an appropriate acid or base or by means of a suitable ion exchange column.

All three reactions are typically carried out in solution. The resulting salt may precipitate out and be collected by filtration or may be recovered by evaporation of the solvent. The degree of ionisation in the resulting salt may vary from completely ionised to almost non-ionised.

The compounds of the invention may exist in both unsolvated and solvated forms. The term 'solvate' is used herein to describe a molecular complex comprising the compound of the invention and a stoichiometric amount of one or more pharmaceutically acceptable solvent molecules, for example, ethanol. The term 'hydrate' is employed when said solvent is water.

Included within the scope of the invention are complexes such as clathrates, drug-host inclusion complexes wherein, in contrast to the aforementioned solvates, the drug and host are present in stoichiometric or non-stoichiometric amounts. Also included are complexes of the drug containing two or more organic and/or inorganic components which may be in stoichiometric or non-stoichiometric amounts. The resulting complexes may be ionised, partially ionised, or non-ionised. For a review of such complexes, see J Pharm Sci, 64 (8), 1269-1288 by Haleblian (August 1975).

Hereinafter all references to compounds of formula (1) include references to salts, solvates and complexes thereof and to solvates and complexes of salts thereof.

The compounds of the invention include compounds of formula (1) as hereinbefore defined, including all polymorphs and crystal habits thereof, prodrugs and isomers thereof (including optical, geometric and tautomeric isomers) as hereinafter defined and isotopically-labeled compounds of formula (1).

As indicated, so-called 'pro-drugs' of the compounds of formula (1) are also within the scope of the invention. Thus certain derivatives of compounds of formula (1) which may have little or no pharmacological activity themselves can, when administered into or onto the body, be converted into compounds of formula (1) having the desired activity, for example, by hydrolytic cleavage. Such derivatives are referred to as 'prodrugs'. Further information on the use of prodrugs may be found in 'Pro-drugs as Novel Delivery Systems, Vol. 14, ACS Symposium Series (T. Higuchi and W. Stella) and 'Bioreversible Carriers in Drug Design', Pergamon Press, 1987 (ed. E. B Roche, American Pharmaceutical Association).

Prodrugs in accordance with the invention can, for example, be produced by replacing appropriate functionalities present in the compounds of formula (1) with certain moieties known to those skilled in the art as 'pro-moieties' as described, for example, in "Design of Prodrugs" by H. Bundgaard (Elsevier, 1985).

Some examples of prodrugs in accordance with the invention include:
(i) where the compound of formula (1) contains a carboxylic acid functionality (-COOH), an ester thereof, for example, a compound wherein the hydrogen of the carboxylic acid functionality of the compound of formula (1) is replaced by (C₁-C₈)alkyl;
(ii) where the compound of formula (1) contains an alcohol functionality (-OH), an ether thereof, for example, a compound wherein the hydrogen of the alcohol functionality of the compound of formula (1) is replaced by (C₁-C₆)alkanoyloxymethyl; and
(iii) where the compound of formula (1) contains a primary or secondary amino functionality (-NH₂ or -NHR where R ≠H), an amide thereof, for example, a compound wherein, as the case may be, one or both hydrogens of the amino functionality of the compound of formula (1) is/are replaced by (C₁-C₁₀)alkanoyl.

Further examples of replacement groups in accordance with the foregoing examples and examples of other prodrug types may be found in the aforementioned references.

Moreover, certain compounds of formula (1) may themselves act as prodrugs of other compounds of formula (1).

Also included within the scope of the invention are metabolites of compounds of formula (1), that is, compounds formed *in vivo* upon administration of the drug. Some examples of metabolites in accordance with the invention include
(i) where the compound of formula (1) contains a methyl group, an hydroxymethyl derivative thereof (-CH₃ → -CH₂OH):
(ii) where the compound of formula (1) contains an alkoxy group, an hydroxy derivative thereof (-OR → -OH);
(iii) where the compound of formula (1) contains a tertiary amino group, a secondary amino derivative thereof (-NR¹R² → -NHR¹ or -NHR²);
(iv) where the compound of formula (1) contains a secondary amino group, a primary derivative thereof (-NHR¹ -> -NH₂);
(v) where the compound of formula (1) contains a phenyl moiety, a phenol derivative thereof (-Ph → -PhOH); and
(I).(vi) where the compound of formula (1) contains an amide group, a carboxylic acid derivative thereof (-CONH₂ → COOH).

Compounds of formula (1) containing one or more asymmetric carbon atoms can exist as two or more stereoisomers. Where a compound of formula (1) contains an alkenyl or alkenylene group, geometric *cis*/*trans* (or Z/E) isomers are possible. Where the compound contains, for example, a keto orstructural isomers are interconvertible *via* a low oxime group or an aromatic moiety,energy barrier, tautomeric isomerism ('tautomerism') can occur. This can take the form of proton tautomerism in compounds of formula (1) containing, for example, an imino, keto, or oxime group, or so-called valence tautomerism in compounds which contain an aromatic moiety. It follows that a single compound may exhibit more than one type of isomerism.

Included within the scope of the present invention are all stereoisomers, geometric isomers and tautomeric forms of the compounds of formula (1), including compounds exhibiting more than one type of isomerism, and mixtures of one or more thereof. Also included are acid addition or base salts wherein the counterion is optically active, for example, *d*-lactate or *l*-lysine, or racemic, for example, *dl*-tartrate or *dl*-arginine.

*Cis*/*trans* isomers may be separated by conventional techniques well known to those skilled in the art, for example, chromatography and fractional crystallisation.

Conventional techniques for the preparation/isolation of individual enantiomers include chiral synthesis from a suitable optically pure precursor or resolution of the racemate (or the racemate of a salt or derivative) using, for example, chiral high pressure liquid chromatography (HPLC).

Alternatively, the racemate (or a racemic precursor) may be reacted with a suitable optically active compound, for example, an alcohol, or, in the case where the compound of formula (1) contains an acidic or basic moiety, an acid or base such as tartaric acid or 1-phenylethylamine. The resulting diastereomeric mixture may be separated by chromatography and/or fractional crystallization and one or both of the diastereoisomers converted to the corresponding pure enantiomer(s) by means well known to a skilled person.

Chiral compounds of the invention (and chiral precursors thereof) may be obtained in enantiomerically-enriched form using chromatography, typically HPLC, on an asymmetric resin with a mobile phase consisting of a hydrocarbon, typically heptane or hexane, containing from 0 to 50% by volume of isopropanol, typically from 2% to 20%, and from 0 to 5% by volume of an alkylamine, typically 0.1% diethylamine. Concentration of the eluate affords the enriched mixture.

Stereoisomeric conglomerates may be separated by conventional techniques known to those skilled in the art - see, for example, "Stereochemistry of Organic Compounds" by E. L. Eliel (Wiley, New York, 1994).

According to one aspect of the present invention, the (R,R)-stereoisomer of the formula below, wherein Q¹, Q², Q³ and Q⁴ are as defined above, is generally preferred:

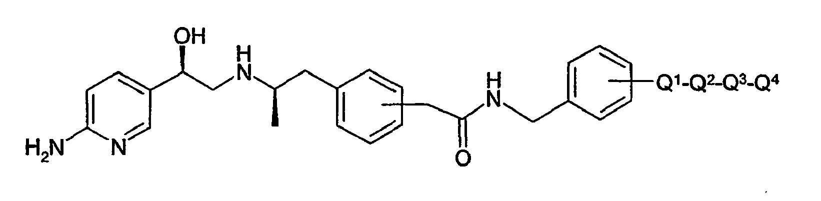

The compounds of the formula (1) according to the invention can moreover contain mobile hydrogen atoms, i.e. be present in various tautomeric forms. The present invention also relates to all tautomers of the compounds of the formula (1).

The present invention includes all pharmaceutically acceptable isotopically-labelled compounds of formula (1) wherein one or more atoms are replaced by atoms having the same atomic number, but an atomic mass or mass number different from the atomic mass or mass number which predominates in nature.

Examples of isotopes suitable for inclusion in the compounds of the invention include isotopes of hydrogen, such as ²H and ³H, carbon, such as ¹¹C, ¹³C and ¹⁴C, chlorine, such as ³⁶Cl, fluorine, such as ¹⁸F, iodine, such as ¹²³I and ¹²⁵I, nitrogen, such as ¹³N and ¹⁵N, oxygen, such as ¹⁵O, ¹⁷O and ¹⁸O, phosphorus, such as ³²P, and sulphur, such as ³⁵S.

Certain isotopically-labelled compounds of formula (1), for example, those incorporating a radioactive isotope, are useful in drug and/or substrate tissue distribution studies. The radioactive isotopes tritium, *i.e.* ³H, and carbon-14, *i.e.* ¹⁴C, are particularly useful for this purpose in view of their ease of incorporation and ready means of detection.

Substitution with heavier isotopes such as deuterium, *i.e.* ²H, may afford certain therapeutic advantages resulting from greater metabolic stability, for example, increased *in vivo* half-life or reduced dosage requirements, and hence may be preferred in some circumstances.

Substitution with positron emitting isotopes, such as ¹¹C, ¹⁸F, ¹⁵O and ¹³N, can be useful in Positron Emission Topography (PET) studies for examining substrate receptor occupancy.

Isotopically-labeled compounds of formula (1) can generally be prepared by conventional techniques known to those skilled in the art or by processes analogous to those described in the accompanying Examples and Preparations using an appropriate isotopically-labeled reagents in place of the non-labeled reagent previously employed.

Pharmaceutically acceptable solvates in accordance with the invention include those wherein the solvent of crystallization may be isotopically substituted, e.g. D₂O, d₆-acetone, d₆-DMSO.

The compounds of formula (1), their pharmaceutically acceptable salts and/or derived forms, are valuable pharmaceutically active compounds, which are suitable for the therapy and prophylaxis of numerous disorders in which the β2 receptor is involved or in which agonism of this receptor may induce benefit, in particular the allergic and non-allergic airways diseases but also in the treatment of other diseases such as, but not limited to those of the nervous system, premature labor, congestive heart failure, depression, inflammatory and allergic skin diseases, psoriasis, proliferative skin diseases, glaucoma and in conditions where there is an advantage in lowering gastric acidity, particularly in gastric and peptic ulceration.

The compounds of formula (1) and their pharmaceutically acceptable salts and derived forms as mentioned above can be administered according to the invention to animals, preferably to mammals, and in particular to humans, as pharmaceuticals for therapy and/or prophylaxis. They can be administered per se, in mixtures with one another or in the form of pharmaceutical preparations which as active constituent contain an efficacious dose of at least one compounds of formula (1), its pharmaceutically acceptable salts and/or derived forms, in addition to customary pharmaceutically innocuous excipients and/or additives.

The compounds of formula (1), their pharmaceutically acceptable salts and/or derived forms may be freeze-dried, spray-dried, or evaporatively dried to provide a solid plug, powder, or film of crystalline or amorphous material. Microwave or radio frequency drying may be used for this purpose.

The compounds of formula (1), their pharmaceutically acceptable salts and/or derived forms may be administered alone or in combination with other drugs and will generally be administered as a formulation in association with one or more pharmaceutically acceptable excipients. The term "excipient" is used herein to describe any ingredient other than the compound of the invention. The choice of excipient will to a large extent depend on the particular mode of administration.

The compounds of the invention may be administered orally. Oral administration may involve swallowing, so that the compound enters the gastrointestinal tract, or buccal or sublingual administration may be employed by which the compound enters the blood stream directly from the mouth.

Formulations suitable for oral administration include solid formulations such as tablets, capsules containing particulates, liquids, or powders, lozenges (including liquid-filled), chews, multi- and nano-particulates, gels, solid solution, liposome, films, ovules, sprays and liquid formulations.

Liquid formulations include suspensions, solutions, syrups and elixirs. Such formulations may be employed as fillers in soft or hard capsules and typically comprise a carrier, for example, water, ethanol, polyethylene glycol, propylene glycol, methylcellulose, or a suitable oil, and one or more emulsifying agents and/or suspending agents. Liquid formulations may also be prepared by the reconstitution of a solid, for example, from a sachet.

The compounds of the invention may also be used in fast-dissolving, fast-disintegrating dosage forms such as those described in Expert Opinion in Therapeutic Patents, 11 (6), 981-986, by Liang and Chen (2001).

For tablet dosage forms, depending on dose, the drug may make up from 1 weight % to 80 weight % of the dosage form, more typically from 5 weight % to 60 weight % of the dosage form. In addition to the drug, tablets generally contain a disintegrant. Examples of disintegrants include sodium starch glycolate, sodium carboxymethyl cellulose, calcium carboxymethyl cellulose,_croscarmellose sodium, crospovidone, polyvinylpyrrolidone, methyl cellulose, microcrystalline cellulose, lower alkyl-substituted hydroxypropyl cellulose, starch, pregelatinised starch and sodium alginate. Generally, the disintegrant will comprise from 1 weight % to 25 weight %, preferably from 5 weight % to 20 weight % of the dosage form.

Binders are generally used to impart cohesive qualities to a tablet formulation. Suitable binders include microcrystalline cellulose, gelatin, sugars, polyethylene glycol, natural and synthetic gums, polyvinylpyrrolidone, pregelatinised starch, hydroxypropyl cellulose and hydroxypropyl methylcellulose. Tablets may also contain diluents, such as lactose (monohydrate, spray-dried monohydrate, anhydrous and the like), mannitol, xylitol, dextrose, sucrose, sorbitol, microcrystalline cellulose, starch and dibasic calcium phosphate dihydrate.

Tablets may also optionally comprise surface active agents, such as sodium lauryl sulfate and polysorbate 80, and glidants such as silicon dioxide and talc. When present, surface active agents may comprise from 0.2 weight % to 5 weight % of the tablet, and glidants may comprise from 0.2 weight % to 1 weight % of the tablet.

Tablets also generally contain lubricants such as magnesium stearate, calcium stearate, zinc stearate, sodium stearyl fumarate, and mixtures of magnesium stearate with sodium lauryl sulphate. Lubricants generally comprise from 0.25 weight % to 10 weight %, preferably from 0.5 weight % to 3 weight % of the tablet.

Other possible ingredients include anti-oxidants, colourants, flavouring agents, preservatives and taste-masking agents.

Exemplary tablets contain up to about 80% drug, from about 10 weight % to about 90 weight % binder, from about 0 weight % to about 85 weight % diluent, from about 2 weight % to about 10 weight % disintegrant, and from about 0.25 weight % to about 10 weight % lubricant.

Tablet blends may be compressed directly or by roller to form tablets. Tablet blends or portions of blends may alternatively be wet-, dry-, or melt-granulated, melt congealed, or extruded before tabletting. The final formulation may comprise one or more layers and may be coated or uncoated; it may even be encapsulated.

The formulation of tablets is discussed in Pharmaceutical Dosage Forms: Tablets, Vol. 1, by H. Lieberman and L. Lachman (Marcel Dekker, New York, 1980).

Consumable oral films for human or veterinary use are typically pliable water-soluble or water-swellable thin film dosage forms which may be rapidly dissolving or mucoadhesive and typically comprise a compound of formula (1), a film-forming polymer, a binder, a solvent, a humectant, a plasticiser, a stabiliser or emulsifier, a viscosity-modifying agent and a solvent. Some components of the formulation may perform more than one function.

The compound of formula (1) may be water-soluble or insoluble. A water-soluble compound typically comprises from 1 weight % to 80 weight %, more typically from 20 weight % to 50 weight %, of the solutes. Less soluble compounds may comprise a greater proportion of the composition, typically up to 88 weight % of the solutes. Alternatively, the compound of formula (1) may be in the form of multiparticulate beads.

The film-forming polymer may be selected from natural polysaccharides, proteins, or synthetic hydrocolloids and is typically present in the range 0.01 to 99 weight %, more typically in the range 30 to 80 weight %.

Other possible ingredients include anti-oxidants, colorants, flavourings and flavour enhancers, preservatives, salivary stimulating agents, cooling agents, co-solvents (including oils), emollients, bulking agents, anti-foaming agents, surfactants and taste-masking agents.

Films in accordance with the invention are typically prepared by evaporative drying of thin aqueous films coated onto a peelable backing support or paper. This may be done in a drying oven or tunnel, typically a combined coater dryer, or by freeze-drying or vacuuming.

Solid formulations for oral administration may be formulated to be immediate and/or modified release. Modified release formulations include delayed-, sustained-, pulsed-, controlled-, targeted and programmed release.

Suitable modified release formulations for the purposes of the invention are described in US Patent No. 6,106,864. Details of other suitable release technologies such as high energy dispersions and osmotic and coated particles are to be found in Pharmaceutical Technology On-line, 25(2), 1-14, by Verma et al (2001). The use of chewing gum to achieve controlled release is described in WO 00/35298.

The compounds of the invention may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

Parenteral formulations are typically aqueous solutions which may contain excipients such as salts, carbohydrates and buffering agents (preferably to a pH of from 3 to 9), but, for some applications, they may be more suitably formulated as a sterile non-aqueous solution or as a dried form to be used in conjunction with a suitable vehicle such as sterile, pyrogen-free water.

The preparation of parenteral formulations under sterile conditions, for example, by lyophilisation, may readily be accomplished using standard pharmaceutical techniques well known to those skilled in the art.

The solubility of compounds of formula (1) used in the preparation of parenteral solutions may be increased by the use of appropriate formulation techniques, such as the incorporation of solubility-enhancing agents.

Formulations for parenteral administration may be formulated to be immediate and/or modified release. Modified release formulations include delayed-, sustained-, pulsed-, controlled-, targeted and programmed release. Thus compounds of the invention may be formulated as a solid, semi-solid, or thixotropic liquid for administration as an implanted depot providing modified release of the active compound. Examples of such formulations include drug-coated stents and PGLApoly(*dl*-lactic-coglycolic)acid (PGLA) microspheres.

The compounds of the invention may also be administered topically to the skin or mucosa, that is, dermally or transdermally. Typical formulations for this purpose include gels, hydrogels, lotions, solutions, creams, ointments, dusting powders, dressings, foams, films, skin patches, wafers, implants, sponges, fibres, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated - see, for example, J Pharm Sci, 88 (10), 955-958 by Finnin and Morgan (October 1999).

Other means of topical administration include delivery by electroporation, iontophoresis, phonophoresis, sonophoresis and microneedle or needle-free (*e.g*. Powderject^{™}, Bioject^{™}, etc.) injection.

Formulations for topical administration may be formulated to be immediate and/or modified release. Modified release formulations include delayed-, sustained-, pulsed-, controlled-, targeted and programmed release.

The compounds of the invention can also be administered intranasally or by inhalation, typically in the form of a dry powder (either alone, as a mixture, for example, in a dry blend with lactose, or as a mixed component particle, for example, mixed with phospholipids, such as phosphatidylcholine) from a dry powder inhaler or as an aerosol spray from a pressurised container, pump, spray, atomiser (preferably an atomiser using electrohydrodynamics to produce a fine mist), or nebuliser, with or without the use of a suitable propellant, such as 1,1,1,2-tetrafluoroethane or 1,1,1,2,3,3,3-heptafluoropropane. For intranasal use, the powder may comprise a bioadhesive agent, for example, chitosan or cyclodextrin.

The pressurised container, pump, spray, atomizer, or nebuliser contains a solution or suspension of the compound(s) of the invention comprising, for example, ethanol, aqueous ethanol, or a suitable alternative agent for dispersing, solubilising, or extending release of the active, a propellant(s) as solvent and an optional surfactant, such as sorbitan trioleate, oleic acid, or an oligolactic acid.

Prior to use in a dry powder or suspension formulation, the drug product is micronised to a size suitable for delivery by inhalation (typically less than 5 microns). This may be achieved by any appropriate comminuting method, such as spiral jet milling, fluid bed jet milling, supercritical fluid processing to form nanoparticles, high pressure homogenisation, or spray drying.

Capsules (made, for example, from gelatin or hydroxypropylmethylcellulose), blisters and cartridges for use in an inhaler or insufflator may be formulated to contain a powder mix of the compound of the invention, a suitable powder base such as lactose or starch and a performance modifier such as *l*-leucine, mannitol, or magnesium stearate. The lactose may be anhydrous or in the form of the monohydrate, preferably the latter. Other suitable excipients include dextran, glucose, maltose, sorbitol, xylitol, fructose, sucrose and trehalose.

A suitable solution formulation for use in an atomiser using electrohydrodynamics to produce a fine mist may contain from 1µg to 20mg of the compound of the invention per actuation and the actuation volume may vary from 1µl to 100µl. A typical formulation may comprise a compound of formula (1), propylene glycol, sterile water, ethanol and sodium chloride. Alternative solvents which may be used instead of propylene glycol include glycerol and polyethylene glycol.

Suitable flavours, such as menthol and levomenthol, or sweeteners, such as saccharin or saccharin sodium, may be added to those formulations of the invention intended for inhaled/intranasal administration.

Formulations for inhaled/intranasal administration may be formulated to be immediate and/or modified release using, for example, PGLA. Modified release formulations include delayed-, sustained-, pulsed-, controlled-, targeted and programmed release.

In the case of dry powder inhalers and aerosols, the dosage unit is determined by means of a valve which delivers a metered amount. Units in accordance with the invention are typically arranged to administer a metered dose or "puff' containing from 0.001mg to 10mg of the compound of formula (1). The overall daily dose will typically be in the range 0.001mg to 40mg which may be administered in a single dose or, more usually, as divided doses throughout the day.

The compounds of formula (1) are particularly suitable for an administration by inhalation

The compounds of the invention may be administered rectally or vaginally, for example, in the form of a suppository, pessary, or enema. Cocoa butter is a traditional suppository base, but various alternatives may be used as appropriate.

Formulations for rectal/vaginal administration may be formulated to be immediate and/or modified release. Modified release formulations include delayed-, sustained-, pulsed-, controlled-, targeted and programmed release.

The compounds of the invention may also be administered directly to the eye or ear, typically in the form of drops of a micronised suspension or solution in isotonic, pH-adjusted, sterile saline. Other formulations suitable for ocular and aural administration include ointments, biodegradable (*e.g*. absorbable gel sponges, collagen) and non-biodegradable (*e.g.* silicone) implants, wafers, lenses and particulate or vesicular systems, such as niosomes or liposomes. A polymer such as crossed-linked polyacrylic acid, polyvinylalcohol, hyaluronic acid, a cellulosic polymer, for example, hydroxypropylmethylcellulose, hydroxyethylcellulose, or methyl cellulose, or a heteropolysaccharide polymer, for example, gelan gum, may be incorporated together with a preservative, such as benzalkonium chloride. Such formulations may also be delivered by iontophoresis.

Formulations for ocular/aural administration may be formulated to be immediate and/or modified release. Modified release formulations include delayed-, sustained-, pulsed-, controlled-, targeted, or programmed release.

The compounds of the invention may be combined with soluble macromolecular entities, such as cyclodextrin and suitable derivatives thereof or polyethylene glycol-containing polymers, in order to improve their solubility, dissolution rate, taste-masking, bioavailability and/or stability for use in any of the aforementioned modes of administration.

Drug-cyclodextrin complexes, for example, are found to be generally useful for most dosage forms and administration routes. Both inclusion and non-inclusion complexes may be used. As an alternative to direct complexation with the drug, the cyclodextrin may be used as an auxiliary additive, *i.e*. as a carrier, diluent, or solubiliser. Most commonly used for these purposes are alpha-, beta- and gamma-cyclodextrins, examples of which may be found in International Patent Applications Nos. WO 91/11172, WO 94/02518 and WO 98/55148.

inasmuch as it may desirable to administer a combination of active compounds, for example, for the purpose of treating a particular disease or condition, it is within the scope of the present invention that two or more pharmaceutical compositions, at least one of which contains a compound in accordance with the invention, may conveniently be combined in the form of a kit suitable for coadministration of the compositions.

Thus the kit of the invention comprises two or more separate pharmaceutical compositions, at least one of which contains a compound of formula (1) in accordance with the invention, and means for separately retaining said compositions, such as a container, divided bottle, or divided foil packet. An example of such a kit is the familiar blister pack used for the packaging of tablets, capsules and the like.

The kit of the invention is particularly suitable for administering different dosage forms, for example parenteral, for administering the separate compositions at different dosage intervals, or for titrating the separate compositions against one another. To assist compliance, the kit typically comprises directions for administration and may be provided with a so-called memory aid.

For administration to human patients, the total daily dose of the compounds of the invention is typically in the range 0.001mg to 5000mg depending, of course, on the mode of administration. For example, an intravenous daily dose may only require from 0.001mg to 40mg. The total daily dose may be administered in single or divided doses_and may, at the physician's discretion, fall outside of the typical range given herein.

These dosages are based on an average human subject having a weight of about 65kg to 70kg. The physician will readily be able to determine doses for subjects whose weight falls outside this range, such as infants and the elderly.

For the avoidance of doubt, references herein to "treatment" include references to curative, palliative and prophylactic treatment.

According to another embodiment of the present invention, the compounds of the formula (1), or pharmaceutically acceptable salts, derived forms or compositions thereof, can also be used as a combination with one or more additional therapeutic agents to be co-administered to a patient to obtain some particularly desired therapeutic end result such as the treatment of pathophysiologically-relevant disease processes including, but not limited to (i) bronchoconstriction, (ii) inflammation, (iii) allergy, (iv) tissue destruction, (v) signs and symptoms such as breathlessness, cough. The second and more additional therapeutic agents may also be a compound of formula (1), or a pharmaceutically acceptable salt, derived forms or compositions thereof, or one or more β2 agonists known in the art. More typically, the second and more therapeutic agents will be selected from a different class of therapeutic agents.

As used herein, the terms "co-administration", "co-administered" and "in combination with", referring to the compounds of formula (1) and one or more other therapeutic agents, is intended to mean, and does refer to and include the following:
- simultaneous administration of such combination of compound(s) of formula (1) and therapeutic agent(s) to a patient in need of treatment, when such components are formulated together into a single dosage form which releases said components at substantially the same time to said patient,
- substantially simultaneous administration of such combination of compound(s) of formula (1) and therapeutic agent(s) to a patient in need of treatment, when such components are formulated apart from each other into separate dosage forms which are taken at substantially the same time by said patient, whereupon said components are released at substantially the same time to said patient,
- sequential administration of such combination compound(s) of formula (1) and therapeutic agent(s) to a patient in need of treatment, when such components are formulated apart from each other into separate dosage forms which are taken at consecutive times by said patient with a significant time interval between each administration, whereupon said components are released at substantially different times to said patient; and
- sequential administration of such combination of compound(s) of formula (1) and therapeutic agent(s) to a patient in need of treatment, when such components are formulated together into a single dosage form which releases said components in a controlled manner whereupon they are concurrently, consecutively, and/or overlapingly administered at the same and/or different times by said patient,
where each part may be administered by either the same or different route.

Suitable examples of other therapeutic agents which may be used in combination with the compound(s) of formula (1), or pharmaceutically acceptable salts, derived forms or compositions thereof, include, but are by no means limited to :
(a) 5-Lipoxygenase (5-LO) inhibitors or 5-lipoxygenase activating protein (FLAP) antagonists,
(b) Leukotriene antagonists (LTRAs) including antagonists of LTB₄, LTC₄, LTD₄, and LTE₄,
(c) Histamine receptor antagonists including H1 and H3 antagonists,
(d) α₁- and α₂-adrenoceptor agonist vasoconstrictor sympathomimetic agents for decongestant use,
(e) muscarinic M3 receptor antagonists or anticholinergic agents,
(f) PDE inhibitors, e.g. PDE3, PDE4 and PDE5 inhibitors,
(g) Theophylline,
(h) Sodium cromoglycate,
(i) COX inhibitors both non-selective and selective COX-1 or COX-2 inhibitors (NSAIDs),
(j) Oral and inhaled glucocorticosteroids,
(k) Monoclonal antibodies active against endogenous inflammatory entities,
(l) Anti-tumor necrosis factor (anti-TNF-α) agents,
(m)Adhesion molecule inhibitors including VLA-4 antagonists,
(n) Kinin-B₁ - and B₂-receptor antagonists,
(o) Immunosuppressive agents,
(p) Inhibitors of matrix metalloproteases (MMPs),
(q) Tachykinin NK₁, NK₂ and NK₃ receptor antagonists,
(r) Elastase inhibitors,
(s) Adenosine A2a receptor agonists,
(t) Inhibitors of urokinase,
(u) Compounds that act on dopamine receptors, e.g. D2 agonists,
(v) Modulators of the NFκβ pathway, e.g. IKK inhibitors,
(w) modulators of cytokine signalling pathyways such as p38 MAP kinase or syk kinase,
(x) Agents that can be classed as mucolytics or anti-tussive, and
(y) Antibiotics.

According to the present invention, combination of the compounds of formula (1) with :
- H3 antagonists,
- Muscarinic M3 receptor antagonists,
- PDE4 inhibitors,
- glucocorticosteroids,
- Adenosine A2a receptor agonists,
- Modulators of cytokine signalling pathyways such as p38 MAP kinase or syk kinase, or,
- Leukotriene antagonists (LTRAs) including antagonists of LTB₄, LTC₄, LTD₄, and LTE₄,
are preferred.

According to the present invention, combination of the compounds of formula (1) with :
- glucocorticosteroids, in particular inhaled glucocorticosteroids with reduced systemic side effects, including prednisone, prednisolone, flunisolide, triamcinolone acetonide, beclomethasone dipropionate, budesonide, fluticasone propionate, ciclesonide, and mometasone furoate, or
- muscarinic M3 receptor antagonists or anticholinergic agents including in particular ipratropium salts, namely bromide, tiotropium salts, namely bromide, oxitropium salts, namely bromide, perenzepine, and telenzepine,
are further preferred.

It is to be appreciated that all references herein to treatment include curative, palliative and prophylactic treatment. The description, which follows, concerns the therapeutic applications to which the compounds of formula (1) may be put.

The compounds of formula (1) have the ability to interact with the β2 receptor and thereby have a wide range of therapeutic applications, as described further below, because of the essential role which the β2 receptor plays in the physiology of all mammals.

Therefore, a further aspect of the present invention relates to the compounds of formula (1), or pharmaceutically acceptable salts, derived forms or compositions thereof, for use in the treatment of diseases, disorders, and conditions in which the β2 receptor is involved. More specifically, the present invention also concerns the compounds of formula (1), or pharmaceutically acceptable salts, derived forms or compositions thereof, for use in the treatment of diseases, disorders, and conditions selected from the group consisting of:
- asthma of whatever type, etiology, or pathogenesis, in particular asthma that is a member selected from the group consisting of atopic asthma, non-atopic asthma, allergic asthma, atopic bronchial IgE-mediated asthma, bronchial asthma, essential asthma, true asthma, intrinsic asthma caused by pathophysiologic disturbances, extrinsic asthma caused by environmental factors, essential asthma of unknown or inapparent cause, non-atopic asthma, bronchitic asthma, emphysematous asthma, exercise-induced asthma, allergen induced asthma, cold air induced asthma, occupational asthma, infective asthma caused by bacterial, fungal, protozoal, or viral infection, non-allergic asthma, incipient asthma, wheezy infant syndrome and bronchiolytis,
- chronic or acute bronchoconstriction, chronic bronchitis, small airways obstruction, and emphysema,
- obstructive or inflammatory airways diseases of whatever type, etiology, or pathogenesis, in particular an obstructive or inflammatory airways disease that is a member selected from the group consisting of chronic eosinophilic pneumonia, chronic obstructive pulmonary disease (COPD), COPD that includes chronic bronchitis, pulmonary emphysema or dyspnea associated or not associated with COPD, COPD that is characterized by irreversible, progressive airways obstruction, adult respiratory distress syndrome (ARDS), exacerbation of airways hyper reactivity consequent to other drug therapy and airways disease that is associated with pulmonary hypertension,
- bronchitis of whatever type, etiology, or pathogenesis, in particular bronchitis that is a member selected from the group consisting of acute bronchitis, acute laryngotracheal bronchitis, arachidic bronchitis, catarrhal bronchitis, croupus bronchitis, dry bronchitis, infectious asthmatic bronchitis, productive bronchitis, staphylococcus or streptococcal bronchitis and vesicular bronchitis,
- acute lung injury,
- bronchiectasis of whatever type, etiology, or pathogenesis, in particular bronchiectasis that is a member selected from the group consisting of cylindric bronchiectasis, sacculated bronchiectasis, fusiform bronchiectasis, capillary bronchiectasis, cystic bronchiectasis, dry bronchiectasis and follicular bronchiectasis.

A still further aspect of the present invention also relates to the use of the compounds of formula (1), or pharmaceutically acceptable salts, derived forms or compositions thereof, for the manufacture of a drug having a β2 agonist activity. In particular, the present inventions concerns the use of the compounds of formula (1), or pharmaceutically acceptable salts, derived forms or compositions thereof, for the manufacture of a drug for the treatment of 2-mediated diseases and/or conditions, in particular the diseases and/or conditions listed above.

As a consequence, the present invention provides a particularly interesting method for preparing a medicament to treat a mammal, including a human being, with an effective amount of a compound of formula (1), or a pharmaceutically acceptable salt, derived form or composition thereof. More precisely, the present invention provides a particularly interesting methods for preparing a medicament for the treatment of a β2-mediated diseases and/or conditions in a mammal, including a human being, in particular the diseases and/or conditions listed above, comprising admidministering said mammal with an effective amount of a compound of formula (1), its pharmaceutically acceptable salts and/or derived forms.

The following examples illustrate the preparation of the compounds of the formula (1):

### Example 1: 4-{[2-(3-{2-[(2R)-2-(6-Amino-pyridin-3-yl)-2-hydroxy-ethylamino]-propyl}-phenyl)-acetylamino]-methyl}-N-(3,4-dimethoxybenzyl)-benzamide

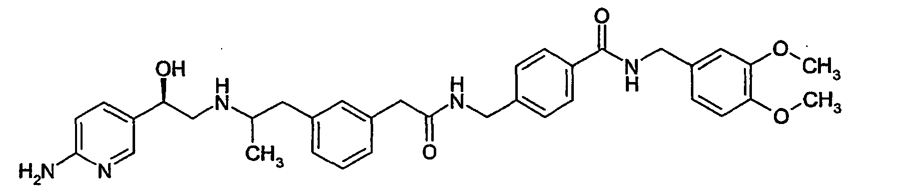

A solution of *N*-(3,4-dimethoxy-benzyl)-4-({2-[3-(2-{(2*R*)-2-[6-(2,5-dimethyl-pyrrol-1-yl)-pyridin-3-yl]-2-hydroxy-ethylamino}-propyl)-phenyl]-acetylamino}-methyl)-benzamide (48mg, 0.07mmol) in ethanol (1ml) was treated with hydroxylamine hydrochloride (24mg, 0.35mmol) and the resulting mixture heated in a Reactivial^{™} at 80°C for 16 hours. The reaction mixture was cooled, passed through a Strong Cation Exchange column eluting with methanol and then 2N ammonia in methanol to elute the product. Further purification by flash column chromatography eluting with dichloromethane:methanol:880 ammonia (97:3:0.5 changing to 90:10:1 by volume) gave the title compound (28mg)as a pale yellow foam.
¹H NMR (400MHz, CD₃OD): δ = 7.87-7.63 (3H, m), 7.44-6.78 (10H, m), 6.55-6.42 (1H, m), 4.58-4.50 (1H, m), 4.48 (2H, s), 4.41 (2H, s), 3.80 (6H, s), 3.57-3.36 (2H, m), 3.07-2.43 (5H, m), 1.08-0.88 (3H, m) ppm.
LRMS (electrospray) : m/z [M+H]⁺ 612, [M+Na]⁺ 634.

### Example 2 : 4-{[2-(3-{2-[(2R)-2-(6-Amino-pyridin-3-yl)-2-hydroxy-ethylamino]-propyl}-phenyl)-acetylamino]-methyl}-N-(4-sulfamoyl-benzyl)-benzamide

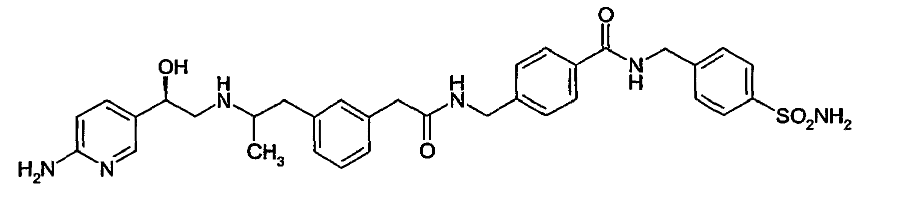

Prepared using the method for example 1 using 4-({2-[3-(2-{(2R)-2-[6-(2,5-dimethyl-pyrrol-1-yl)-pyridin-3-yl]-2-hydroxy-ethylamino}-propyl)-phenyl]-acetylamino}-methyl)-*N*-(4-sulfamoyl-benzyl)-benzamide (preparation 2) to give the title compound (1:1 mixture of diastereoisomers) as a pale yellow foam.
¹H NMR (400MHz, CD₃OD): δ = 7.97-7.76 (5H, m), 7.56-7.46 (2H, m), 7.46-7.00 (7H, m), 6.59-6.47 (1H, m), 4.62 (2H, s), 4.59-4.50 (1H, m), 4.42 (2H, s), 3.59-3.51 (2H, m), 2.99-2.55 (5H, m), 1.09-0.97 (3H, m) ppm.
LRMS (electrospray) : m/z [M+H]⁺ 631, [M+Na]⁺ 653.

### Example 3 : 4-{[2-(3-{2-[(2R)-2-(6-Amino-pyridin-3-yl)-2-hydroxy-ethylamino]-propyl}-phenyl)-acetylamino]-methyl}-N-(2-ethoxy-benzyl)-benzamide

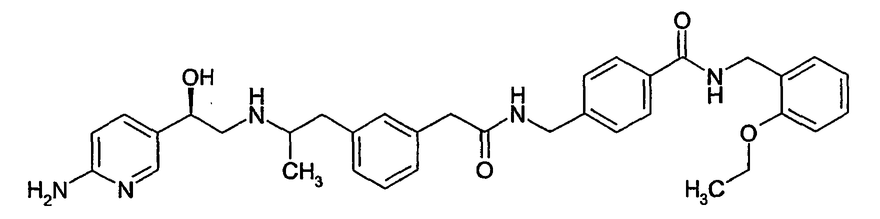

Prepared using the method for example 1 using 4-({2-[3-(2-{(2*R*)-2-[6-(2,5-dimethyl-pyrrol-1-yl)-pyridin-3-yl]-2-hydroxy-ethylamino}-propyl)-phenyl]-acetylamino}-methyl)-*N*-(2-ethoxy-benzyl)-benzamide (preparation 3) to give the title compound (1:1 mixture of diastereoisomers) as a pale yellow foam.
¹H NMR (400MHz, CD₃OD): δ = 7.87-7.72 (3H, m), 7.46-6.82 (11H, m), 6.58-6.46 (1H, m), 4.57 (2H, s), 4.54-4.47 (1H, m), 4.41 (2H, s), 4.08 (2H, q), 3.57-3.49 (2H, m), 2.95-2.51 (5H, m), 1.39 (3H, t), 1.07-0.93 (3H, m) ppm.
LRMS (electrospray) : m/z [M+H]⁺ 596, [M+Na]⁺ 618.

### Example 4 : 4-{[2-(3-{2-[(2R)-2-(6-Amino-pyridin-3-yl)-2-hydroxy-ethylamino]-propyl}-phenyl)-acetylamino]-methyl}-N-[(1R)-1-(4-methoxyphenyl)-ethyl]-benzamide

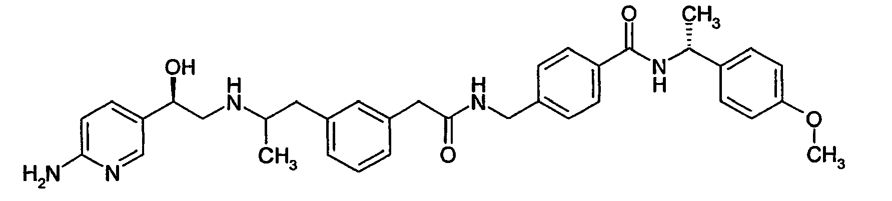

Prepared using the method for example 1 using 4-{[({3-[2-({(2*R*)-2-[6-(2,5-dimethyl-1*H*-pyrrol-1-yl)-3-pyridinyl]-2-hydroxyethyl}amino)propyl]phenyl} acetyl)amino]methyl}-*N*-[(1*R*)-1-(4-methoxypheny))ethy)]benzamide (preparation 4) to give the title compound (1:1 mixture of diastereoisomers) as a pale yellow foam.
¹H NMR (400MHz, CD₃OD): δ = 7.83-7.71 (3H, m), 7.43-7.00 (9H, m), 6.92-6.83 (2H, m), 6.56-6.46 (1H, m), 5.25-5.12 (1H, m), 4.58-4.46 (1H, m), 4.40 (2H, s), 3.76 (3H, s), 3.56-3.46 (2H, m), 2.96-2.43 (5H, m), 1.54-1.39 (3H, m), 1.08-0.89 (3H, m) ppm.
LRMS (electrospray) : m/z [M+H]⁺ 612, [M+Na]⁺ 634.

### Example 5 : 4-{[2-(3-{2-[(2R)-2-(6-Amino-pyridin-3-yl)-2-hydroxy-ethylamino]-propyl}-phenyl)-acetylamino]-methyl}-N-((1R)-1-hydroxymethyl-2-phenyl-ethyl)-benzamide

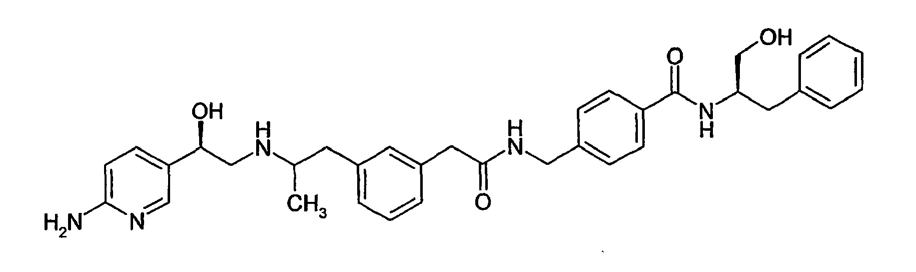

Prepared using the method for example 1 using 4-({2-[3-(2-{(2*R*)-2-[6-(2,5-dimethyl-pyrrol-1-yl)-pyridin-3-yl]-2-hydroxy-ethylamino}-propyl)-phenyl]-acetylamino}-methyl)-*N*-((1*R*)-1-hydroxymethyl-2-phenyl-ethyl)-benzamide (preparation 5) to give the title compound (1:1 mixture of diastereoisomers) as a pale yellow foam.
¹H NMR (400MHz, CD₃OD): δ = 7.83-7.76 (1H, m), 7.71-7.61 (2H, m), 7.44-7.33 (1H, m), 7.32-7.00 (11H, m), 6.57-6.47 (1H, m), 4.57-4.52 (1H, m), 4.39 (2H, s), 4.35-4.27 (1H, m), 3.67-3.56 (2H, s), 3.56-3.49 (2H, m), 3.04-2.56 (7H, m), 1.08-0.94 (3H, m) ppm.
LRMS (electrospray) : m/z [M+H]⁺ 612, [M+Na]⁺ 634.

### Example 6: 4-{[2-(3-{2-[(2R)-2-(6-Amino-pyridin-3-yl)-2-hydroxy-ethylamino]-propyl}-phenyl)-acetylamino]-methyl}-N-((1R, 2S)-2-hydroxy-1-methyl-2-phenyl-ethyl)-benzamide

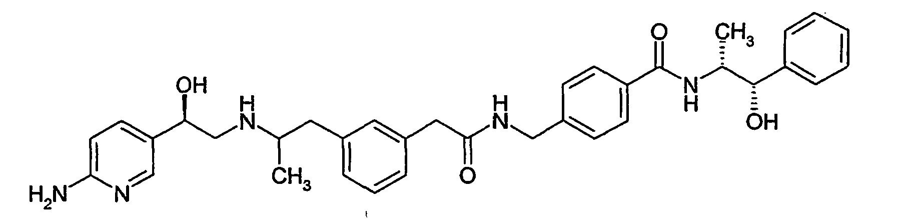

Prepared using the method for example 1 using 4-({2-[3-(2-{(2*R*)-2-[6-(2,5-dimethyl-pyrrol-1-yl)-pyridin-3-yl]-2-hydroxy-ethylamino}-propyl)-phenyl]-acetylamino}-methyl)-*N-*((1*R*,2*S*)-2-hydroxy-1-methyl-2-phenyl-ethyl)-benzamide (preparation 6) to give the title compound (1:1 mixture of diastereoisomers) as a pale yellow foam.
¹H NMR (400MHz, CD₃OD): δ = 7.82-7.76 (1H, m), 7.67-7.60 (2H, m), 7.44-7.01 (12H, m), 6.55-6.49 (1H, m), 4.56-4.51 (1H, m), 4.39 (2H, s), 4.36-4.27 (1H, m), 3.57-3.51 (2H, m), 3.36-3.24 (1H, m, partially obscured by solvent) 3.07-2.50 (5H, m), 1.22-1.13 (3H, m), 1.13-0.96 (3H, m) ppm.
LRMS (electrospray) : m/z [M+H]⁺ 612, [M+Na]⁺ 634.

### Example 7: 4-{[2-(3-{2-[(2R)-2-(6-Amino-pyridin-3-yl)-2-hydroxy-ethylamino]-propyl}-phenyl)-acetylamino]-methyl}-N-((1S, 2R)-2-hydroxy-1-methyl-2-phenyl-ethyl)-benzamide

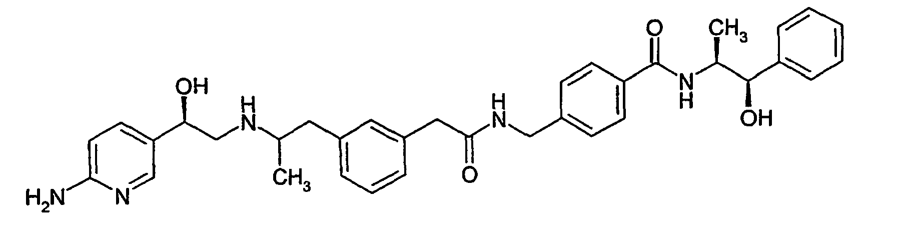

Prepared using the method for example 1 using 4-({2-[3-(2-{(2*R*)-2-[6-(2,5-dimethyl-pyrrol-1-yl)-pyridin-3-yl]-2-hyd roxy-ethylamino}-propyl)-phenyl]-acetylamino}-methyl)-*N*-((1*S*,2*R*)-2-hydroxy-1-methyl-2-phenyl-ethyl)-benzamide (preparation 7) to give the title compound (1:1 mixture of diastereoisomers) as a pale yellow foam.
¹H NMR (400MHz, CD₃OD): δ = 7.82-7.76 (1H, m), 7.67-7.60 (2H, m), 7.44-7.01 (12H, m), 6.55-6.49 (1H, m), 4.56-4.51 (1H, m), 4.39 (2H, s), 4.36-4.27 (1H, m), 3.57-3.51 (2H, m), 3.36-3.24 (1H, m, partially obscured by solvent) 3.07-2.50 (5H, m), 1.22-1.13 (3H, m), 1.13-0.96 (3H, m) ppm.
LRMS (electrospray) : m/z [M+H]⁺ 612, [M+Na]⁺ 634.

### Example 8 : 4-{[2-(3-{2-[(2R)-2-(6-Amino-pyridin-3-yl)-2-hydroxy-ethylamino]-propyl}-phenyl)-acetytamino]-methyl}-N-[2-(3-methoxyphenyl)-ethyl]-benzamide

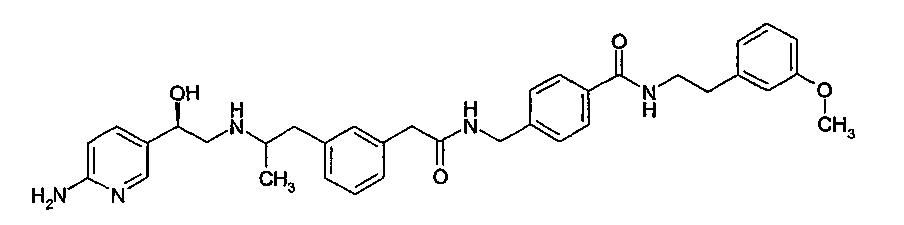

Prepared using the method for example 1 using 4-({2-[3-(2-{(2*R*)-2-[6-(2,5-dimethyl-pyrrol-1-yl)-pyridin-3-yl]-2-hydroxy-ethylamino}-propyl)-phenyl]-acetylamino}-methyl)-*N*-[2-(3-methoxy-phenyl)-ethyl]-benzamide (preparation 8) to give the title compound (1:1 mixture of diastereoisomers) as a pale yellow foam.
¹H NMR (400MHz, CD₃OD): δ = 7.84-7.76 (1H, m), 7.75-7.66 (2H, m), 7.44-7.00 (8H, m), 6.85-6.78 (2H, m), 6.78-6.72 (1H, m), 6.55-6.45 (1H, m), 4.59-4.49 (1H, m), 4.41 (2H, s), 3.74(3H, s), 3.62-3.52 (4H, m), 3.54-3.22 (2H, m, partially obscured by solvent), 3.00-2.54 (5H, m), 1.08-0.97 (3H, m) ppm.
LRMS (electrospray) : m/z [M+H]⁺ 612, [M+Na]⁺ 634.

### Example 9 : 4-{[2-(3-{(2R)-2-[(2R)-2-(6-Amino-pyridin-3-yl)-2-hydroxy-ethylamino]-propyl}-phenyl)-acetylamino]-methyl}-N-(3,4-dimethoxybenzyl)-benzamide

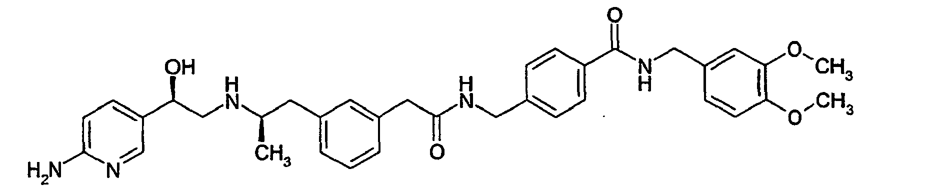

Prepared using the method for example 1 using *N*-(3,4-dimethoxy-benzyl)-4-({2-[3-((2*R*)-2-{(2*R*)-2-[6-(2,5-dimethyl-pyrrol-1-yl)-pyridin-3-yl]-2-hydroxy-ethylamino}-propyl)-phenyl]-acetylamino}-methyl)-benzamide (preparation 9) to give the title compound as a colourless foam.
¹H NMR (400MHz, CD₃OD): δ = 7.77 (2H, s), 7.75 (1H, s), 7.35 (1H, d), 7.29 (2H, d), 7.18 (1H, d), 7.14 (1H, d), 7.08 (1H, s), 7.02 (1H, d), 6.96 (1H, s), 6.89 (2H, s), 6.49 (1H, d), 4.52 (1H, m), 4.49 (2H, s), 4.41 (2H, s), 3.81 (6H, s), 3.54 (2H, s), 2.91-2.82 (2H, m), 2.67-2.60 (3H, m), 1.07 (3H, d) ppm.
LRMS (APCI) : m/z [M+H]⁺ 612.
Optical Rotation [α]^{D}₂₅= -23.00° c=1, methanol

### Example 10 : 4-{[2-(3-{(2R)-2-[(2R)-2-(6-Amino-pyridin-3-yl)-2-hydroxy-ethylamino]-propyl}-phenyl)-acetylamino]-methyl}-N-(2-ethoxy-benzyl)-benzamide

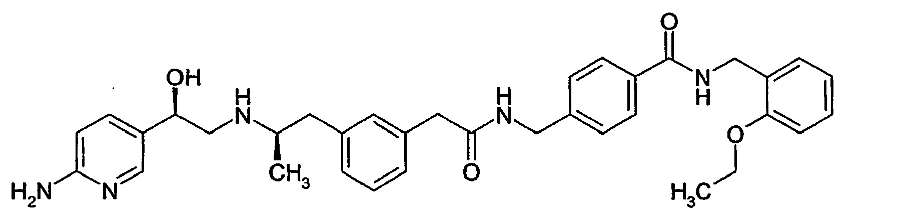

Prepared using the method for example 1 using 4-({2-[3-((2*R*)-2-{(2*R*)-[6-(2,5-dimethyl-pyrrol-1-yl)-pyridin-3-yl]-2-hydroxy-ethylamino}-propyl)-phenyl]-acetylamino}-methyl)-*N*-(2-ethoxy-benzyl)-benzamide (preparation 10) to give the title compound as a colourless foam.
¹H NMR (400MHz, CD₃OD): δ = 7.78 (3H, m), 7.36 (1H, d), 7.32 (2H, d), 7.23-7.18 (3H, m), 7.15 (1H, d), 7.09 (1H, s), 7.03 (1H, d), 6.94 (1H, d), 6.88 (1H, m), 6.50 (1H, d), 4.57 (2H, s), 4.52 (1H, m), 4.42 (2H, s), 4.10 (2H, q), 3.54 (2H, s), 2.93-2.82 (2H, m), 2.71-2.57 (3H, m), 1.42 (3H, t), 1.06 (3H, d) ppm.
LRMS (APCI) : m/z [M+H]⁺ 596.
Optical Rotation [α]^{D}₂₅ = -22.80° c=1, methanol

### Example 11 : 4-{[2-(3-{(2R)-2-[(2R)-2-(6-Amino-pyridin-3-yl)-2-hydroxy-ethylamino]-propyl}-phenyl)-acetylamino]-methyl}-N-(4-hydroxy-3-methoxy-benzyl)-benzamide

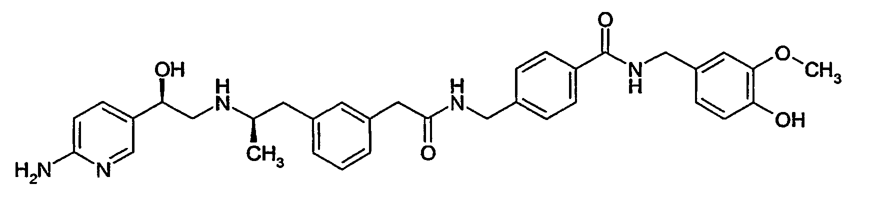

Prepared using the method for example 1 using 4-({2-[3-((2*R*)-2-{(2*R*)-2-[6-(2,5-dimethyl-pyrrol-1-yl)-pyridin-3-yl]-2-hydroxy-ethylamino}-propyl)-phenyl]-acetylamino}-methyl)-*N*-(4-hydroxy-3-methoxy-benzyl)-benzamide (preparation 11) to give the title compound as a colourless foam.
¹H NMR (400MHz, CD₃OD): δ = 7.76 (2H, s), 7.74 (1H, s), 7.36 (1H, d), 7.30 (2H, d), 7.19-6.93 (5H, m), 6.75 (2H, m), 6.50 (1H, d), 4.52 (1H, m), 4.46 (2H, s), 4.40 (2H, s), 3.82 (3H, s), 3.53 (2H, s), 2.90-2.81 (2H, m), 2.70-2.59 (3H, m), 1.06 (3H, d) ppm.
LRMS (APCl) : m/z [M+H]⁺ 598.
Optical Rotation [α]^{D}₂₅ = -23.0° c=1, methanol

### Example 12 : 4-{[2-(3-{(2R)-2-[(2R)-2-(6 Amino-pyridin-3-yl)-2-hydroxy-ethylamino]-propyl}-phenyl)-acetylamino]-methyl}-N-(4-sulfamoyl-benzyl)-benzamide

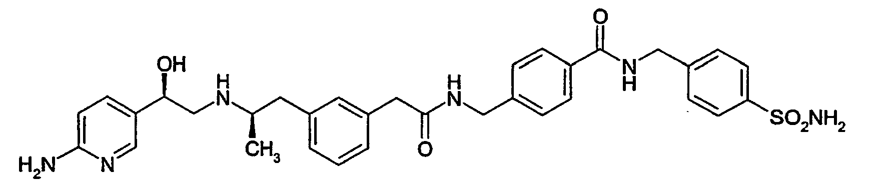

Prepared using the method for example 1 using 4-({2-[3-((2*R*)-2-{(2*R*)-2-[6-(2,5-dimethyl-pyrrol-1-yl)-pyridin-3-yl]-2-hydroxy-ethylamino}-propyl)-phenyl]-acetylamino}-methyl)-*N-*(4-sulfamoyl-benzyl)-benzamide (preparation 12) to give the title compound as a colourless foam.
¹H NMR (400MHz, CD₃OD): δ = 7.85 (2H, d), 7.80 (1H, s), 7.77 (2H, m), 7.49 (2H, d), 7.35 (1H, m), 7.32 (2H, d), 7.20 (1H, m), 7.14 (1H, d), 7.09 (1H, s), 7.02 (1H, s), 6.49 (1H, d), 4.61 (2H, s), 4.52 (1H, m), 4.41 (2H, s), 3.53 (2H, s), 2.92-2.82 (2H, m), 2.70-2.56 (3H, m), 1.05 (3H, d) ppm.
LRMS (APCI) : m/z [M+H]⁺ 631.
Optical Rotation [α]^{D}₂₅ = -31.0° c=1, methanol

### Example 13 : 4-{[2-(3-{(2R)-2-[(2R)-2-(6-Amino-pyridin-3-yl)-2-hydroxy-ethylamino]-propyl}-phenyl)-acetylamino]-methyl}-N-((1R, 2S)-2-hydroxy-1-methyl-2-phenyl-ethyl)-benzamide

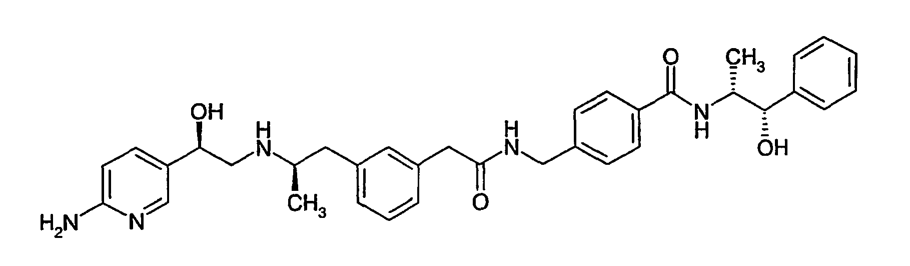

Prepared using the method for example 1 using 4-({2-[3-((2*R*)-2-{(2*R*)-2-[6-(2,5-dimethyl-pyrrol-1-yl)-pyridin-3-yl]-2-hydroxy-ethylamino}-propyl)-phenyl]-acetylamino}-methyl)-*N*-((1*R*,2*S*)-2-hydroxy-1-methyl-2-phenyl-ethyl)-benzamide (preparation 13) to give the title compound as a orange oil.
¹H NMR (400MHz, CD₃OD): δ = 7.78 (1H, s), 7.63 (2H, d), 7.43 (2H, d), 7.37 (1H, d), 7.32-7.19 (6H, m), 7.15 (1H, d), 7.09 (1H, s), 7.03 (1H, d), 6.51 (1H, d), 4.79 (1H, d), 4.53 (1H, m), 4.39 (2H, s), 4.32 (1H, m), 3.53 (2H, s), 2.94-2.82 (2H, m), 2.71-2.57 (3H, m), 1.19 (3H, d), 1.07 (3H, d) ppm.
LRMS (APCI) : m/z [M+H]⁺ 596.
Optical Rotation [α]^{D}₂₅ = -54.41° c=1, methanol

### Example 14: N-(4-{[2-(3-{(2R)-2-[(2R)-2-(6 Amino-pyridin-3-yl)-2-hydroxy-ethylamino]-propyl}-phenyl)-acetylamino]-methyl}-benzyl)-2,2-diphenylacetamide

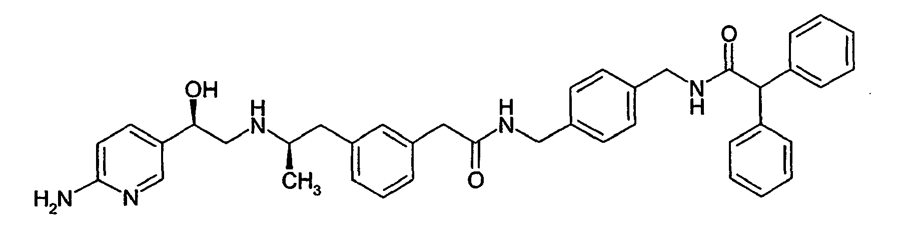

Prepared using the method for example 1 using *N-*[4-({2-[3-((2*R*)-2-{(2*R*)-2-[6-(2,5-dimethyl-pyrrol-1-yl)-pyridin-3-yl]-2-hydroxy-ethylamino}-propyl)-phenyl]-acetylamino}-methyl)-benzyl]-2,2-diphenyl-acetamide (preparation 14) to give the title compound as a colourless foam.
¹H NMR (400MHz, CD₃OD): δ = 7.79 (1H, bs), 7.37-7.01 (19H, m), 6.49 (1H, d), 4.99 (1H, s), 4.57-4.53 (1H, m), 4.34 (2H, s), 4.30 (2H, s), 3.50 (2H, s), 2.97-2.82 (2H, m), 2.73-2.55 (3H, m), 1.06 (3H, d) ppm.
LRMS (electrospray): m/z [M+H]⁺ 642, [M+Na]⁺ 664.

### Example 15 : 2-(3-{(2R)-2-[(2R)-2-(6-Amino-pyridin-3-yl)-2-hydroxy-ethylamino]-propyl}-phenyl)-N-{4-[(benzhydryl-amino)-methyl]-benzyl}-acetamide

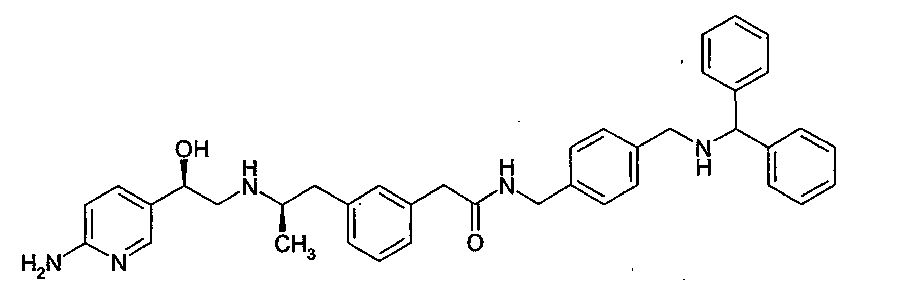

Prepared using the method for example 1 using *N*-{4-[(benzhydryl-amino)-methyl]-benzyl}-2-[3-((2*R*)-2-{(2*R*)-2-[6-(2,5-dimethyl-pyrrol-1-yl)-pyridin-3-yl]-2-hydroxy-ethylamino}-propyl)-phenyl]-acetamide (preparation 15) to give the title compound as a colourless foam.
¹H NMR (400MHz, CD₃OD): δ = 7.77 (1H, bs), 7.36-7.06 (18H, m), 7.01 (1H, d), 6.48 (1H, d), 4.75 (1H, s), 4.50 (1H, m), 4.34 (2H, s), 3.64 (2H, s), 3.51 (2H, s), 2.90-2.80 (2H, m), 2.69-2.54 (3H, m), 1.04 (3H, d) ppm.
LRMS (electrospray) : m/z [M+H]⁺ 614, [M+Na]⁺ 636.

### Example 16: 4-{[2-(3-{(2R)-2-[(2R)-2-(6-Amino-pyridin-3-yl)-2-hydroxy-ethylamino]-propyl}-phenyl)-acetylamino]-methyl}-N-(2-benzyloxy-ethyl)-benzamide

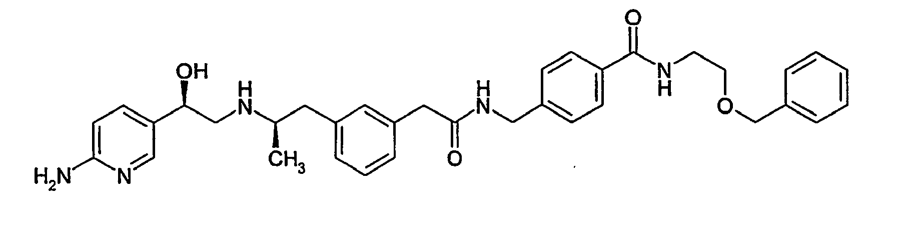

Prepared using the method for example 1 using *N*-(2-benzyloxy-ethyl)-4-({2-[3-((2*R*)-2-{(2*R*)-2-[6-(2,5-dimethyl-pyrrol-1-yl)-pyridin-3-yl]-2-hydroxy-ethylamino}-propyl)-phenyl]-acetylamino}-methyl)-benzamide (preparation 16) to give the title compound as a colourless foam.
¹H NMR (400MHz, CD₃OD): δ = 7.79 (1H, s), 7.73 (2H, d), 7.40-7.02 (12H, m), 6.50 (1H, d), 4.59-4.54 (3H, m), 4.41 ((2H, s), 3.65-3.62 (2H, m), 3.57-3.54 (4H, m), 3.02-2.85 (2H, m), 2.75-2.56 (3H, m), 1.07 (3H, d) ppm.
LRMS (electrospray) : m/z [M+H]⁺ 596, [M+Na]⁺ 618.

### Example 17 : 4-{[2-(3-{(2R)-2-[(2R)-2-(6-Amino-pyridin-3-yl)-2-hydroxy-ethylamino]-propyl}-phenyl)-acetylamino]-methyl}-N-[2-(3-phenyl-propoxy)-ethyl]-benzamide

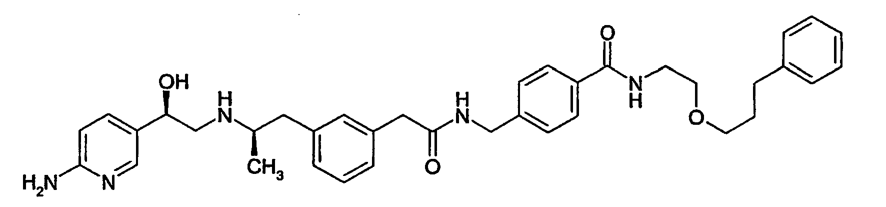

Prepared using the method for example 1 using 4-({2-[3-((2*R*)-2-{(2*R*)-2-[6-(2,5-dimethyl-pyrrol-1-yl)-pyridin-3-yl]-2-hydroxy-ethylamino}-propyl)-phenyl]-acetylamino}-methyl)-*N*-[2-(3-phenyl-propoxy)-ethyl]-benzamide (preparation 17) to give the title compound as a colourless foam.
¹H NMR (400MHz, CD₃OD): δ = 7.77-7.74 (3H, m), 7.37-7.00 (12H, m), 6.49 (1H, d), 4.53-4.50 (1H, m), 4.40 (2H, s), 3.58-3.53 (6H, m), 3.47-3.44 (2H, m), 2.93-2.81 (2H, m), 2.64-2.55 (5H, m), 1.89-1.81 (2H, m), 1.05 (3H, d) ppm.

### Example 18 : 4-{(2-(3-{(2R)-2-[(2R)-2-(6-Amino-pyridin-3-yl)-2-hydroxy-ethylamino]-propyl}-phenyl)-acetylamino]-methyl}-N-[2-(naphthalen-1-ylmethoxy)-ethyl]-benzamide

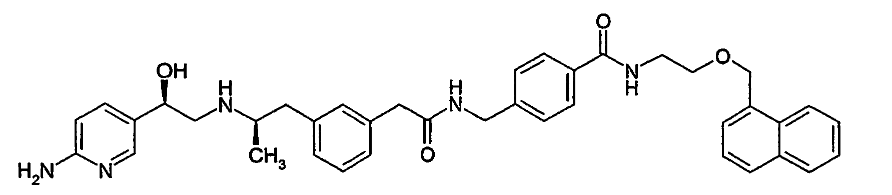

Prepared using the method for example 1 using 4-({2-[3-((2R)-2-{(2R)-2-[6-(2,5-dimethyl-pyrrol-1-yl)-pyridin-3-yl]-2-hydroxy-ethylamino}-propyl)-phenyl]-acetylamino}methyl)-*N*-[2-(naphthalen-1-ylmethoxy)-ethyl]-benzamide (preparation 18) to give the title compound as a colourless foam.
¹H NMR (400MHz, CD₃OD): δ = 8.12 (1H, d), 7.83-7.77 (3H, m), 7.63 (2H, d), 7.50-7.31 (5H, m), 7.27 (2H, d), 7.22-7.12 (2H, m), 7.10 (1H, bs), 7.02 (1H, d), 6.48 (1H, d), 4.99 (2H, s), 4.56-4.51 (1H, m), 4.41 (2H, s), 3.71 (2H, t), 3.57 (2H, t), 3.54 (2H, s) 2.94-2.81 (2H, m), 2.70-2.54 (3H, m), 1.04 (3H, d) ppm.

### Example 19 : 4-{[2-(3-{(2R)-2-[(2R)-2-(6-Amino-pyridin-3-yl)-2-hydroxy-ethylamino]-propyl}-phenyl)-acetylamino]-methyl}-N-(3-benzylsulfamoyl-benzyl)-benzamide

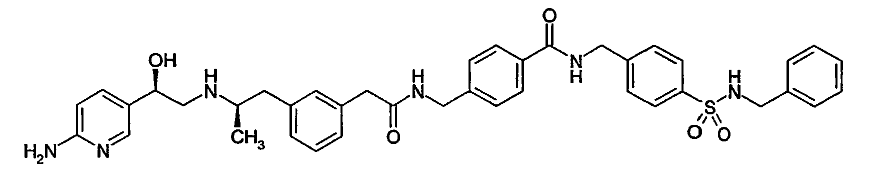

Prepared using the method for example 1 using *N*-(4-benzylsulfamoyl-benzyl)-4-({2-[3-((2*R*)-2-{(2*R*)-2-[6-(2,5-dimethyl-pyrrol-1-yl)-pyridin-3-yl]-2-hydroxy-ethylamino}-propyl)-phenyl]-acetylamino}-methyl)-benzamide (preparation 19) to give the title compound as a colourless foam.
¹H NMR (400MHz, CD₃OD): δ = 7.81-7.76 (5H, m), 7.49 (2H, d), 7.36-7.32 (3H, m), 7.22-7.13 (7H, m), 7.09 (1H, s), 7.03 (1H, d), 6.50 (1H, d), 4.61 (2H, s), 4.52 (1H, m), 4.42 (2H, s), 4.03 (2H, s), 3.54 (2H, s), 2.92-2.81 (2H, m), 2.70 (3H, m), 1.06 (3H, d) ppm.
LRMS (APCI) : m/z [M+H]⁺ 721.

### Example 20 : 4-{[2-(3-{(2R)-2-[(2R)-2-(6-Amino-pyridin-3-yl)-2-hydroxy-ethylamino]-propyl}-phenyl)-acetylamino]-methyl}-N-(4-methylsulfamoyl-benzyl)-benzamide

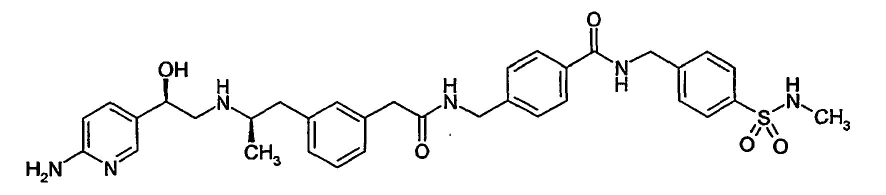

Prepared using the method for example 1 using 4-({2-[3-((2*R*)-2-{(2*R*)-2-[6-(2,5-dimethyl-pyrrol-1-yl)-pyridin-3-yl]-2-hydroxy-ethylamino}-propyl)-phenyl]-acetylamino}-methyl)-*N*-(4-methylsulfamoyl-benzyl)-benzamide (preparation 20) to give the title compound as a colourless foam.
¹H NMR (400MHz, CD₃OD): δ = 7.80-7.76 (5H, m), 7.54 (2H, d), 7.37-7.31 (3H, m), 7.22-7.01 (4H, m), 6.50 (1H, d), 4.63 (2H, s), 4.52 (1H, m), 4.41 (2H, s), 3.54 (2H, s), 2.91-2.81 (2H, m0, 2.66-2.59 (3H, m), 2.50 (3H, s), 1.05 (3H, d) ppm.
LRMS (APCI) : m/z [M+H]⁺ 645.

### Example 21 : 4-{[2-(3-{(2R)-2-[(2R)-2-(6-Amino-pyridin-3-yl)-2-hydroxy-ethylamino]-propyl}-phenyl)-acetylamino]-methyl}-N-(4-ethylsulfamoyl-benzyl)-benzamide

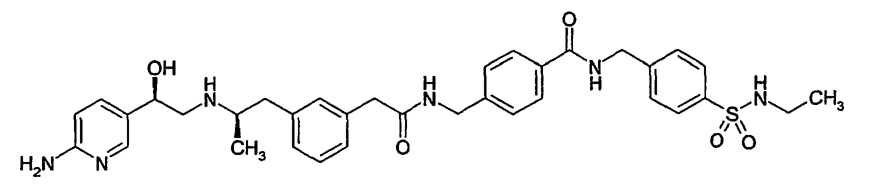

Prepared using the method for example 1 using 4-({2-[3-((2*R*)-2-{(2*R*)-2-[6-(2,5-dimethyl-pyrrol-1-yl)-pyridin-3-yl]-2-hydroxy-ethylamino}-propyl)-phenyl]-acetylamino}-methyl)-*N-*(4-ethylsulfamoyl-benzyl)-benzamide (preparation 21) to give the title compound as a colourless foam.
¹H NMR (400MHz, CD₃OD): δ = 7.80-7.76 (5H, m), 7.53 (2H, d), 7.36-7.31 (3H, m), 7.20-7.01 (4H, m), 6.50 (1H, d), 4.63 (2H, s), 4.52 (1H, m), 4.41 (2H, s), 3.54 (2H, s), 2.89-2.81 (4H, m), 2.66-2.59 (3H, m), 1.06-1.02 (6H, m) ppm.
LRMS (APCI) : m/z [M+H]⁺ 659.

### Example 22 : 4-{[2-(3-{(2R)-2-[(2R)-2-(6-Amino-pyridin-3-yl)-2-hydroxy-ethylamino]-propyl}-phenyl)-acetylamino]-methyl}-N-(3-benzylsulfamoyl-benzyl)-benzamide

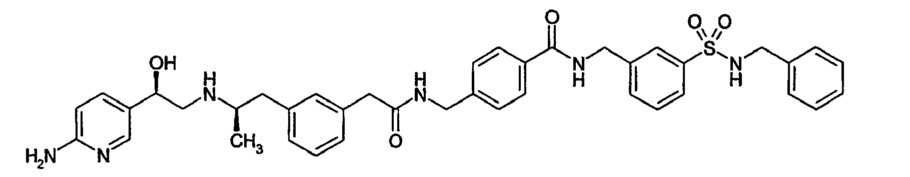

Prepared using the method for example 1 using *N*-(3-benzylsulfamoyl-benzyl)-4-({2-[3-((2*R*)-2-{(2*R*)-2-[6-(2,5-dimethyl-pyrrol-1-yl)-pyridin-3-yl]-2-hydroxy-ethylamino}-propyl)-phenyl]-acetylamino}-methyl)-benzamide (preparation 22) to give the title compound as a colourless foam.
¹H NMR (400MHz, CD₃OD): δ = 7.82-7.77 (4H, m), 7.72 (1H, d), 7.58 (1H, d), 7.48 (1H, m), 7.36-7.30 (3H, m), 7.21-7.14 (7H, m), 7.09 (1H, s), 7.02 (1H, d), 6.50 (1H, d), 4.59 (2H, s), 4.51 (1H, m), 4.41 (2H, s), 4.02 (2H, s), 3.53 (2H, s), 2.89-2.80 (2H, m), 2.65-2.58 (3H, m), 1.05 (3H, d) ppm.
LRMS (APCI) : m/z [M+H]⁺ 721.

### Example 23 : 4-{[2-(3-{(2R)-2-[(2R)-2-(6-Amino-pyridin-3-yl)-2-hydroxy-ethylamino]-propyl}-phenyl)-acetylamino]-methyl}-N-(3-methylsulfamoyl-benzyl)-benzamide

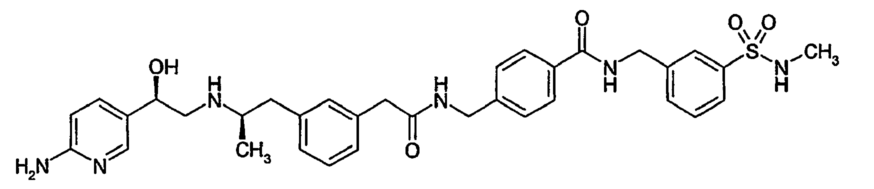

Prepared using the method for example 1 using 4-({2-[3-((2*R*)-2-{(2*R*)-2-[6-(2,5-dimethyl-pyrrol-1-yl)-pyridin-3-yl]-2-hydroxy-ethylamino}-propyl)-phenyl]-acetylamino}-methyl)-*N*-(3-methylsulfamoyl-benzyl)-benzamide (preparation 23) to give the title compound as a colourless foam.
¹H NMR (400MHz, CD₃OD): δ = 7.82-7.77 (4H, m), 7.73 (1H, d), 7.59 (1H. m), 7.53 (1H, m), 7.37-7.30 (3H, m), 7.22-7.01 (4H, m), 6.50 (1H, d), 4.63 (2H, s), 4.51 (1H, m), 4.41 (2H, s), 3.53 (2H, s), 2.91-2.81 (2H, m), 2.70-2.59 (3H, m), 2.50 (3H, s), 1.06 (3H, d) ppm.
LRMS (APCl) : m/z [M+H]⁺ 645.

### Example 24 : 4-{[2-(3-{(2R)-2-[(2R)-2-(6-Amino-pyridin-3-yl)-2-hydroxy-ethylamino]-propyl}-phenyl)-acetylamino]-methyl)-N-(3-ethylsulfamoyl-benzyl)-benzamide

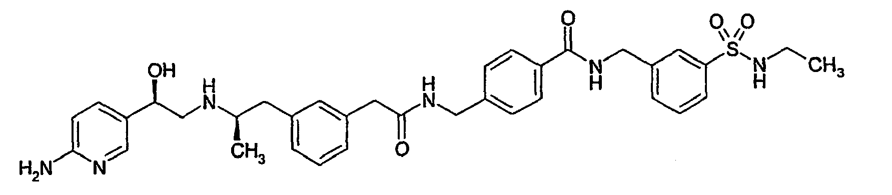

Prepared using the method for example 1 using 4-({2-[3-((2*R*)-2-{(2*R*)-2-[6-(2,5-dimethyl-pyrrol-1-yl)-pyridin-3-yl]-2-hydroxy-ethylamino}-propyl)-phenyl]-acetylamino}-methyl)-*N*-(3-ethylsulfamoyl-benzyl)-benzamide (preparation 24) to give the title compound as a colourless foam.
¹H NMR (400MHz, CD₃OD): δ = 7.83 (1H, s), 7.77 (3H, m), 7.73 (1H, d), 7.59 (1H, d), 7.51 (1H, m), 7.37-7.30 (3H, m), 7.20-7.01 (4H, m), 6.50 (1H, d), 4.62 (2H, s), 4.52 (1H, m), 4.41 (2H, s), 3.53 (2H, s), 2.90-2.81 (4H, m), 2.66-2.55 (3H, m), 1.06-1.00 (6H, m) ppm.
LRMS (APCI) : m/z [M+H]⁺ 659, [M+Na]⁺ 681.

### Example 25 : 2-(3-{(2R)-2-[(2R)-2-(6-Amino-pyridin-3-yl)-2-hydroxy-ethylamino]-propyl}-phenyl)-N-(4-benzylsulfamoyl-benzyl)-acetamide hydrochloride

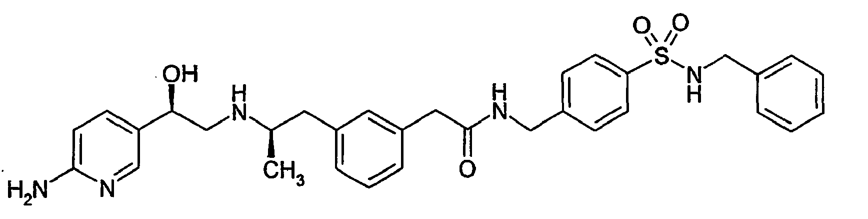

Prepared using the method for example 1 using *N*-(4-benzylsulfamoyl-benzyl)-2-[3-((2*R*)-2-{(2*R*)-2-[6-(2,5-dimethyl-pyrrol-1-yl)-pyridin-3-yl]-2-hydroxy-ethylamino}-propyl)-phenyl]-acetamide (preparation 25) to give the title compound as a colourless foam.
¹H NMR (400MHz, CD₃OD): δ = 8.02 (1H, d), 7.91 (1H, s), 7.72 (2H, d), 7.38-7.31 (3, m), 7.26-7.17 (8H, m), 7.06 (1H, d), 5.00-4.96 (1H. m), 4.43 (2H, s), 4.01 (2H, s), 3.59-3.49 (3H, m), 3.27-3.19 (3H, m), 2.81-2.74 (1H, m), 1.22 (3H, d) ppm.
LRMS (APCI) : m/z [M+H]⁺ 588, [M+Na]⁺ 610, [M-H]⁻ 586.

### Example 26 : 2-(3-{(2R)-2-[(2R)-2-(6-Amino-pyridin-3-yl)-2-hydroxy-ethylamino]-propyl}-phenyl)-N-(3-benzylsulfamoyl-benzyl)-acetamide hydrochloride

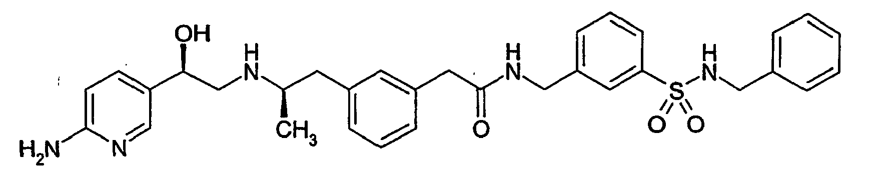

Prepared using the method for example 1 using *N*-(3-benzylsulfamoyl-benzyl)-2-[3-((2*R*)-2-{(2*R*)-2-[6-(2,5-dimethyl-pyrrol-1-yl)-pyridin-3-yl]-2-hydroxy-ethylamino}-propyl)-phenyl]-acetamide (preparation 26) to give the title compound as a colourless foam.
¹H NMR (400MHz, CD₃OD): δ = 8.02 (1H, d), 7.91 (1H, s), 7.71-7.68 (2H, m), 7.51-7.44 (2H, m), 7.32-7.14 (9H, m), 7.04 (1H, d), 4.99-4.96 (1H, m), 4.44 (2H, s), 3.98 (2H, s), 3.61-3.55 (3H, m), 3.25-3.13 (3H, m), 2.81-2.75 (1H, m), 1.24 (3H, d) ppm.
LRMS (APCI) : m/z [M+H]⁺ 588, [M+Na]⁺ 610, [M-H]⁻ 586.

### Example 27 : 4-{[2-(4-{2-[(2R)-2-(6-Amino-pyridin-3-yl)-2-hydroxy-ethylamino]-propyl}-phenyl)-acetylamino]-methyl}-N-(3,4-dimethoxybenzyl)-benzamide

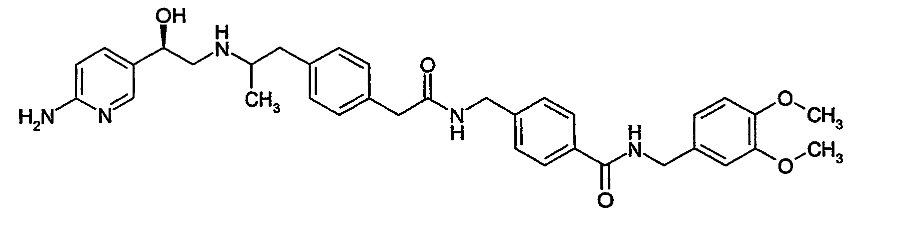

Prepared using the method for example 1 using *N-*(3,4-dimethoxy-benzyl)-4-({2-[4-(2-{(2*R*)-2-[6-(2,5-dimethyl-pyrrol-1-yl)-pyridin-3-yl]-2-hydroxy-ethylamino}-propyl)-phenyl]-acetylamino}-methyl)-benzamide (preparation 27) to give the title compound as a colourless foam.
¹H NMR (400MHz, CD₃OD): δ = 7.77 (3H, m), 7.40-6.89 (10H, m), 6.51 (1H, t), 4.55 (1H, m), 4.48 (2H, s), 4.41 (2H, s), 2.98 (3H, s), 2.52 (3H, s), 3.54 (2H, s), 2.96-2.61 (5H, m), 1.06 (3H, d).
LRMS (APCI) : m/z [M+H]⁺ 612.

### Example 28: 4-{[2-(4-{2-[(2R)-2-(6-Amino-pyridin-3-yl)-2-hydroxy-ethylamino]-propyl}-phenyl)-acetylamino]-methyl}-N-(2-ethoxy-benzyl)-benzamide

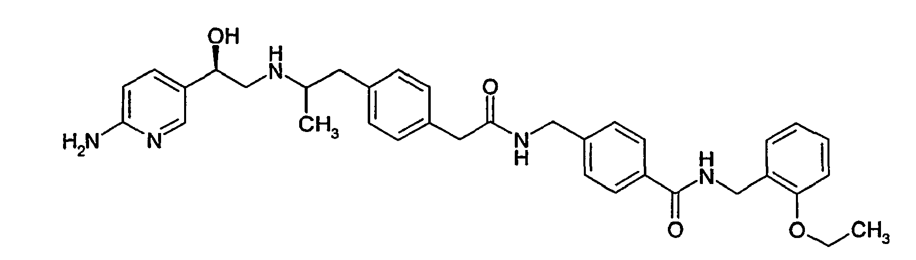

Prepared using the method for example 1 using 4-({2-[4-(2-{(2*R*)-2-[6-(2,5-dimethyl-pyrrol-1-yl)-pyridin-3-yl]-2-hydroxy-ethylamino}-propyl)-phenyl]-acetylamino}-methyl)-*N*-(2-ethoxy-benzyl)-benzamide (preparation 28) to give the title compound as a colourless foam.
¹H NMR (400MHz, CD₃OD): δ = 7.77 (3H, m), 7.40-6.87 (11H, m), 6.51 (1H, t), 4.56 (2H, s), 4.54 (1H, m), 4.41 (2H, s), 4.08 (2H, q), 3.54 (2H, m), 2.92-2.61 (5H, m), 1.40 (3H, t), 1.06 (3H, m) ppm.
LRMS (APCl) : m/z [M+H]⁺ 596.

### Example 29 : 4-{[2-(4-{2-[(2R)-2-(6-Amino-pyridin-3-yl)-2-hydroxy-ethylamino]-propyl}-phenyl)-acetylamino]-methyl}-N-[(1R)-1-(4-methoxyphenyl)-ethyl]-benzamide

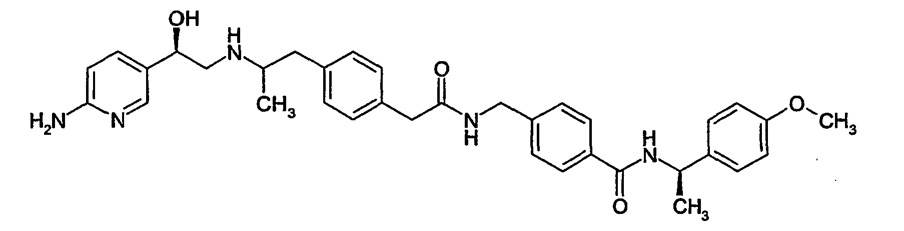

Prepared using the method for example 1 using 4-({2-[4-(2-{(2*R*)-2-[6-(2,5-dimethyl-pyrrol-1-yl)-pyridin-3-yl]-2-hydroxy-ethylamino}-propyl)-phenyl]-acetylamino}-methyl)-*N*-[(1*R*)-1-(4-methoxy-phenyl)-ethyl]-benzamide (preparation 29) to give the title compound as a colourless foam.
¹H NMR (400MHz, CD₃OD): δ = 7.79 (1H, m), 7.74 (2H, d), 7.40-7.09 (9H, m), 6.88 (2H, d), 6.51 (1H, t), 5.19 (1H, m), 4.54 (1H, m), 4.40 (2H, s), 3.76 (3H, s), 3.54 (2H, s), 2.93-2.62 (5H, m), 1.52 (3H, d), 1.06 (3H, m) ppm.
LRMS (APCl): m/z [M+H]⁺ 596.

### Example 30 : 4-{[2-(4-{2-[(2R)-2-(6-Amino-pyridin-3-yl)-2-hydroxy-ethylamino]-propyl}-phenyl)-acetylamino]-methyl)-N-((1R)-1-benzyl-2-hydroxy-ethyl)-benzamide

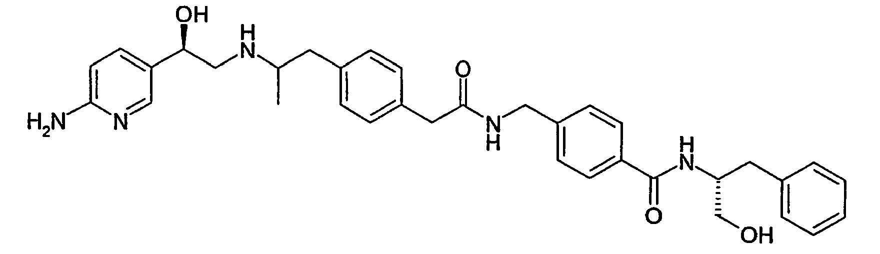

Prepared using the method for example 1 using 4-({2-[4-(2-{(2*R*)-2-[6-(2,5-dimethyl-pyrrol-1-yl)-pyridin-3-yl]-2-hydroxy-ethylamino}-propyl)-phenyl]-acetylamino}-methyl)-*N*-((1*R*)-1-benzyl-2-hydroxy-ethyl)-benzamide (preparation 30) to give the title compound as a colourless foam.
¹H NMR (400MHz, CD₃OD): δ = 7.78 (1H, d), 7.65 (2H, d), 7.38 (1H, t), 7.27-7.07 (11H, m), 6.51 (1H, t), 4.53 (1H, m), 4.39 (2H, s), 4.33 (1H, m), 3.63 (2H, d), 3.54 (2H, s), 3.04-2.60 (7H, m), 1.06 (3H, m) ppm.
LRMS (APCI) : m/z [M+H]⁺ 596.

### Example 31 : 4-{[2-(4-{2-[(2R)-2-(6 Amino-pyridin-3-yl)-2-hydroxy-ethylamino]-propyl}-phenyl)-acetylamino]-methyl}-N-((1R, 2S)-2-hydroxy-1-methyl-2-phenyl-ethyl)-benzamide

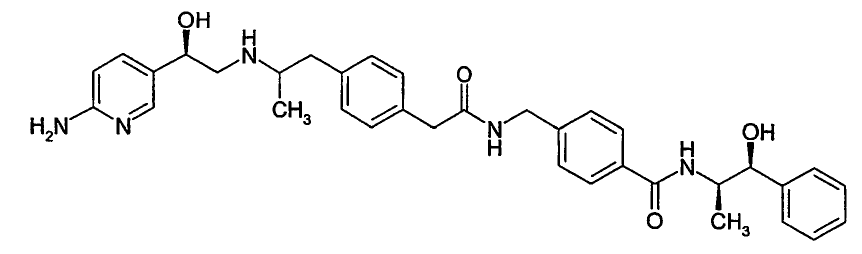

Prepared using the method for example 1 using 4-({2-[4-(2-{(2*R*)-2-[6-(2,5-dimethyl-pyrrol-1-yl)-pyridin-3-yl]-2-hydroxy-ethylamino}-propyl)-phenyl]-acetylamino}-methyl)-*N*-((1*R*,2*S*)-2-hydroxy-1-methyl-2-phenyl-ethyl)-benzamide (preparation 31) to give the title compound as a colourless foam.
¹H NMR (400MHz, CD₃OD): δ = 7.78 (1H, d), 7.63 (2H, d), 7.42-7.07 (12H, m), 6.51 (1H, t), 4.54 (1H, m), 4.39 (2H, s), 4.32 (1H, m), 3.54 (2H, s), 2.95-2.55 (6H, m), 1.18 (3H, d), 1.06 (3H, m) ppm.
LRMS (APCI) : m/z [M+H]⁺ 596.

### Example 32 : 4-{[2-(4-{2-[(2R)-2-(6-Amino-pyridin-3-yl)-2-hydroxy-ethylamino]-propyl}-phenyl)-acetylamino]-methyl}-N-((1S, 2R)-2-hydroxy-1-methyl-2-phenyl-ethyl)-benzamide

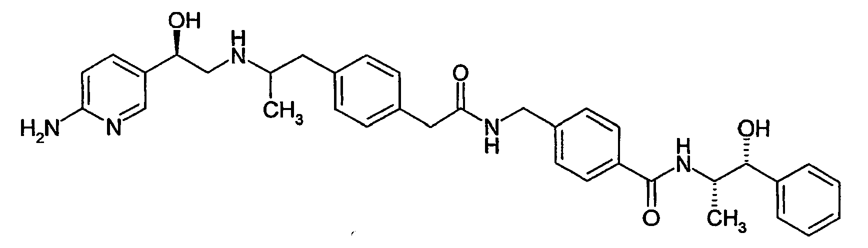

Prepared using the method for example 1 using 4-({2-[4-(2-{(2*R*)-2-[6-(2,5-dimethyl-pyrrol-1-yl)-pyridin-3-yl]-2-hydroxy-ethylamino}-propyl)-phenyl]-acetylamino}-methyl)-*N*-((1*S*,2*R*)-2-hydroxy-1-methyl-2-phenyl-ethyl)-benzamide (preparation 32) to give the title compound as a colourless foam.
¹H NMR (400MHz, CD₃OD): δ = 7.79 (1H, d), 7.63 (2H, d), 7.42-7.09 (12H, m), 6.51 (1H, t), 4.54 (1H, m), 4.39 (2H, s), 4.33 (1H, m), 3.54 (2H, s), 2.96-2.55 (6H, m), 1.19 (3H, d), 1.07 (3H, m) ppm.
LRMS (APCI) : m/z [M+H]⁺ 596.

### Example 33 : 4-{[2-(4-{2-[(2R)-2-(6-Amino-pyridin-3-yl)-2-hydroxy-ethylamino]-propyl}-phenyl)-acetylamino]-methyl}-N-[2-(3-methoxyphenyl)-ethyl]-benzamide

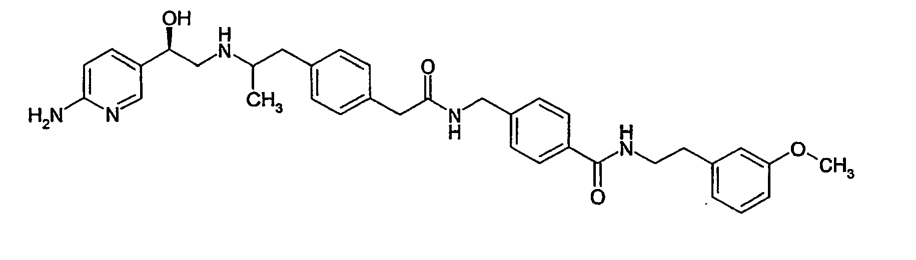

Prepared using the method for example 1 using 4-({2-[4-(2-{(2R)-2-[6-(2,5-dimethyl-pyrrol-1-yl)-pyridin-3-yl]-2-hydroxy-ethylamino}-propyl)-phenyl]-acetylamino}-methyl)-*N-*[2-(3-methoxy-phenyl)-ethyl]-benzamide (preparation 33) to give the title compound as a colourless foam.
¹H NMR (400MHz, CD₃OD): δ = 7.80 (1H, d), 7.69 (2H, d), 7.41-7.07 (8H, m), 6.81 (2H, d), 6.76 (1H, d), 6.51 (1H, t), 4.51 (1H, m), 4.40 (2H, s), 3.74 (3H, s), 3.62-3.52 (4H, m), 2.90-2.65 (7H, m), 1.07 (3H, m) ppm.
LRMS (APCI): m/z [M+H]⁺ 596.

### Example 34 : 2-(3-{(2R)-2-[(2R)-2-(6-Amino-pyridin-3-yl)-2-hydroxy-ethylamino]-propyl}-phenyl)-N-{4-[3-(3-phenoxy-benzyl)-ureidomethyl]-benzyl}-acetamide

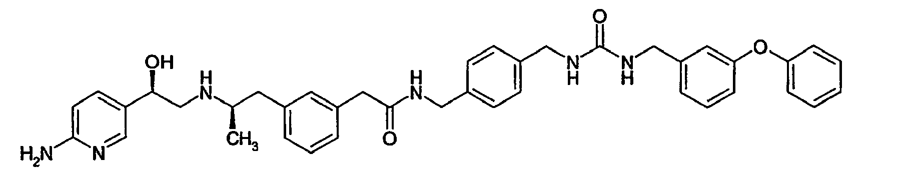

A solution of 2-[3-((2*R*)-2-{(2*R*)-2-[6-(2,5-Dimethyl-pyrrol-1-yl)-pyridin-3-yl]-2-hydroxy-ethylamino}-propyl)-phenyl]-*N*-{4-[3-(3-phenoxy-benzyl)-ureidomethyl]-benzyl}-acetamide (184mg, 0.24mmol) in ethanol (5ml) was treated with hydroxylamine hydrochloride (85mg, 1.22mmol) and the resulting solution heated to 80°C for 24 hours. The reaction mixture was cooled to room temperature and loaded onto a strong cation exchange column eluting products with methanol and then 1M ammonia in methanol. The basic fractions were combined and the solvent removed *in vacuo.* Purification by flash column chromatography eluting with dichloromethane:methanol: 880 ammonia (100:0:0 changing to 92:8:1 by volume) gave the title compound as a colourless foam (47mg, 29%).
¹H NMR (400MHz, CD₃OD): δ = 7.77 (1H, s), 7.36-7.25 (3H, m), 7.20-6.93 (14H, m), 6.84 (1H, d), 6.48 (1H, d), 4.52 (1H, m), 4.32 (2H, s), 4.30 (2H, s), 4.28 (2H, s), 3.50 (2H, s), 2.91-2.81 (2H, m), 2.69-2.55 (3H, m), 1.05 (3H, d) ppm.
LRMS (APCI) : m/z [M+H]⁺ 673.

### Example 35 : 2-(3-{(2R)-2-[(2R)-2-(6-Amino-pyridin-3-yl)-2-hydroxy-ethylamino]-propyl}-phenyl)-N-{4-[3-(3,4-dimethoxy-benzyl)-ureidomethyl]-benzyl}-acetamide

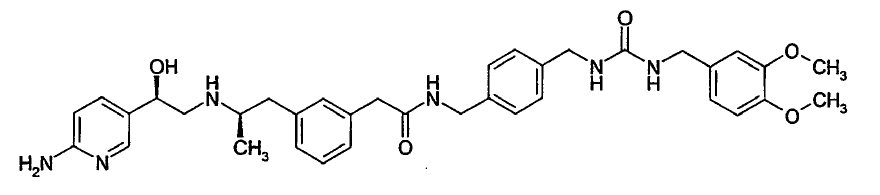

Prepared from *N*-{4-[3-(3,4-Dimethoxy-benzyl)-ureidomethyl]-benzyl}-2-[3-((2*R*)-2-{(2*R*)-2-[6-(2,5-dimethyl-pyrrol-1-yl)-pyridin-3-yl]-2-hydroxy-ethylamino}-propyl)-phenyl]-acetamide using the method described for example 34 to give the title compound as a pale yellow foam.
¹H NMR (400MHz, CD₃OD): δ = 7.78 (1H, s), 7.35 (1H, d), 7.21-7.12 (6H, m), 7.08 (1H, s), 7.02 (1H, d0, 6.88 (2H, m), 6.82 (1H, m), 6.50 (1H, d), 4.52 (1H, m), 4.33 (2H, s), 4.30 (2H, s), 4.25 (2H, s), 3.79 (3H, s), 3.77 (3H, s), 3.51 (2H, s), 2.91-2.82 (2H, m), 2.70-2.55 (3H, m), 1.05 (3H, d) ppm.
LRMS (APCl) : m/z [M+H]⁺ 641.

### Example 36 : 2-(3-{(2R)-2-[(2R)-2-(6-Amino-pyridin-3-yl)-2-hydroxy-ethylamino]-propyl}-phenyl)-N-{4-[3-(4-phenoxy-benzyl)-ureidomethyl]-benzyl}-acetamide

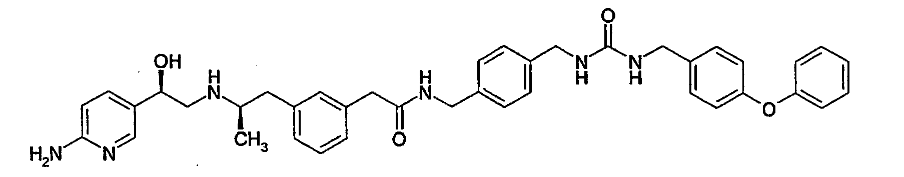

Prepared from 2-[3-(2-{2-[6-(2,5-Dimethyl-pyrrol-1-yl)-pyridin-3-yl]-2-hydroxy-ethylamino}-propyl)-phenyl]-N-{4-[3-(4-phenoxy-benzyl)-ureidomethyl]-benzyl}-acetamide using the method described for example 34 to give the title compounds as a pale yellow foam.
¹H NMR (400MHz, CD₃OD): δ = 8.50 (1H, d), 7.77 (1H, s), 7.34-6.91 (18H, m), 4.52 (1H, m), 4.33 (2H, s), 4.30 (4H, s), 3.50 (2H, s), 2.92-2.81 (2H, m), 2.69-2.55 (3H, m), 1.06 (3H, d) ppm.

The following Preparations describe the preparation of certain intermediates used in the preceding Examples.

### Preparation 1 : N-(3,4-Dimethoxy-benzyl)-4-({2-[3-(2-{(2R)-2-[6-(2,5-dimethyl-pyrrol-1-yl)-pyridin-3-yl]-2-hydroxy-ethylamino}-propyl)-phenyl]-acetylamino}-methyl)-benzamide

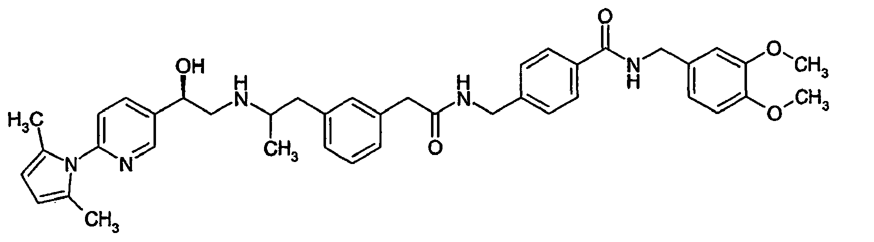

A solution of the acid from preparation 34 (105 mg, 0.19 mmol), 1-hydroxybenzotriazole hydrate (41 mg, 0.3 mmol), 1-(3-dimethylaminopropyl)-3-ethylcarbodiimide hydrochloride (50 mg, 0.26 mmol), triethylamine (80 µl, 0.58 mmol) and 3,4-dimethoxybenzylamine (37 µl, 0.24 mmol) in *N,N-*dimethylformamide (1 ml) was left to stir at room temperature for 40 hours. The solvent was removed *in vacuo* and the residue partitioned between water (2 ml) and dichloromethane (2 ml). The organic phase was separated and reduced *in vacuo.* Purification by flash column chromatography eluting with dichloromethane: methanol: 880 ammonia (98:2 changing to 95:5:0.5 by volume) gave 48 mg of *N-*(3,4-dimethoxybenzyl)-4-{[({3-[2-({(2*R*)-2-[6-(2,5-dimethyl-1*H-*pyrrol-1-yl)-3-pyridinyl]-2-hydroxyethyl}-amino)-propyl]-phenyl}-acetyl)-amino]-methyl}-benzamide (1:1 mixture of diastereoisomers) as a pale yellow foam.
¹H NMR (400MHz, CD₃OD): δ = 8.58-8.45 (1H, m), 8.01-7.90 (1H, m), 7.83-7.72 (2H, m), 7.42-7.06 (7H, m), 6.96 (1H, s), 6.89 (2H, s), 5.81 (2H, s), 4.79-4.70 (1H, m, partially obscured by solvent), 4.48 (2H, s), 4.40 (2H, s), 3.79 (6H, s), 3.54 (2H, s), 3.07-2.51 (5H, m), 2.02 (6H, s), 1.09-0.95 (3H, m) ppm.
LRMS (electrospray) : m/z [M-H]⁻ 688.

### Preparation 2 : 4-({2-[3-(2-{(2R)-2-[6-(2,5-Dimethyl-pyrrol-1-yl)-pyridin-3-yl]-2-hydroxy-ethylamino}-propyl)-phenyl]-acetylamino}-methyl)-N-(4-sulfamoyl-benzyl)-benzamide

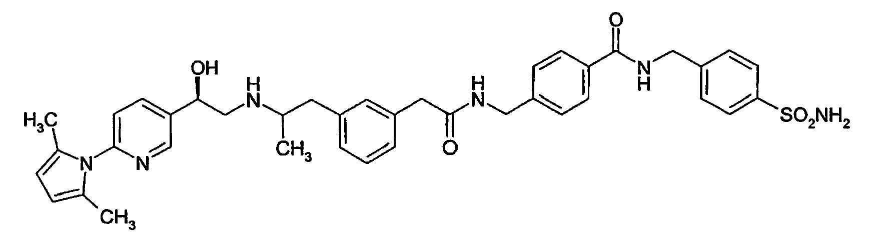

Prepared using the method for 1 using the acid from preparation 34 and the appropriate amine to give the title compound (1:1 mixture of diastereoisomers) as a pale yellow foam.
¹H NMR (400MHz, CD₃OD): δ = 8.56-8.45 (1H, m), 7.98-7.74 (5H, m), 7.56-7.46 (2H, m), 7.36-7.05 (7H, m), 5.81 (2H, s), 4.87-4.77 (1H, m, partially obscured by solvent), 4.61 (2H, s), 4.42 (2H, s), 3.56 (2H, s), 3.05-2.55 (5H, m), 2.02 (6H, s), 1.12-1.01 (3H, m) ppm.
LRMS (electrospray) : m/z [M-H]⁻ 707.

### Preparation 3 : 4-({2-[3-(2-{(2R)-2-[6-(2,5-Dimethyl-pyrrol-1-yl)-pyridin-3-yl]-2-hydroxy-ethylamino}-propyl)-phenyl]-acetylamino}-methyl)-N-(2-ethoxy-benzyl)-benzamide

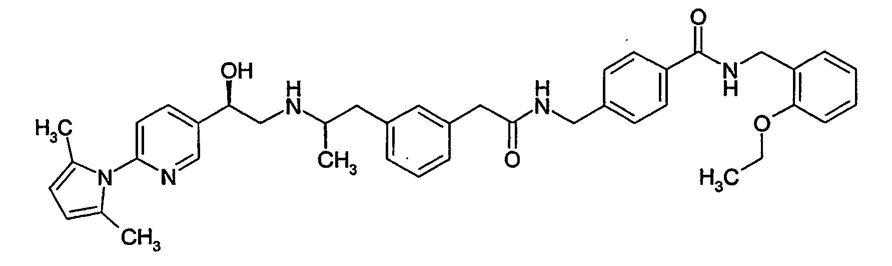

Prepared using the method for preparation 1 using the acid from preparation 34 and the appropriate amine to give the title compound (1:1 mixture of diastereoisomers) as a pale yellow foam.
¹H NMR (400MHz, CD₃OD): δ = 8.57-8.46 (1H, m), 7.99-7.90 (1H, m), 7.84-7.73 (2H, m), 7.44-7.06 (9H, m), 6.97-6.84 (2H, m), 5.81 (2H, s), 4.79-4.72 (1H, m, partially obscured by solvent), 4.56 (2H, s), 4.41 (2H, s), 4.08 (2H, q), 3.55 (2H, s), 3.07-2.54 (5H, m), 2.02 (6H, s), 1.40 (3H, t), 1.09-0.91 (3H, m) ppm.
LRMS (electrospray) : m/z [M+H]⁺ 674, [M+Na]⁺ 696.

### Preparation 4 : 4-({2-[3-(2-{(2R)-2-[6-(2,5-Dimethyl-pyrrol-1-yl)-pyridin-3-yl]-2-hydroxy-ethylamino}-propyl)-phenyl]-acetylamino}-methyl)-N-[(1R)-1-(4-methoxy-phenyl)-ethyl]-benzamide

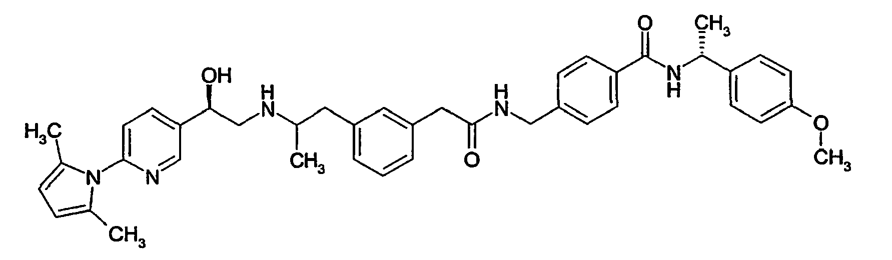

Prepared using the method for preparation 1 using the acid from preparation 34 and the appropriate amine to give the title compound (1:1 mixture of diastereoisomers) as a pale yellow foam.
¹H NMR (400MHz, CD₃OD): δ = 8.52-8.45 (1H, m), 7.97-7.89 (1H, m), 7.80-7.70 (2H, m), 7.41-7.07 (9H, m), 6.91-6.82 (2H, m), 5.81 (2H, s), 5.23-5.13 (1H, m), 4.81-4.74 (1H, m, partially obscured by solvent), 4.40 (2H, s), 3.76 (3H, s), 3.54 (2H, s), 3.07-2.54 (5H, m), 2.02 (6H, s), 1.56-1.46 (3H, m), 1.10-0.97 (3H, m) ppm.
LRMS (electrospray) : m/z [M+H]⁺ 674, [M+Na]⁺ 696.

### Preparation 5 : 4-({2-[3-(2-{(2R)-2-[6-(2,5-Dimethyl-pyrrol-1-yl)-pyridin-3-yl]-2-hydroxy-ethylamino}-propyl)-phenyl]-acetylamino}-methyl)-N-((1R)-1-hydroxymethyl-2-phenyl-ethyl)-benzamide

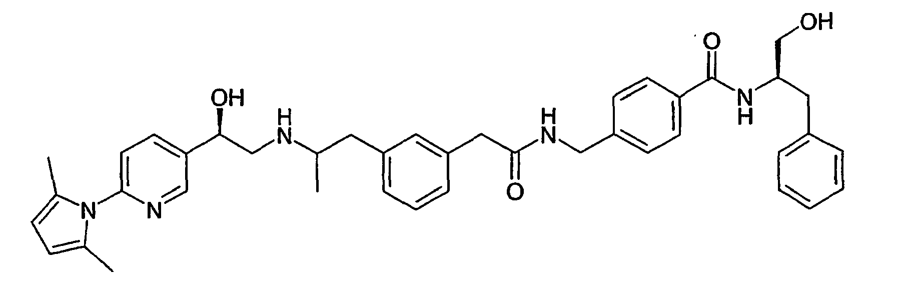

Prepared using the method for preparation 1 using the acid from preparation 34 and the appropriate amine to give the title compound (1:1 mixture of diastereoisomers) as a pale yellow foam.
¹H NMR (400MHz, CD₃OD): δ = 8.57-8.44 (1H, m), 7.98-7.88 (1H, m), 7.70-7.61 (2H, m), 7.40-7.06 (12H, m), 5.81 (2H, s), 4.90-4.74 (1H, m, partially obscured by solvent), 4.39 (2H, s), 4.36-4.26 (1H, m), 3.62 (2H, d), 3.54 (2H, s), 3.30 (2H, m, obscured by solvent) 3.07-2.55 (5H, m), 2.02 (6H, s), 1.11-0.99 (3H, m) ppm.
LRMS (electrospray) : m/z [M+H]⁺ 674, [M+Na]⁺ 696.

### Preparation 6 : 4-({2-[3-(2-{(2R)-2-[6-(2,5-Dimethyl-pyrrol-1-yl)-pyridin-3-yl]-2-hydroxy-ethylamino}-propyl)-phenyl]-acetylamino}-methyl)-N-((1R, 2S)-2-hydroxy-1-methyl-2-phenyl-ethyl)-benzamide

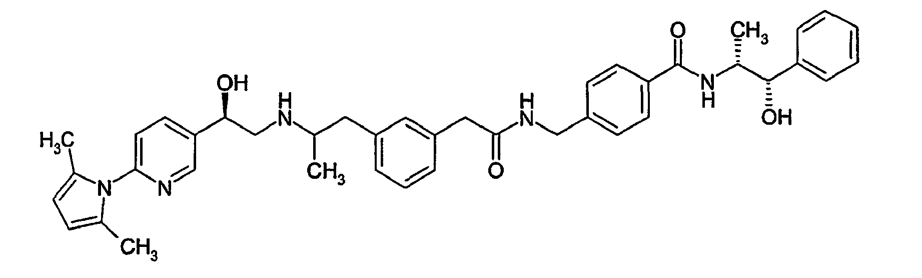

Prepared using the method for preparation 1 using the acid from preparation 34 and the appropriate amine to give the title compound (1:1 mixture of diastereoisomers) as a pale yellow foam.
¹H NMR (400MHz, CD₃OD): δ = 8.55-8.46 (1H, m), 7.98-7.87 (1H, m), 7.68-7.58 (2H, m), 7.46-7.37 (2H, m), 7.37-7.07 (10H, m), 5.81 (2H, s), 4.85-4.70 (1H, m, obscured by solvent), 4.39 (2H, s), 4.36-4.27 (1H, m), 3.55 (2H, s), 3.35-3.30 (1H, m, obscured by solvent), 3.07-2.54 (5H, m), 2.02 (6H, s), 1.21-1.10 (3H, m), 1.10-0.90 (3H, m) ppm.
LRMS (electrospray) : m/z [M+H]⁺ 674, [M+Na]⁺ 696.

### Preparation 7 : 4-({2-[3-(2-{(2R)-2-[6-(2,5-Dimethyl-pyrrol-1-yl)-pyridin-3-yl]-2-hydroxy-ethylamino}-propyl)-phenyl]-acetylamino}-methyl)-N-((1S, 2R)-2-hydroxy-1-methyl-2-phenyl-ethyl)-benzamide

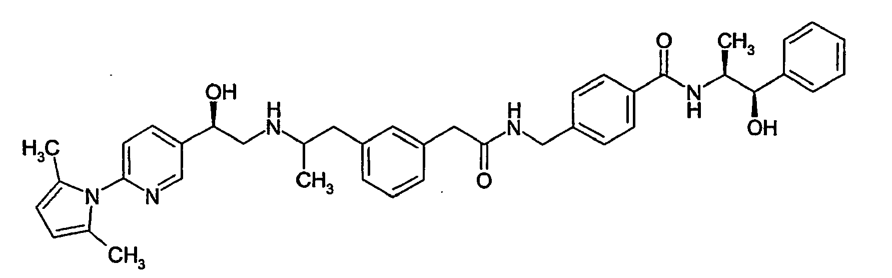

Prepared using the method for preparation 1 using the acid from preparation 34 and the appropriate amine to give the title compound (1:1 mixture of diastereoisomers) as a pale yellow foam.
¹H NMR (400MHz, CD₃OD): δ = 8.55-8.46 (1H, m), 7.98-7.87 (1H, m), 7.68-7.58 (2H, m), 7.46-7.37 (2H, m), 7.37-7.07 (10H, m), 5.81 (2H, s), 4.85-4.70 (1H, m, obscured by solvent), 4.39 (2H, s), 4.36-4.27 (1H, m), 3.55 (2H, s), 3.35-3.30 (1H, m, obscured by solvent), 3.07-2.54 (5H, m), 2.02 (6H, s), 1.21-1.10 (3H, m), 1.10-0.90 (3H, m) ppm.
LRMS (electrospray) : m/z [M+H]⁺ 674, [M+Na]⁺ 696.

### Preparation 8 : 4-({2-[3-(2-{(2R)-2-[6-(2,5-Dimethyl-pyrrol-1-yl)-pyridin-3-yl]-2-hydroxy-ethylamino}-propyl)-phenyl]-acetylamino}-methyl)-N-[2-(3-methoxy-phenyl)-ethyl]-benzamide

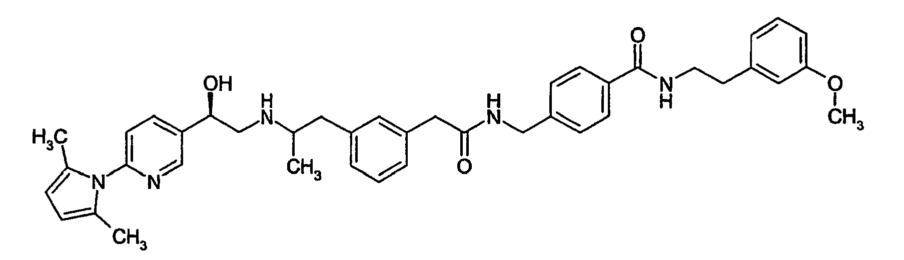

Prepared using the method for preparation 1 using the acid from preparation 34 and the appropriate amine to give the title compound (1:1 mixture of diastereoisomers) as a pale yellow foam.
¹H NMR (400MHz, CD₃OD) δ = 8.57-8.46 (1H, m), 7.98-7.89 (1H, m), 7.76-7.64 (2H, m), 7.41-7.08 (8H, m), 6.86-6.76 (2H, m), 6.76-6.70 (1H, m), 5.81 (2H, s), 4.88-4.79 (1H, m, obscured by solvent), 4.40 (2H, s), 3.74 (3H, s), 3.60-3.49 (4H, m), 3.34-3.22 (2H, m, obscured by solvent), 3.07-2.55 (5H, m), 2.02 (6H, s), 1.10-0.98 (3H, m) ppm.
LRMS (electrospray) : m/z [M+H]⁺ 674, [M+Na]⁺ 696.

### Preparation 9 : N-(3,4-Dimethoxy-benzyl)-4-({2-[3-((2R)-2-{(2R)-2-[6-(2,5-dimethyl-pyrrol-1-yl)-pyridin-3-yl]-2-hydroxy-ethylamino}-propyl)-phenyl]-acetylamino}-methyl)-benzamide

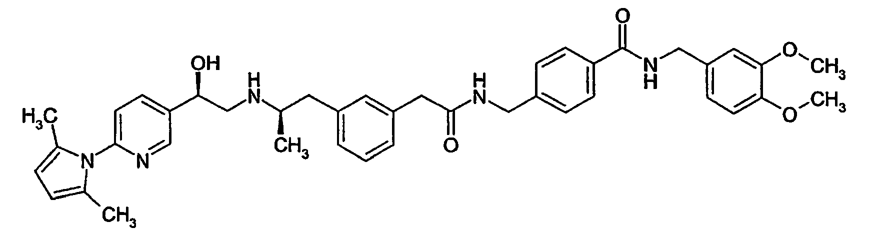

Prepared using the method for preparation 1 using the acid from preparation 38 and the appropriate amine to give the title compound as a colourless foam.
¹H NMR (400MHz, CD₃OD): δ = 8.50 (1H, s), 7.94 (1H, d), 7.76 (2H, d), 7.31-7.09 (7H, m), 6.97 (1H, s), 6.89 (2H, s), 5.81 (2H, s), 4.81 (1H, m, partially obscured by solvent), 4.48 (2H, s), 4.40 (2H, s), 3.79 (6H, s), 3.54 (2H, s), 2.99 (1H, m), 2.86 (2H, m), 2.75 (1H, m), 2.62 (1H, m), 2.02 (6H, s), 1.06 (3H, d) ppm.
LRMS (APCI) : m/z [M+H]⁺ 690.
Optical Rotation [α]^{D}₂₅ = -24.80° c=1, methanol

### Preparation 10 : 4-({2-[3-((2R)-2-{(2R)-[6-(2,5-Dimethyl-pyrrol-1-yl)-pyridin-3-yl]-2-hydroxy-ethylamino}-propyl)-phenyl]-acetylamino}-methyl)-N-(2-ethoxy-benzyl)-benzamide

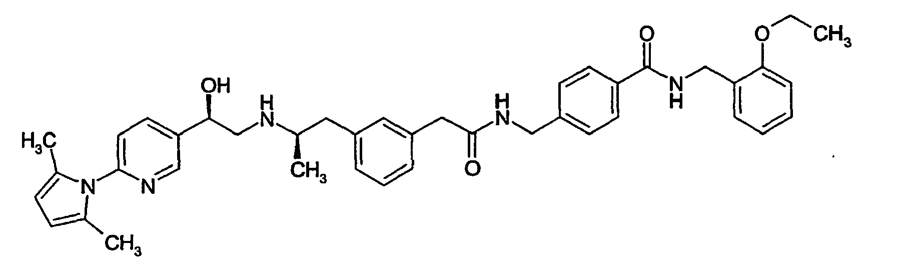

Prepared using the method for preparation 1 using the acid from preparation 38 and the appropriate amine to give the title compound as a colourless foam.
¹H NMR (400MHz, CD₃OD): δ = 8.50 (1H, s), 7.92 (1H, d), 7.76 (2H, d), 7.32-7.09 (9H, m), 6.94 (1H, d), 6.87 (1H, t), 5.81 (2H, s), 4.81 (1H, m, partially obscured by solvent), 4.57 (2H, s), 4.41 (2H, s), 4.08 (2H, q), 3.55 (2H, s), 2.99 (1H, m), 2.85 (2H, m), 2.75 (1H, m), 2.62 (1H, m), 2.02 (6H, s), 1.40 (3H, t), 1.06 (3H, d) ppm.
LRMS (APCI) : m/z [M+H]⁺ 674.
Optical Rotation [α]^{D}₂₅ = -28.01° c=1, MeOH

### Preparation 11 : 4-({2-[3-((2R)-2-{(2R)-2-[6-(2,5-Dimethyl-pyrrol-1-yl)-pyridin-3-yl]-2-hydroxy-ethylamino}-propyl)-phenyl]-acetylamino}-methyl)-N-(4-hydroxy-3-methoxy-benzyl)-benzamide

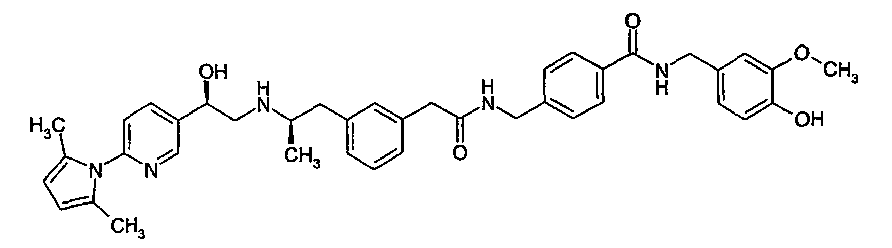

Prepared using the method for preparation 1 using the acid from preparation 38 and the appropriate amine to give the title compound as a colourless foam.
¹H NMR (400MHz, CD₃OD): δ = 8.51 (1H, s), 7.92 (1H, d), 7.76 (2H, d), 7.31-7.11 (7H, m), 7.17 (1H, s), 6.75 (2H, m), 5.82 (2H, s), 4.82 (1H, m, partially obscured by solvent), 4.45 (2H, s), 4.40 (2H, s), 3.82 (3H, s), 3.55 (2H, s), 2.99 (1H, m), 2.86 (2H, m), 2.79 (1H, m), 2.62 (1H, m), 2.01 (6H, s), 1.06 (3H, d) ppm.
LRMS (APCI) : m/z [M+H]⁺ 676.
Optical Rotation [α]^{D}₂₅ = -23.60° c=1, methanol

### Preparation 12 : 4-({2-[3-((2R)-2-{(2R)-2-[6-(2,5-Dimethyl-pyrrol-1-yl)-pyridin-3-yl]-2-hydroxy-ethylamino}-propyl)-phenyl]-acetylamino}-methyl)-N-(4-sulfamoyl-benzyl)-benzamide

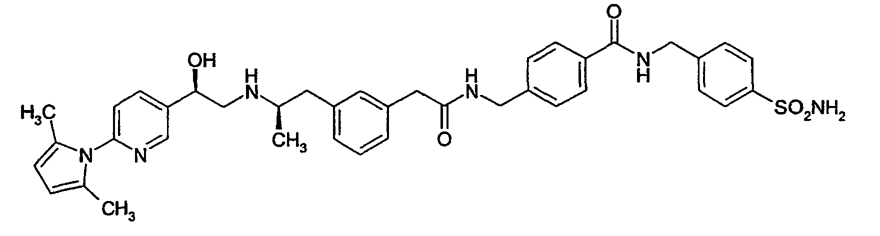

Prepared using the method for preparation 1 using the acid from preparation 38 and the appropriate amine to give the title compound as a colourless foam.
¹H NMR (400MHz, CD₃OD): δ = 8.50 (1H, s), 7.93 (1H, d), 7.85 (2H, d), 7.78 (2H, d), 7.49 (2H, d), 7.33-7.11 (7H, m), 5.81 (2H, s), 4.80 (1H, bs, partially obscured by solvent), 4.61 (2H, s), 4.41 (2H, s), 3.55 (2H, s), 2.98 (1H, m), 2.87 (2H, m), 2.76 (1H, m), 2.60 (1H, m), 2.02 (6H, s), 1.07 (3H, d) ppm.
LRMS (APCI) : m/z [M+H]⁺ 709.
Optical Rotation [α]^{D}₂₅ = -20.4° c=1, methanol

### Preparation 13 : 4-({2-[3-((2R)-2-((2R)-2-[6-(2,5-Dimethyl-pyrrol-1-yl)-pyridin-3-yl]-2-hydroxy-ethylamino}-propyl)-phenyl]-acetylamino}-methyl)-N-((1R,2S)-2-hydroxy-1-methyl-2-phenyl-ethyl)-benzamide

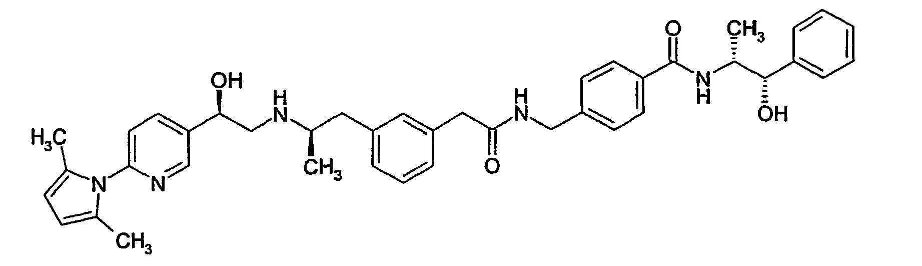

Prepared using the method for preparation 1 using the acid from preparation 38 and the appropriate amine to give the title compound as a colourless foam.
¹H NMR (400MHz, CD₃OD): δ = 8.50 (1H, s), 7.93 (1H, d), 7.62 (2H, d), 7.41 (2H, d), 7.31-7.09 (10H, m), 5.81 (2H, s), 4.53 (1H, bs), 4.39 (2H, s), 3.55 (2H, s), 3.35 (2H, m, partially obscured by solvent), 3.00 (1H, m), 2.88 (2H, m), 2.77 (1H, m), 2.62 (1H, m), 2.03 (6H, s), 1.21 (3H, d), 1.08 (3H, d) ppm.
LRMS (APCI) : m/z [M+H]⁺ 674.
Optical Rotation [α]^{D}₂₅ = -49.81° c=1, methanol

### Preparation 14: N-[4-({2-[3-((2R)-2-((2R)-2-[6-(2,5-Dimethyl-pyrrol-1-yl)-pyridin-3-yl]-2-hydroxy-ethylamino}-propyl)-phenyl]-acetylamino}-methyl)-benzyl]-2,2-diphenyl-acetamide

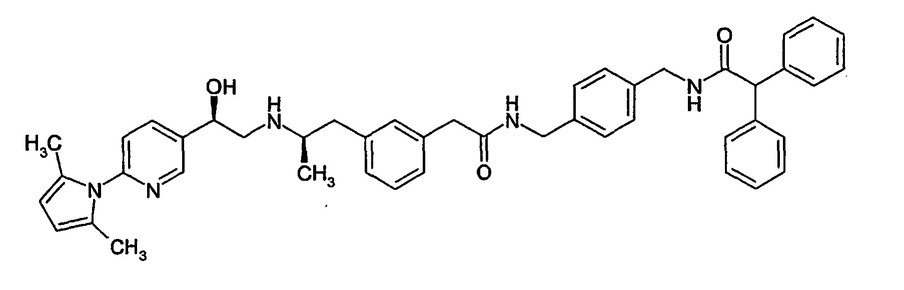

Prepared using the method for preparation 1 using the acid from preparation 38 and the amine from preparation 57 to give the title compound as a colourless foam.
¹H NMR (400MHz, CDCl₃): δ = 8.50 (1H, m), 7.94-7.92 (1H, m), 7.30-7.08 (21 H, m), 5.81 (2H, s), 4.97 (1H, s), 4.83-4.79 (1H, m), 4.35 (2H, s), 4.31 (2H, s), 3.51 (2H, s), 3.02-2.94 (1H, m), 2.89-2.84 (2H, m), 2.79-2.74 (1H, dd), 2.61-2.56 (1H, dd), 2.02 (6H, s), 1.06 (3H, d) ppm.
LRMS (APCI) : m/z [M+H]⁺ 720, [M+Na]⁺ 742.

### Preparation 15 : N-{4-[(Benzhydryl-amino)-methyl]-benzyl}-2-[3-((2R)-2-{(2R)-2-[6-(2,5-dimethyl-pyrrol-1-yl)-pyridin-3-yl]-2-hydroxy-ethylamino}-propyl)-phenyl]-acetamide

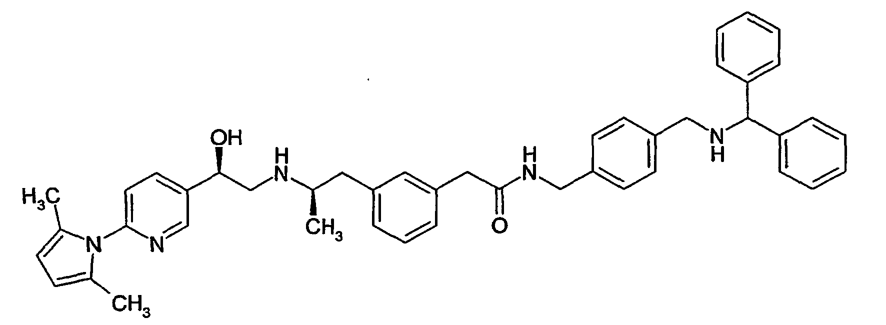

Prepared using the method for preparation 1 using the acid from preparation 38 and the amine from preparation 60 to give the title compound as a colourless foam.
¹H NMR (400MHz, CD₃OD): δ = 8.49 (1H, bs), 7.91 (1H, bd), 7.36-7.08 (19H, m), 5.81 (2H, s), 4.82-4.80 (1H, m), 4.75 (1H, s), 4.34 (2H, s), 3.84 (2H, s), 3.53 (2H, s), 2.85-2.63 (5H, m), 2.02 (6H, s), 1.06 (3H, d) ppm.
LRMS (APCI) : m/z [M+H]⁺ 692, [M+Na]⁺ 714.

### Preparation 16 : N-(2-Benzyloxy-ethyl)-4-({2-[3-((2R)-2-{(2R)-2-[6-(2,5-dimethyl-pyrrol-1-yl)-pyridin-3-yl]-2-hydroxy-ethylamino}-propyl)-phenyl]-acetylamino}-methyl)-benzamide

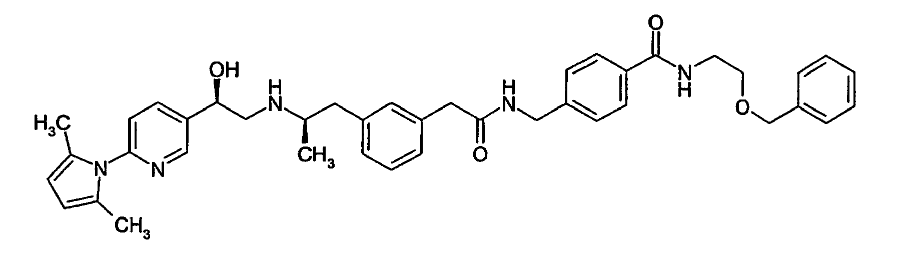

Prepared using the method for preparation 1 using the acid from preparation 38 and the amine from preparation 62 to give the title compound as a colourless foam.
¹H NMR (400MHz, CDCl₃): δ = 8.46 (1H, s), 7.78-7.65 (1H, m), 7.56 (2H, d), 7.32-7.05 (10H, m), 6.97 (1H, d), 6.87 (1H, m), 5.86 (2H, s), 5.16-5.13 (1H, m), 4.50 (2H, s), 4.32 (2H, d), 3.64-3.57 (4H, m), 3.50 (2H, s), 3.37-3.32 (1H, m), 3.14-3.01 (3H, m), 2.78-2.73 (1H, m), 2.04 (6H, s) 1.19 (3H, d) ppm.
LRMS (electrospray) : m/z [M+H]⁺ 674, [M+Na]⁺ 696.

### Preparation 17 : 4-({2-[3-((2R)-2-{(2R)-2-[6-(2,5-Dimethyl-pyrrol-1-yl)-pyridin-3-yl]-2-hydroxy-ethylamino}-propyl)-phenyl]-acetylamino}-methyl)-N-[2-(3-phenyl-propoxy)-ethyl]-benzamide

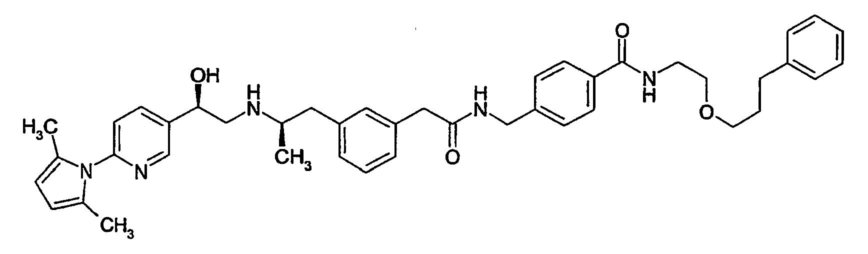

Prepared using the method for preparation 1 using the acid from preparation 38 and the amine from preparation 63 to give the title compound as a colourless foam.
¹H NMR (400MHz, CDCl₃): δ = 8.51 (1H, s), 7.82-7.80 (1H, m), 7.64-7.53 (3H, m), 7.33 (1H, m), 7.26-7.07 (9H, m), 6.95 (1H, d), 6.85 (1H, m), 5.86 (2H, s), 5.36-5.32 (1H, m), 4.37-4.28 (2H, m), 3.59-3.41 (9H, m), 3.26-3.14 (3H, m), 2.81-2.76 (1H, m), 2.64 (2H, t), 2.03 (6H, s), 1.89-1.83 (2H, m), 1.23 (3H, d) ppm.
LRMS (electrospray) : m/z [M+H]⁺ 702, [M+Na]⁺ 724.

### Preparation 18 : 4-({2-[3-((2R)-2-{(2R)-2-[6-(2,5-Dimethyl-pyrrol-1-yl)-pyridin-3-yl]-2-hydroxy-ethylamino}-propyl)-phenyl]-acetylamino}-methyl)-N-[2-(naphthalen-1-ylmethoxy)-ethyl]-benzamide

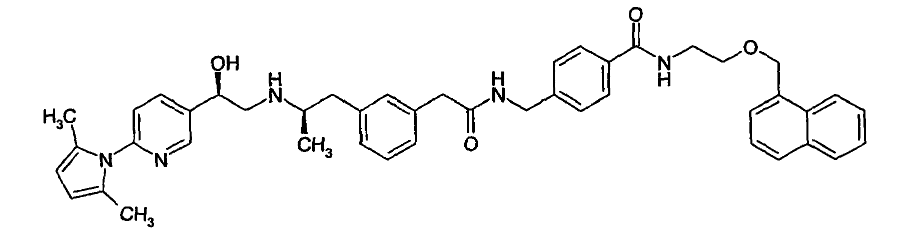

Prepared using the method for preparation 1 using the acid from preparation 38 and the amine from preparation 64 to give the title compound as a colourless foam.
¹H NMR (400MHz, CDCl₃): δ = 8.37 (1H, s), 8.11 (1H, d), 7.87-7.80 (2H, m), 7.69-7.66 (1H, m), 7.48-7.39 (6H, m), 7.32-7.28 (1H, m), 7.15-7.08 (6H, m), 6.50 (1H, m), 6.33 (1H, m), 5.87 (2H, s), 4.97 (2H, s), 4.47-4.43 (1H, m), 4.41-4.27 (2H, m), 3.71-3.69 (2H, m), 3.63-3.59 (4H, m), 2.94-2.86 (2H, m), 2.76-2.58 (3H, m), 2.07 (6H, s), 1.13 (3H, d) ppm.
LRMS (electrospray) : m/z [M+H]⁺ 724, [M+Na]⁺ 746.

### Preparation 19 : N-(4-Benzylsulfamoyl-benzyl)-4-({2-[3-((2R)-2-((2R)-2-[6-(2,5-dimethyl-pyrrol-1-yl)-pyridin-3-yl]-2-hydroxy-ethylamino}-propyl)-phenyl]-acetylamino}-methyl)-benzamide

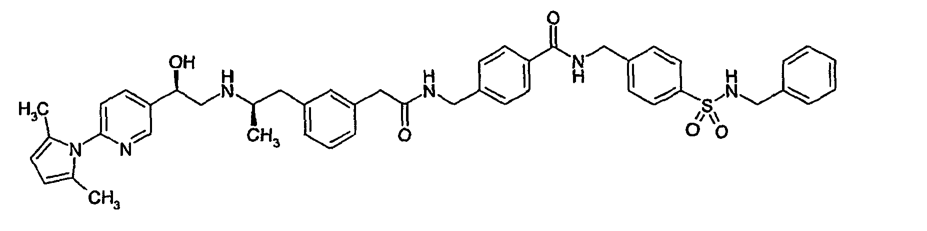

Prepared according to the method for preparation 1 using the acid from preparation 38 and the amine from preparation 67 to give the title compound as a colourless solid.
¹H NMR (400MHz, CD₃OD): δ = 8.50 (1H, s), 7.93 (1H, d), 7.78 (4H, m), 7.49 (2H, d), 7.33-7.10 (12H, m), 5.81 (2H, s), 4.84 (1H, m), 4.61 (2H, s), 4.41 (2H, s), 4.02 (2H, s), 3.55 (2H, s), 2.99 (1H, m), 2.86 (2H, m), 2.77 (1H, m), 2.60 (1H, m), 2.02 (6H, s), 1.07 (3H, d) ppm.
LRMS (APC!) : m/z [M+H]⁺ 799.

### Preparation 20 : 4-({2-(3-((2R)-2-{(2R)-2-[6-(2,5-Dimethyl-pyrrol-1-yl)-pyridin-3-yl]-2-hydroxy-ethylamino}-propyl)-phenyl]-acetylamino}-methyl)-N-(4-methylsulfamoyl-benzyl)-benzamide

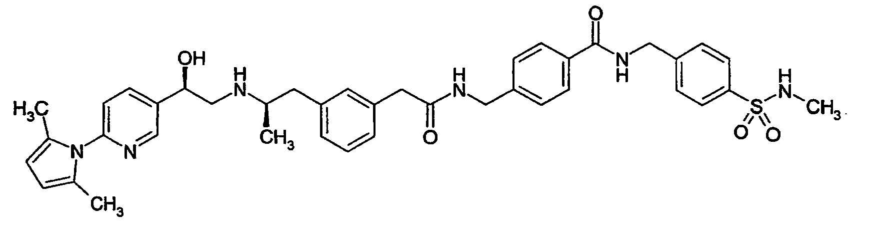

Prepared according to the method for preparation 1 using the acid from preparation 38 and the amine from preparation 65 to give the title compound as a colourless solid.
¹H NMR (400MHz, CD₃OD): δ = 8.50 (1H, s), 7.93 (1H, d), 7.78 (4H, m), 7.53 (2H, d), 7.32-7.08 (7H, m), 5.81 (2H, s), 4.81 (1H, m), 4.62 (2H, s), 4.40 (2H, s), 3.55 (2H, s), 2.97 (1H, m), 2.86 (2H, m), 2.77 (1H, m), 2.58 (1H, m), 2.49 (3H, s), 2.02 (6H, s), 1.06 (3H, d) ppm.
LRMS (APCI) : m/z [M+H]⁺ 723.

### Preparation 21 : 4-({2-[3-((2R)-2-{(2R)-2-[6-(2,5-Dimethyl-pyrrol-1-yl)-pyridin-3-yl]-2-hydroxy-ethylamino}-propyl)-phenyl]-acetylamino}-methyl)-N-(4-ethylsulfamoyl-benzyl)-benzamide

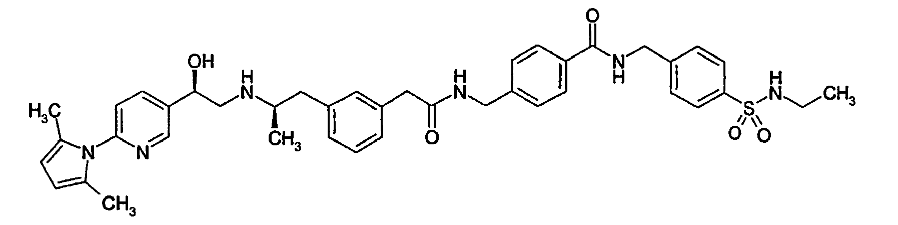

Prepared according to the method for preparation 1 using the acid from preparation 38 and the amine from preparation 66 to give the title compound as a colourless solid.
¹H NMR (400MHz, CD₃OD): δ = 8.50 (1H, s), 7.94 (1H, d), 7.80 (4H, d), 7.52 (2H, d), 7.32-7.08 (7H, m), 5.81 (2H, s), 4.81 (1H, m), 4.62 (2H, s), 4.40 (2H, s), 3.55 (2H, s), 2.99 (1H, m), 2.86 (4H, m), 2.75 (1H, m), 2.60 (1H, m), 2.02 (6H, s), 1.06 (3H, d), 1.03 (3H, t) ppm.
LRMS (APCl): m/z [M+H]⁺ 737.

### Preparation 22 : N-(3-Benzylsulfamoyl-benzyl)-4-({2-[3-((2R)-2-{(2R)-2-[6-(2,5-dimethyl-pyrrol-1-yl)-pyridin-3-yl]-2-hydroxy-ethylamino}-propyl)-phenyl]-acetylamino}-methyl)-benzamide

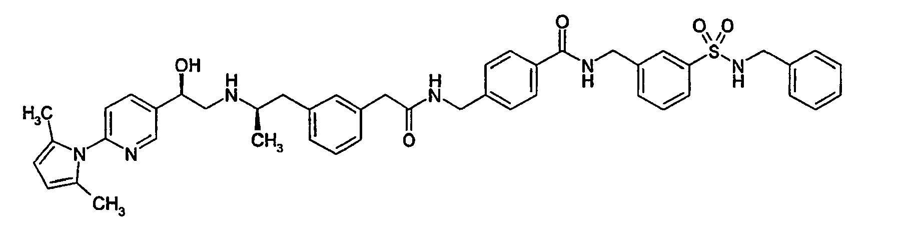

Prepared according to the method for preparation 1 using the acid from preparation 38 and the amine from preparation 70 to give the title compound as a colourless solid.
¹H NMR (400MHz, CD₃OD): δ = 8.50 (1H, s); 7.91 (1H, d), 7.79 (3H, m), 7.71 (1H, d), 7.57 (1H, d), 7.46 (1H, t), 7.31-7.07 (12H, m), 5.81 (2H, s), 4.81 (1H, m), 4.58 (2H, s), 4.40 (2H, s), 4.02 (2H, s), 3.54 (2H, s), 2.96 (1H, m), 2.85 (2H, m), 2.75 (1H, m), 2.59 (1H, m), 2.02 (6H, s), 1.06 (3H, d) ppm.
LRMS (APCI) : m/z [M+H]⁺ 799.

### Preparation 23 : 4-({2-[3-((2R)-2-{(2R)-2-[6-(2,5-Dimethyl-pyrrol-1-yl)-pyridin-3-yl]-2-hydroxy-ethylamino}-propyl)-phenyl]-acetylamino}-methyl)-N-(3-methylsulfamoyl-benzyl)-benzamide

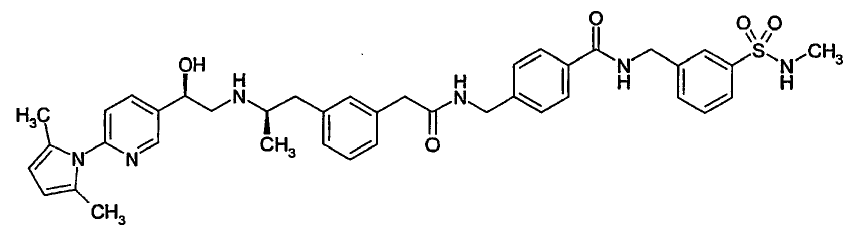

Prepared according to the method for preparation 1 using the acid from preparation 38 and the amine from preparation 68 to give the title compound as a colourless solid.
¹H NMR (400MHz, CD₃OD): δ = 8.50 (1H, s), 7.92 (1H, d), 7.82 (1H, s), 7.79 (2H, d), 7.72 (1H, d), 7.58 (1H, d), 7.52 (1H, t), 7.32-7.10 (7H, m), 5.81 (2H, s), 4.83 (1H, m), 4.62 (2H, s), 4.40 (2H, s), 3.55 (2H, s), 2.99 (1H, m), 2.86 (2H, m), 2.76 (1H, m), 2.60 (1H, m), 2.49 (3H, s), 2.02 (6H, m), 1.07 (3H, d) ppm.
LRMS (APCI) : m/z [M+H]⁺ 723.

### Preparation 24 : 4-({2-[3-((2R)-2-{(2R)-2-[6-(2,5-Dimethyl-pyrrol-1-yl)-pyridin-3-yl]-2-hydroxy-ethylamino}-propyl)-phenyl]-acetylamino}-methyl)-N-(3-ethylsulfamoyl-benzyl)-benzamide

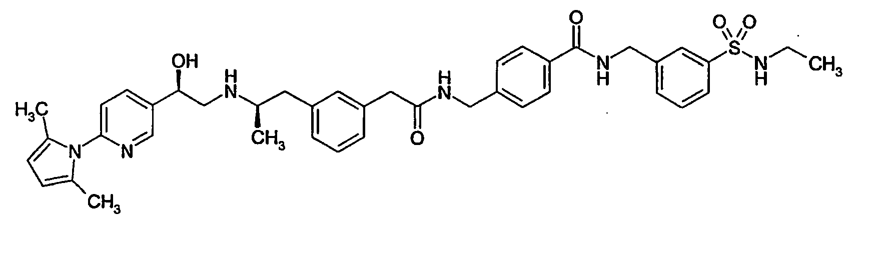

Prepared according to the method for preparation 1 using the acid from preparation 38 and the amine from preparation 69 to give the title compound as a colourless solid.
¹H NMR (400MHz, CD₃OD): δ = 8.50 (1H, s), 7.94 (1H, d), 7.83 (1H, s), 7.79 (2H, d), 7.73 (1H, d), 7.59 (1H, d), 7.50 (1H, m), 7.32-7.08 (7H, m), 5.81 (2H, s), 4.81 (1H, m), 4.61 (2H, s), 4.40 (2H, s), 3.55 (2H, s), 2.99 (1H, m), 2.87 (4H, m), 2.76 (1H, m), 2.60 (1H, m), 2.02 (6H, s), 1.07 (3H, d), 1.01 (3H, t) ppm.
LRMS (APCl) : m/z [M+H]⁺ 737.

### Preparation 25 : N-(4-Benzylsulfamoyl-benzyl)-2-[3-((2R)-2-{(2R)-2-[6-(2,5-dimethyl-pyrrol-1-yl)-pyridin-3-yl]-2-hydroxy-ethylamino}-propyl)-phenyl]-acetamide

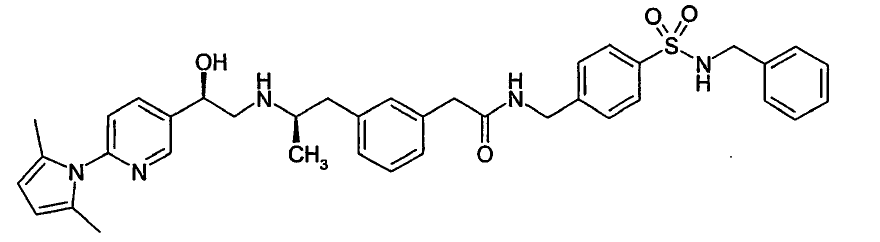

Prepared using the method for preparation 1 using the acid from preparation 40 and the amine from preparation 67 to give the title compound as a colourless solid.
¹H NMR (400MHz, CDCl₃): δ = 8.38 (1H, s), 7.70-7.65 (3H, m), 7.29-7.10 (12H, m), 6.66-6.63 (1H, m), 5.86 (2H, s), 4.51-4.48 (1 H, m), 4.39-4.26 (2H, m), 4.03 (2H, s), 3.60 (2H, s), 2.97-2.87 (2H, m), 2.75-2.59 (3H, m), 2.04 (6H, s), 1.14 (3H, d) ppm.
LRMS (APCI) : m/z [M+H]⁺ 666.

### Preparation 26 : N-(3-Benzylsulfamoyl-benzyl)-2-[3-((2R)-2-{(2R)-2-[6-(2,5-dimethyl-pyrrol-1-yl)-pyridin-3-yl]-2-hydroxy-ethylamino}-propyl)-phenyl]-acetamide

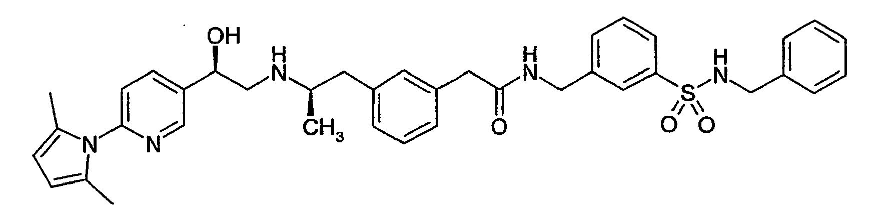

Prepared using the method for preparation 1 using the acid from preparation 40 and the amine from preparation 70 to give the title compound as a colourless solid.
¹H NMR (400MHz, CDCl₃): δ = 8.37 (1H, s), 7.66-7.60 (3H, m), 7.35-7.11 (12H, m), 6.72 (1H, m), 5.86 (2H, s), 4.48-4.45 (1H, m), 4.34-4.24 (2H, m), 4.01 (2H, s), 3.58 (2H, s), 2.96-2.55 (5H, m), 2.01 (6H, s), 1.14 (3H, d) ppm.
LRMS (APCI) : m/z [M+H]⁺ 666.

### Preparation 27 : N-(3,4-Dimethoxy-benzyl)-4-({2-[4-(2-{(2R)-2-[6-(2,5-dimethyl-pyrrol-1-yl)-pyridin-3-yl]-2-hydroxy-ethylamino}-propyl)-phenyl]-acetylamino}-methyl)-benzamide

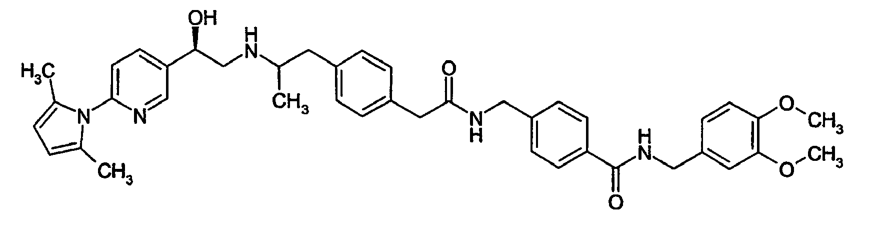

Prepared according to the method for preparation 1 using the acid from preparation 42 and the appropriate amine to give the title compound as a pale yellow foam which was used without further purification.
LRMS (APCI) : m/z [M+H]⁺ 690.

### Preparation 28 : 4-({2-[4-(2-{(2R)-2-[6-(2,5-Dimethyl-pyrrol-1-yl)-pyridin-3-yl]-2-hydroxy-ethylamino}-propyl)-phenyl]-acetylamino}-methyl)-N-(2-ethoxy-benzyl)-benzamide

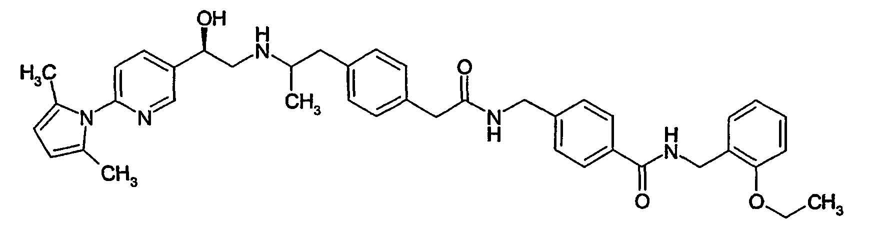

Prepared according to the method for preparation 1 using the acid from preparation 42 and the appropriate amine to give the title compound as a pale yellow foam which was used without further purification.
LRMS (APCI) : m/z [M+H]⁺ 674.

### Preparation 29 : 4-({2-[4-(2-{(2R)-2-[6-(2,5-Dimethyl-pyrrol-1-yl)-pyridin-3-yl]-2-hydroxy-ethylamino}-propyl)-phenyl]-acetylamino}-methyl)-N-[(1R)-1-(4-methoxy-phenyl)-ethyl]-benzamide

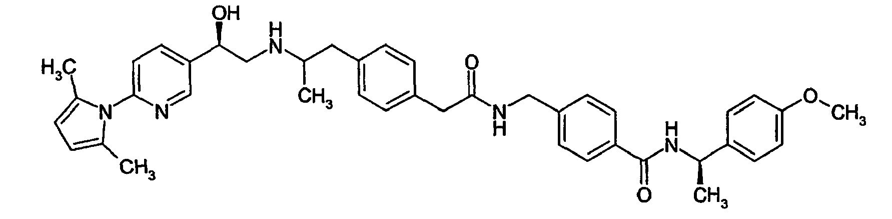

Prepared according to the method for preparation 1 using the acid from preparation 42 and the appropriate amine to give the title compound as a pale yellow foam which was used without further purification.
LRMS (APCl): m/z [M+H]⁺ 674.

### Preparation 30 : 4-({2-[4-(2-{(2R)-2-[6-(2,5-Dimethyl-pyrrol-1-yl)-pyridin-3-yl]-2-hydroxy-ethylamino}-propyl)-phenyl]-acetylamino}-methyl)-N-((1R)-1-hydroxymethyl-2-phenyl-ethyl)-benzamide

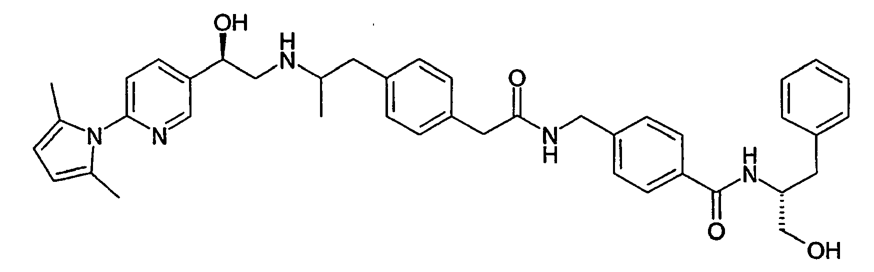

Prepared according to the method for preparation 1 using the acid from preparation 42 and the appropriate amine to give the title compound as a pale yellow foam which was used without further purification.
LRMS (APCI): m/z [M+H]⁺ 674.

### Preparation 31: :4-({2-[4-(2-{(2R)-2-[6-(2,5-Dimethyl-pyrrol-1-yl)-pyridin-3-yl]-2-hydroxy-ethylamino}-propyl)-phenyl]-acetylamino}-methyl)-N-((1R, 2S)-2-hydroxy-1-methyl-2-phenyl-ethyl)-benzamide

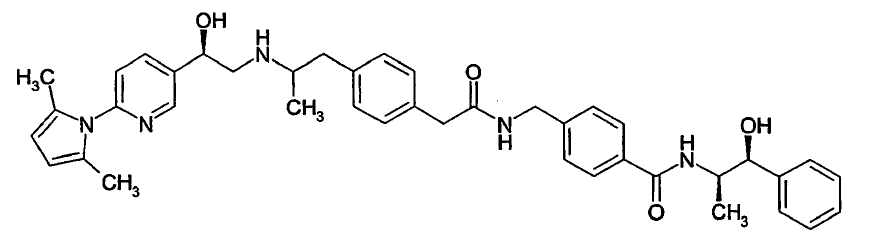

Prepared according to the method for preparation 1 using the acid from preparation 42 and the appropriate amine to give the title compound as a pale yellow foam which was used without further purification.
LRMS (APCI) : m/z [M+H]⁺ 674.

### Preparation 32 : 4-({2-[4-(2-{(2R)-2-[6-(2,5-Dimethyl-pyrrol-1-yl)-pyridin-3-yl]-2-hydroxy-ethylamino}-propyl)-phenyl]-acetylamino}-methyl)-N-((1S, 2R)-2-hydroxy-1-methyl-2-phenyl-ethyl)-benzamide

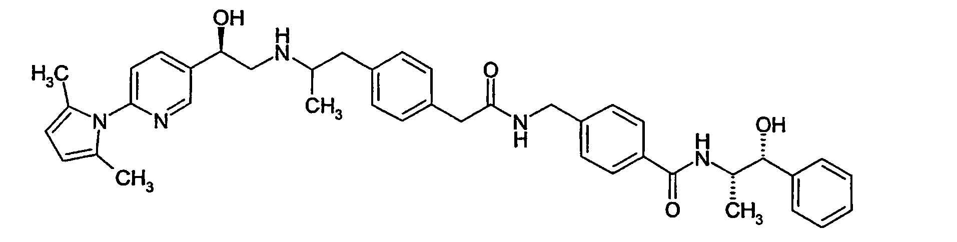

Prepared according to the method for preparation 1 using the acid from preparation 42 and the appropriate amine to give the title compound as a pale yellow foam which was used without further purification.
LRMS (APCI) : m/z [M+H]⁺ 674.

### Preparation 33 : 4-({2-[4-(2-{(2R)-2-[6-(2,5-Dimethyl-pyrrol-1-yl)-pyridin-3-yl]-2-hydroxy-ethylamino}-propyl)-phenyl]-acetylamino}-methyl)-N-[2-(3-methoxy-phenyl)-ethyl]-benzamide

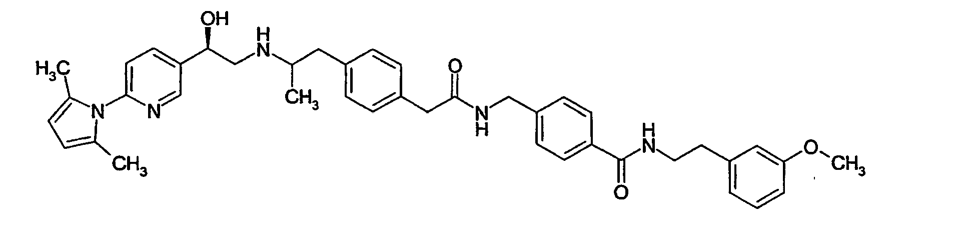

Prepared according to the method for preparation 1 using the acid from preparation 42 and the appropriate amine to give the title compound as a pale yellow foam which was used without further purification.
LRMS (APCI) : m/z [M+H]⁺ 674.

### Preparation 34 : 4-({2-[3-(2-{(2R)-2-[6-(2,5-Dimethyl-pyrrol-1-yl)-pyridin-3-yl]-2-hydroxy-ethylamino}-propyl)-phenyl]-acetylamino}-methyl)-benzoic acid

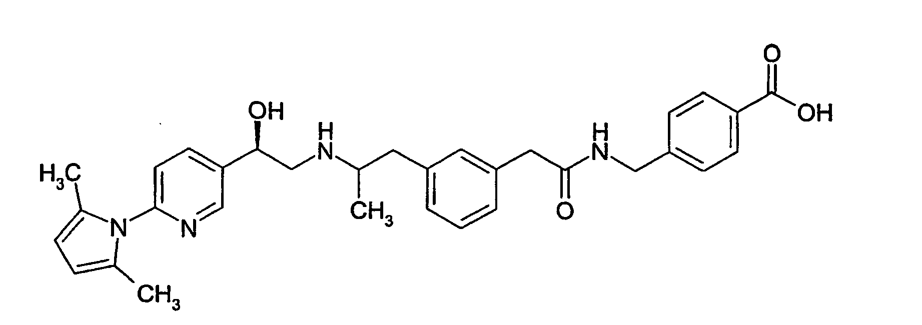

A solution of the ester from preparation 35 (1.31 g, 2.36mmol) in tetrahydrofuran (10ml) was treated with 1N lithium hydroxide (4.7ml, 4.7mmol) and the resulting mixture left to stir at room temperature for 16 hours. The solvent was removed *in vacuo* and the residue purified by flash column chromatography eluting with dichloromethane:methanol:880 ammonia (90:10:1 by volume) to give the title compound (1:1 mixture of diastereoisomers) as a colourless solid (0.88g).
¹H NMR (400MHz, CD₃OD): δ = 8.72-8.58 (1H, m), 8.14-8.04 (1H, m), 7.95-7.84 (2H, m), 7.45-7.07 (7H, m), 5.83 (2H, s), 5.17-5.04 (1H, m), 4.39 (2H, s), 3.55 (2H, s), 3.46-3.01 (4H, m), 2.77-2.58 (1H, m), 2.05 (6H, s), 1.23-1.01 (3H, m) ppm.
LRMS (electrospray): m/z [M+H]⁺ 541, [M+Na]⁺ 563.

### Preparation 35 : 4-({2-[3-(2-{(2R)-2-[6-(2,5-Dimethyl-pyrrol-1-yl)-pyridin-3-yl]-2-hydroxy-ethylamino}-propyl)-phenyl]-acetylamino}-methyl)-benzoic acid methyl ester

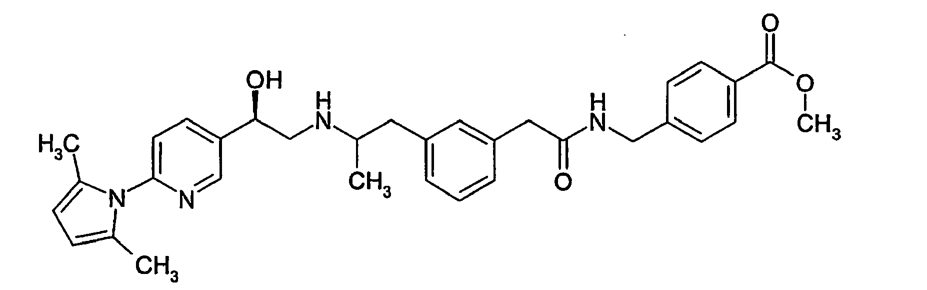

Prepared using the procedure for preparation 1 using the acid from preparation 36 and the appropriate amine to give the title compound (1:1 mixture of diastereoisomers) as a pale yellow oil.
¹H NMR (400MHz, CDCl₃): δ = 8.46-8.42 (1H, m), 7.94-7.90 (2H, m), 7.76-7.67 (1H, m), 7.32-7.10 (7H, m), 6.31-6.20 (1H, m), 5.88 (2H, s), 4.72-4.51 (1H, m), 4.44-4.33 (2H, m), 3.88 (3H, s), 3.64-3.61 (2H, m), 3.13-3.11 (1H, m), 2.85-2.57 (4H, m), 2.08-2.07 (6H, s), 1.19-1.14 (3H, m) ppm.
LRMS (electrospray) : m/z [M+H]⁺ 555, [M+Na]⁺ 577.

### Preparation 36: [3-(2-{(2R)-2-[6-(2,5-Dimethyl-pyrrol-1-yl)-pyridin-3-yl]-2-hydroxy-ethylamino}-propyl)-phenyl]-acetic acid

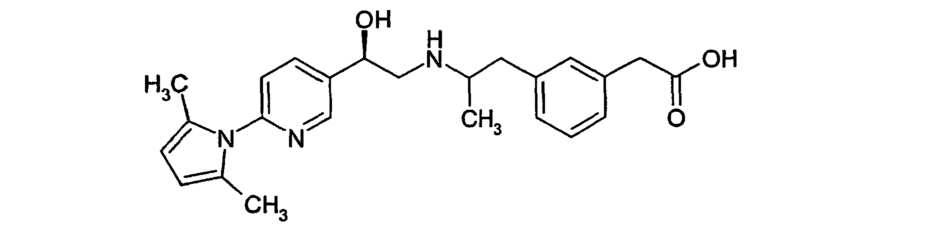

Prepared using the method for preparation 34 using the ester from preparation 37 to give the title compound (1:1 mixture of diastereoisomers) as an off white solid.
¹H NMR (400MHz, DMSO_{d6}): δ = 8.55 (1H, s), 7.93 (1H, t), 7.35 (1H, d), 7.18-7.15 (1H, m), 7.10-7.03 (3H, m), 5.77 (2H, s), 4.87-4.80 (1H, m), 3.48 (2H, s), 3.01-2.89 (4H, m), 2.50-2.40 (1H, m, partially masked by solvent), 2.01 (6H, s), 0.96 (3H, d) ppm.
LRMS (electrospray) : m/z [M+H]⁺ 408.

### Preparation 37 : [3-(2-{(2R)-2-[6-(2,5-Dimethyl-pyrrol-1-yl)-pyridin-3-yl]-2-hydroxy-ethylamino}-propyl)-phenyl]-acetic acid methyl ester

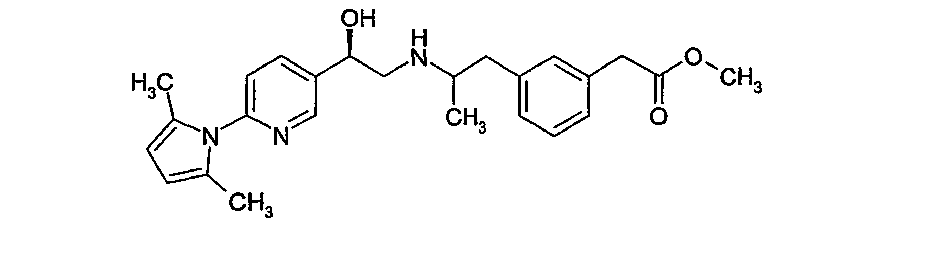

Prepared according to the procedure for preparation 52 using the ketone from preparation 53 and the amine from preparation 46 to give the title compound (1:1 mixture of diastereoisomers) as a pale yellow oil.
¹H NMR (400MHz, CDCl₃): δ = 8.56 (1H, s), 7.87-7.84 (1H, m), 7.30-7.26 (1H, m, partially masked by solvent), 7.20-7.09 (4H, m), 5.88 (2H, s), 4.97-4.87 (1H, m), 3.69 (3H, s), 3.62 (2H, s), 3.18-3.03 (2H, m), 2.94-2.74 (3H, m), 2.10 (6H, s), 1.22 (3H, d) ppm.
LRMS (electrospray) : m/z [M+H]⁺ 422.

### Preparation 38 : 4-({2-[3-((2R)-2-{(2R)-2-[6-(2,5-Dimethyl-pyrrol-1-yl)-pyridin-3-yl]-2-hydroxy-ethylamino}-propyl)-phenyl]-acetylamino}-methyl)-benzoic acid

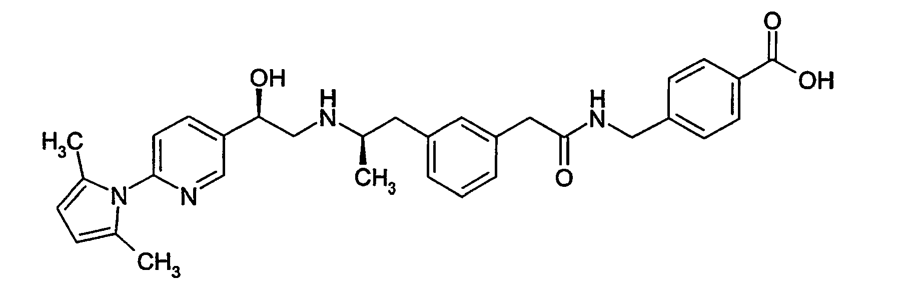

Prepared using the method for preparation 34 using the ester from preparation 39 to give the title compound as a colourless solid.
¹H NMR (400MHz, CD₃OD): δ = 8.70 (1H, m), 8.64 (1H, d), 8.09 (1H, d), 7.93 (2H, d), 7.41 (1H, d), 7.34-7.19 (5H, m), 5.83 (2H, s), 5.12 (1H, m), 4.44 (2H, s), 3.59 (3H, m), 3.39 (2H, m), 3.22 (1H, m), 2.82 (1H, m), 2.05 (6H, s), 1.28 (3H, d) ppm.
LCMS : m/z [M+H]⁺ 541.
Optical Rotation [α]^{D}₂₅ = -27.71° c=1, methanol

### Preparation 39 : 4-({2-[3-((2R)-2-{(2R)-2-[6-(2,5-Dimethyl-pyrrol-1-yl)-pyridin-3-yl]-2-hydroxy-ethylamino}-propyl)-phenyl]-acetylamino}-methyl)-benzoic acid methyl ester

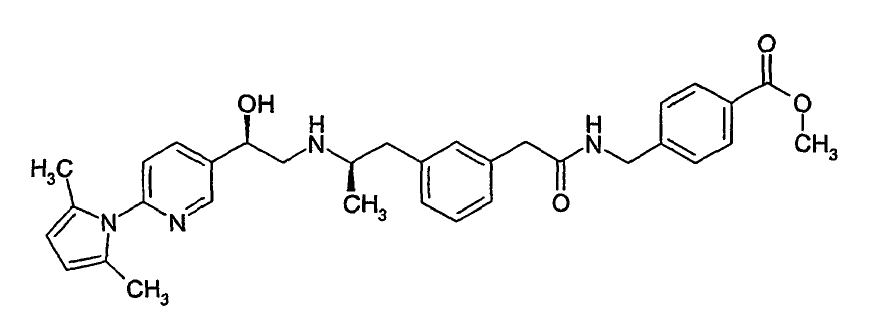

Prepared using the procedure for preparation 1 using the acid from preparation 40 and the appropriate amine to give the title compound as a pale yellow solid.
¹H NMR (400MHz, CD₃OD) δ = 8.62 (1H, s), 8.06 (1H, d), 7.92 (2H, d), 7.70 (1H, d), 7.68 (1H, d), 7.37-7.16 (5H, m), 5.83 (2H, s), 5.11 (1H, m), 4.42 (2H, s), 3.87 (3H, s), 3.60-3.52 (3H, m), 3.38-3.24 (2H, m, partially obscured by solvent), 3.18 (1H, m), 2.80 (1H, m), 2.04 (6H, s), 1.26 (3H, d) ppm.
LRMS (APCl) : m/z [M+H]⁺ 555.
Optical Rotation [α]^{D}₂₅ = -27.71° c=1, methanol

### Preparation 40 : [3-((2R)-2-{(2R)-2-[6-(2,5-Dimethyl-pyrrol-1-yl)-pyridin-3-yl]-2-hydroxy-ethylamino}-propyl)-phenyl]-acetic acid

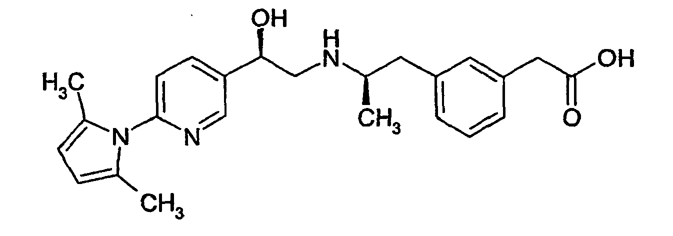

Prepared using the method for preparation 34 using the ester from preparation 41 to give the title compound as a colourless solid.
¹H NMR (400MHz, DMSO_{d6}): δ = 8.54 (1H, bs), 7.92-7.90 (1H, m), 7.33 (1H, d), 7.16 (1H, d), 7.07-7.00 (3H, m), 5.77 (2H, s), 4.77-4.74 (1H, m), 3.47 (2H, s), 2.91-2.75 (4H, m), 2.43-2.38 (1H, m), 2.01 (6H, s), 0.92 (3H, d) ppm.
LRMS (APCI): m/z [M+H]⁺ 408.

### Preparation 41 : [3-((2R)-2-{(2R)-2-[6-(2,5-Dimethyl-pyrrol-1-yl)-pyridin-3-yl]-2-hydroxy-ethylamino}-propyl)-phenyl]-acetic acid methyl ester

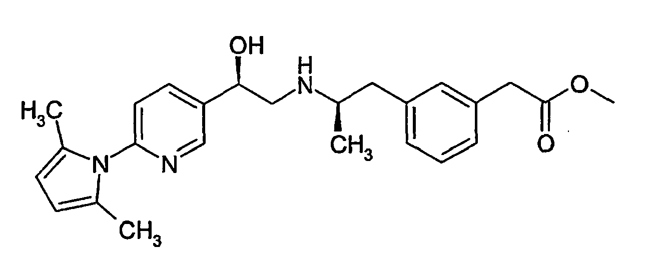

A solution of the epoxide from preparation 48 (5.43g of 66% b/w crude material, 3.58g, 16.7mmol) and the amine from preparation 51 (4.15g, 20.02mmol) in dimethyl sulfoxide (50ml) was heated at 85°C under nitrogen for a period of 16 hours. The reaction mixture was cooled to room temperature and loaded directly onto a Strong Cation Exchange column. The column was eluted with methanol (300ml) and then the product eluted with 2M ammonia in methanol (100ml). The solvent was removed *in vacuo* and the residue purified by flash column chromatography eluting with dichloromethane:methanol:880 ammonia (100 changing to 98:2:0.2 by volume) to give the title compound as a pale orange oil (5.45g).
¹H NMR (400MHz, CD₃OD): δ = 8.54 (1H, bs), 7.79 (1H, dd), 7.31-7.22 (2H, m), 7.15-7.10 (3H, m), 5.82 (2H, s), 491-4.81 (1H, m, partially obscured by solvent), 3.67 (3H, s), 3.63 (2H, s), 3.04-2.96 (1H, m), 2.88 (2H, d), 2.81-2.74 (1H, m), 2.66-2.58 (1H, m), 2.04 (6H, s), 1.09 (3H, d) ppm.
LRMS (electrospray) : m/z [M+H]⁺ 422, [M+Na]⁺ 444, [M-H]⁻ 420.

### Preparation 42: 4-({2-[4-(2-{(2R)-2-[6-(2,5-Dimethyl-pyrrol-1-yl)-pyridin-3-yl]-2-hydroxy-ethylamino}-propyl)-phenyl]-acetylamino}-methyl)-benzoic acid

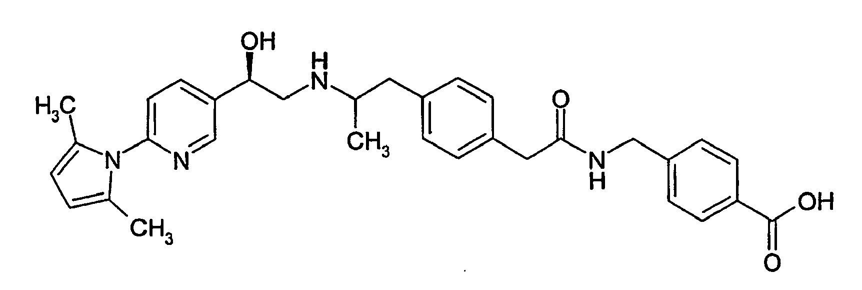

Prepared using the method for preparation 34 using the ester from preparation 43 to give the title compound (1:1 mixture of diastereoisomers) as a pale yellow solid which was used without further purification.
LRMS (electrospray) : m/z [M+H]⁺ 541, [M-H]⁻ 539.

### Preparation 43 : 4-({2-[4-(2-{(2R)-2-[6-(2,5-Dimethyl-pyrrol-1-yl)-pyridin-3-yl]-2-hydroxy-ethylamino}-propyl)-phenyl]-acetylamino}-methyl)-benzoic acid methyl ester

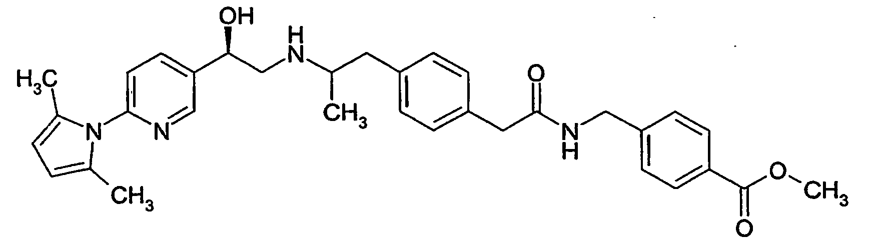

Prepared using the method for preparation 1 using the acid from preparation 44 and the appropriate amine to give the title compound (1:1 mixture of diastereoisomers) as an off white solid.
¹H NMR (400MHz, CD₃OD): δ = 8.50 (1H, d), 7.92 (3H, m), 7.32-7.16 (7H, m), 5.81 (2H, s), 4.81 (1H, m), 4.41 (2H, s), 3.87 (3H, s), 3.53 (2H, d), 2.98-2.75 (5H, m), 2.03 (6H, s), 1.08 (3H, d) ppm.
LRMS (electrospray) : m/z [M+H]⁺ 555.

### Preparation 44 : [4-(2-{(2R)-2-[6-(2,5-Dimethyl-pyrrol-1-yl)-pyridin-3-yl]-2-hydroxy-ethylamino}-propyl)-phenyl]-acetic acid

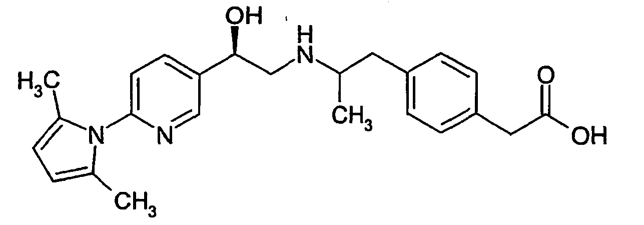

Prepared according to the procedure for preparation 34 using the ester from preparation 45 to give the title compound (1:1 mixture of diastereoisomers) as a colourless solid
¹H NMR (400MHz, DMSO_{d6}): δ = 8.55 (1H, s), 7.91 (1H, m), 7.36 (1H, d), 7.14 (4H, m), 5.77 (2H, s), 4.80 (1H, m), 3.50 (2H, bs), 2.94 (2H, m), 2.90 (3H, m), 2.01 (6H, s), 0.95 (3H, d) ppm.
LRMS (electrospray) : m/z [M+H]⁺ 408, [M-H]⁻ 406.

### Preparation 45 : [4-(2-{(2R)-2-[6-(2,5-Dimethyl-pyrrol-1-yl)-pyridin-3-yl]-2-hydroxy-ethylamino}-propyl)-phenyl]-acetic acid methyl ester

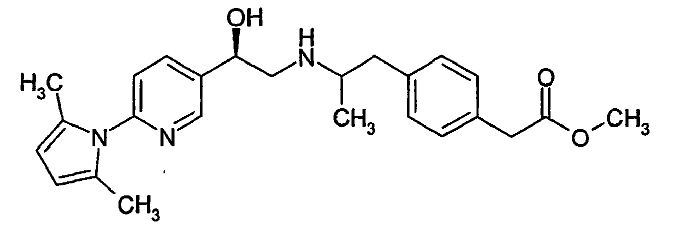

Prepared according to the procedure for preparation 52 using the ketone from preparation 55 and the amine from preparation 46 to give the title compound (1:1 mixture of diastereoisomers) as a yellow gum.
¹H NMR (400MHz, CDCl₃): δ = 8.53 (1H, m), 7.83-7.79 (1H, m), 7.26-7.13 (5H, m), 5.88 (2H, s), 4.73-4.64 (1H, m), 3.69 (3H, s), 3.60 (2H, s), 3.09-2.96 (2H, m), 2.79-2.63 (3H, m), 2.09 (6H, s), 1.13 (3H, t) ppm.
LRMS (electrospray) : m/z [M+H]⁺ 422.

### Preparation 46 : (1R)-2-Amino-1-[6-(2,5-dimethyl-pyrrol-1-yl)-pyridin-3-yl]-ethanol

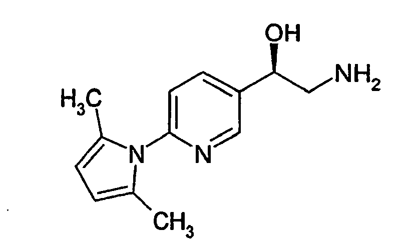

A solution of the phthalimide from preparation 47 (4.85g, 13.4mmol) in 8M methylamine in ethanol (50 ml) was stirred under a nitrogen atmosphere at room temperature for 18 hours. The reaction was concentrated under reduced pressure and the residue was dissolved in methanol. This solution was passed through a Strong Cation Exchange resin cartridge eluting with methanol and then 2N ammonia in methanol to elute the product. The eluent was concentrated *in vacuo* and the residue purified by flash column chromatography on silica gel eluting with dichloromethane:methanol:880 ammonia (95:5:0 changing to 90:10:1, by volume) to give the title compound as a pale yellow solid (1.6g).
¹H NMR (400MHz, CDCl₃): δ = 8.17 (1H, s), 7.85 (1H, d), 7.21 (1H, d), 5.89 (2H, s), 4.69 (1H, t), 3.15-3.11 (1H, dd), 2.85-2.80 (1H, dd), 2.11 (6H, s) ppm.
LRMS (electrospray) : m/z [M+H]⁺ 232, [M+Na]⁺,254.

### Preparation 47 : 2-{(2R)-2-[6-(2,5-Dimethyl-pyrrol-1-yl)-pyridin-3-yl]-2-hydroxy-ethyl}-isoindole-1,3-dione

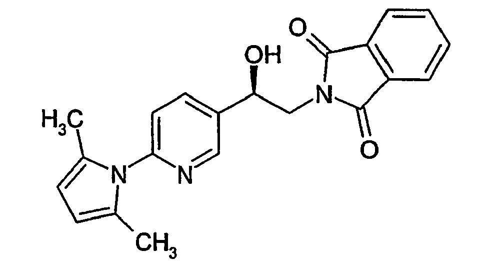

A solution of the crude epoxide from preparation 48 (30.0g of 65% b/w epoxide, 19.50g, 91.0mmol), phthalimide (12.51g, 85.0mmol) and potassium phthalimide (2.78g, 15.0mmol) in *N,N*-dimethylformamide (200ml) was heated at 90°C under nitrogen for 6 hours. After cooling the reaction was stirred at room temperature for 18 hours, concentrated *in vacuo* and the residue partitioned between dichloromethane (600ml) and water (400ml). The organic phase was separated and the aqueous phase extracted with further dichloromethane (200ml). The combined organic extracts were dried (magnesium sulfate) and concentrated *in vacuo.* Crystallisation (ethyl acetate, 300ml) gave the title compound as a pale yellow crystalline solid (22.1 g).
¹H NMR (400MHz, DMSO-d₆): δ = 8.42 (1H, s), 7.90 (1H, d), 7.80 (4H, d), 7.30 (1H, d), 5.90 (1H, s), 5.80 (2H, s), 5.00 (1H, brs), 3.82 (1H, m), 3.75 (1H, m), 1.95 (6H, s) ppm.
LRMS (electrospray) : m/z [M+H]⁺ 362.

### Preparation 48 : 2-(2,5-Dimethyl-pyrrol-1-yl)-5-[(2R)-oxiranyl]pyridine

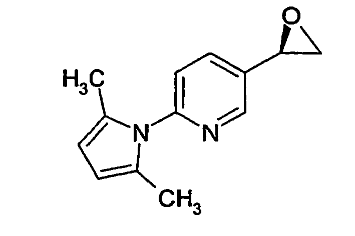

A solution of the chloride from preparation 49 (12.0g, 48.1 mmol) in tetrahydrofuran (20ml) was slowly added to a solution of (-)-*B-*chlorodiisopinocampheylborane (20.1 g, 62.5mmol) in *tert*-butyl methylether (15ml) and tetrahydrofuran (30ml) at -30°C under nitrogen. The reaction was stirred for 6 hours at -30°C and then sodium perborate tetrahydrate (7.4g, 48.1 mmol) followed by *tert-*butyl methylether (50ml) were added. The reaction was stirred at room temperature for 18 hours, treated with 2M aqueous sodium hydroxide (190ml) and stirred for a further 6 hours. The organic phase was separated and the aqueous phase extracted with further *tert-*butyl methylether (50ml). The combined organic extracts were washed with 1M aqueous sodium hydroxide (50ml), saturated aqueous sodium chloride (50 ml), dried (sodium sulfate) and concentrated *in vacuo.* The residue was purified by flash column chromatography on silica gel eluting with pentane:dichloromethane (80:2 changing to 100:0, by volume) to give the crude epoxide (65% b/w, 11.0 g), which was used without further purification.
¹H NMR (400MHz, CDCl₃): δ = 8.58 (1H, bs), 7.68-7.66 (1H, dd), 7.22-7.20 (1H, d), 5.81 (2H, s), 3.97-3.96 (1H, m), 3.26-3.24 (1H, m), 2.91-2.89 (1H, m), 2.12 (6H, s) ppm.
LRMS (electrospray) : m/z [M+H]⁺ 215, [M+Na]⁺, 237.

### Preparation 49 : 2-Chloro-1-[6-(2,5-dimethyl-pyrrol-1-yl)-pyridin-3-yl]-ethanone

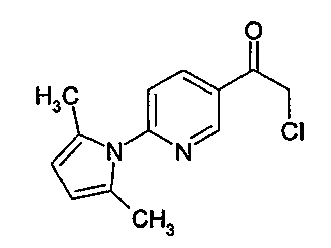

A solution of 2.5 M *n*-butyl lithium in hexane (35ml, 87.6mmol) was added to a solution of the bromide from preparation 50 (20.0g, 79.7mmol) in *tert-*butyl methylether (300ml) at -78°C under nitrogen over a 10 minutes. The reaction was stirred for a further 10 minutes and 2-chloro-N-methoxy-N-methylacetamide (12.1g, 87.6mmol) in *tert*-butyl methylether (40ml) was added slowly. The reaction was stirred at -78°C for 20 minutes and then 1M hydrochloric acid (200ml) was added. The mixture was allowed to warm to room temperature, stirred for 2 hours and the organic phase separated. The aqueous phase was extracted with *tert*-butyl methylether and the combined organic extracts were washed with water (100ml), saturated aqueous sodium chloride (100ml) and 1M sodium hydroxide (100ml). The organic phase was dried (sodium sulfate), concentrated *in vacuo* and the residual oil purified by flash column chromatography on silica gel eluting with pentane:dichloromethane:methanol (75:25:0 changing tp 0:99:1, by volume). Recrystallisation (pentane:dichloromethane) gave the title compound as a yellow solid (11.97g).
¹H NMR (400MHz, CDCl₃): δ = 9.11 (1H, s), 8.34-8.33 (1H, d), 7.32-7.30 (1H, d), 5.91 (2H, s), 4.66 (2H, s), 2.17 (6H, s) ppm.
LRMS (thermospray): m/z [M-H]⁺ 247.

### Preparation 50 : 5-Bromo-2-(2,5-dimethyl-pyrrol-1-yl)-pyridine

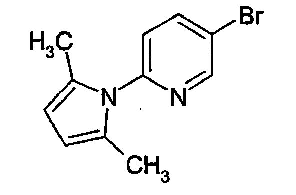

2,5-hexanedione (46.2g, 0.41 mol) was added to a suspension of 2-amino-5-bromopyridine (50.0g, 0.29mol) and the reaction heated to reflux for 24 hours under Dean and Stark conditions. *para*-Toluene sulfonic acid (100mg) was added and the reaction was refluxed for a further 18 hours. 8 ml of water were removed, so the reaction was cooled to room temperature, washed with water (100ml) and passed through a plug of silica gel, eluting with toluene. The eluent was concentrated *in vacuo* and the residue dissolved in pentane:dichloromethane (1:1 by volume) and passed through a plug of silica gel, eluting with pentane:dichloromethane (1:1 by volume). The eluent was concentrated *in vacuo* to give a red liquid, which solidified on standing. The solid was recrystallised from isopropanol to give the title compound as a pale yellow solid (54.4g).
¹H NMR (400MHz, CDCl₃): δ = 8.66 (1H, s), 7.93-7.92 (1H, d), 7.13-7.11 (1H, d), 5.91 (2H, s), 2.13 (6H, s) ppm.
LRMS (thermospray) : m/z [M+H]⁺ 252.

### Preparation 51 : [3-((2R)-2-Amino-propyl)-phenyl]-acetic acid methyl ester

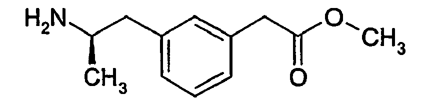

A solution of the amine from preparation 52 (7.69g, 22mmol) and ammonium formate (6.94 g, 110 mmol) was heated to 75°C in the presence of 20% of palladium hydroxide-on-charcoal (2.00g). After 90 minutes the reaction mixture was cooled to room temperature, filtered through arbocel® and the filtrate concentrated *in vacuo.* The residue was partitioned between dichloromethane (100ml) and 880 ammonia (100ml) and the organic phase separated. The aqueous phase was extracted with dichloromethane (100ml) and the combined organic extracts dried (magnesium sulfate) and reduced *in vacuo* to give the title compound as a colourless oil (4.78g).
¹H NMR (400MHz, CD₃OD): δ = 7.27-7.23 (1H, t), 7.13-7.09 (3H, m), 3.67 (3H, s), 3.63 (2H, s), 3.12-3.05 (1H, m), 2.67-2.57 (2H, m), 1.06 (3H, d) ppm.
LRMS (electrospray) : m/z [M+H]⁺ 208, [M+Na]⁺ 230.

### Preparation 52: {3-[(2R)-2-((1R)-1-Phenyl-ethylamino)-propyl]-phenyl}-acetic acid methyl ester

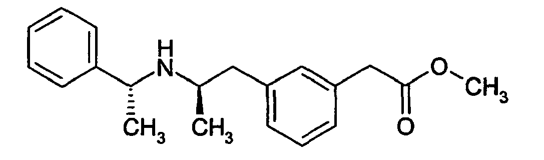

A solution of the ketone from preparation 53 (8.5g, 41.2mmol), (*R*)-α-methyl benzylamine (4.8ml, 37.2mmol), sodium triacetoxyborohydride (11.6g, 56mmol) and acetic acid (2.2ml, 38mmol) in dichloromethane (400ml) was stirred at room temperature for 48 hours. The reaction mixture was quenched by addition of saturated aqueous sodium bicarbonate (200ml) and allowed to stir until effervescence ceased. The organic phase was separated and the aqueous phase extracted with dichloromethane (100ml). The combined organic extracts were dried (magnesium sulfate) and reduced *in vacuo.* Purification by flash column chromatography eluting with dichloromethane: methanol: 880 ammonia (99:1:0.1 changing to 95:5:0.5 by volume) gave a 4:1 mixture of diastereomers (R,R major) as a pale yellow oil (8.71 g). Treatment with 1M hydrogen chloride in methanol followed by three successive crystallisations (diisopropylether/methanol) gave the title compound as a colourless crystalline solid (5.68g).
¹H NMR (400MHz, CD₃OD): δ = 7.52-7.48 (5H, m), 7.28-7.25 (1H, m), 7.18-7.16 (1H, m), 7.02-6.99 (2H, m), 4.59 (1H, q), 3.62 (2H, s), 3.30 (3H, s), 3.30-3.25 (1H, m), 3.26-3.15 (1H, m), 2.66-2.60 (1H, m), 1.68 (3H, d), 1.18, (3H, d) ppm.
LRMS (electrospray) : m/z [M+H]⁺ 312, [M+Na]⁺ 334.

### Preparation 53 : [3-(2-Oxo-propyl)-phenyl]-acetic acid methyl ester

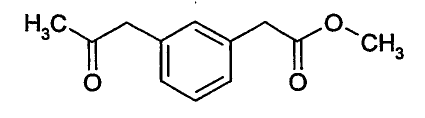

A solution of the bromide from preparation 26 (15.0g, 65.0mmol), tributyltin methoxide (28.3ml, 98mmol), isopropenyl acetate (10.8ml, 98.0mmol), palladium(II) acetate (750mg, 3.30mmol) and tri-*ortho*-tolylphosphine (2.0g, 6.5 mmol) were stirred together in toluene (75ml) at 100°C under nitrogen for 5 hours. After cooling the reaction was diluted with ethyl acetate (150ml) and 4M aqueous potassium fluoride solution (90ml) and stirred for 15 minutes. The mixture was filtered through arbocel and the organic phase separated and reduced *in vacuo*. The residue was purified by flash column chromatography silica gel eluting with a solvent gradient of diethyl ether:pentane:dichloromethane (0:100:0 changing to 25:75:0 then to 0:0:100, by volume) to give the title compound as a pale yellow oil (12.6g).
¹H NMR (400MHz, CDCl₃): δ = 7.30 (1H, t), 7.19 (1H, d), 7.13-7.10 (2H, m), 3.69 (5H, s), 3.61 (2H, s), 2.15 (3H, s) ppm.
LRMS (electrospray) : m/z [M+NH₄]⁺ 224, [M+Na]⁺ 229.

### Preparation 54 : (3-Bromo-phenyl)-acetic acid methyl ester

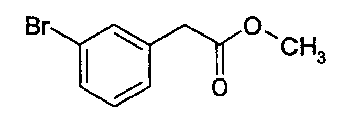

Acetyl chloride (0.7ml, 9.3mmol) was slowly added to a solution of (3-bromophenyl)-acetic acid (20.0g, 93mmol) in methanol (500ml) at 0°C under nitrogen and the reaction was allowed to warm gradually to room temperature over a period of 5 hours. The solvent was removed *in vacuo* and the residue dissolved in dichloromethane, dried (sodium sulfate) and concentrated *in vacuo* to give the title compound as a colourless oil (20.6g).
¹H NMR (400MHz, CDCl₃): δ = 7.37-7.45 (2H, m), 7.24-7.17 (2H, m), 3.70 (3H, s), 3.59 (2H, s) ppm.
LRMS (electrospray) : m/z [M+Na]⁺ 253/255.

### Preparation 55 : [4-(2-Oxo-propyl)-phenyl]-acetic acid methyl ester

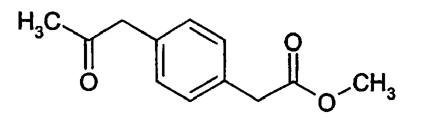

Prepared using the method for preparation 53 using the ester from preparation 56 to give the title compound as a pale yellow oil.
¹H NMR (400MHz, CDCl₃): δ = 7.25 (2H, d), 7.16 (2H, d), 3.68 (3H, s), 3.60 (2H, s), 2.15 (3H, s) ppm.
LRMS (electrospray) : m/z [M+NH₄]⁺ 224, [M+Na]⁺ 229.

### Preparation 56 : (4-Bromo-phenyl)-acetic acid methyl ester

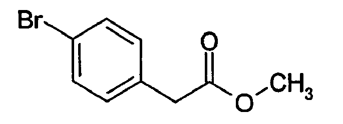

Prepared using the method for preparation 54 using (4-bromo-phenyl)-acetic acid to give the title compound as a colourless oil.
¹H NMR (400MHz, CDCl₃): δ = 7.44 (2H, d), 7.15 (2H, d), 3.69 (3H, s), 3.58 (2H, s) ppm.
LRMS (electrospray) : m/z [M+Na]⁺ 253 / 255.

### Preparation 57 : N-(4-Aminomethyl-benzyl)-2,2-diphenyl-acetamide

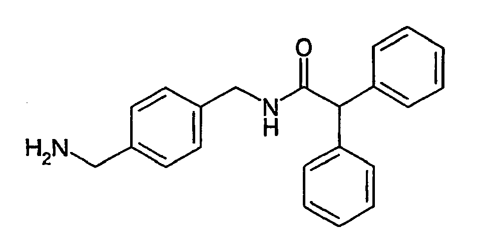

A solution of the amide from preparation 58 (200 mg, 0.43 mmol) in a mixture of ethanol (15 ml) and ethyl acetate (1 ml) was hydrogenated at 60psi / 50°C over 10% palladium-on-charcoal (40 mg) for 3h. The resulting mixture was filtered through arbocel and the filtrate concentrated *in vacuo* to give an oil. Purification by flash column chromatography on silica gel eluting with CH₂Cl₂:MeOH (98:2 to 90:10, by volume) gave the title compound (87 mg) as a white solid.
¹H NMR (400MHz, DMSO d₆): δ = 8.72-8.68 (1H, m), 7.31-7.11(14H, m), 4.98 (1H, s), 4.26 (2H, d), 3.69 (2H, bs) ppm.
LRMS (electrospray) : m/z [M+H]⁺ 331.

### Preparation 58 : [4-(Diphenylacetylamino-methyl)-benzyl]-carbamic acid benzyl ester

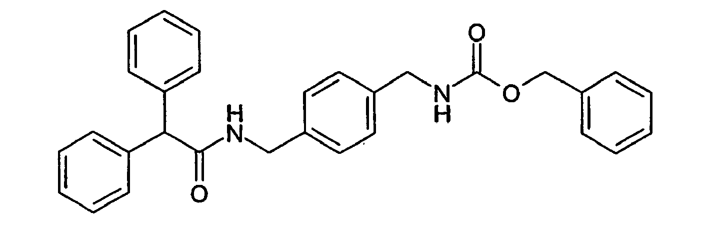

Prepared according to the method for preparation 1 using the amine from preparation 59 and the appropriate acid to give the title compound as a colourless solid.
¹H NMR (400MHz, DMSO d₆): δ = 8.75-8.68 (1H, m), 7.75-7.72 (1H, m), 7.33-7.08 (19H, m), 5.01 (2H, s), 4.98 (1H, s), 4.26 (2H, d), 4.14 (2H, d) ppm.
LRMS (electrospray) : m/z [M-H]⁻ 634.

### Preparation 59 : (4-Aminomethyl-benzyl)-carbamic acid benzyl ester

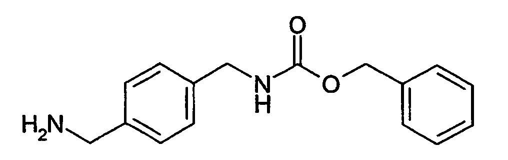

A solution of 1,4-bis(aminomethyl)benzene (10.0g, 74mmol) and triethylamine (9.8 ml, 74 mmol) in dichloromethane (480 ml) was cooled to 0°C and treated with a solution of benzylchloroformate (10.5ml, 74mmol) in dichloromethane (250 ml). The resulting mixture was allowed to warm gradually to room temperature of a period of 16h. The solvent was removed *in vacuo* and the residue partitioned between ethyl acetate (450 ml) and 1M aqueous sodium hydroxide (300 ml). The resulting biphasic mixture was filtered and the organic phase separated, dried (magnesium sulfate) and reduced *in vacuo* to give a waxy solid. Trituration with hot ethyl acetate followed by concentration of the liquors gave the title compound as a waxy solid (7.4g).
¹H NMR (400MHz, CDCl₃): δ = 7.83-7.72 (1H, m), 7.36-7.15 (9H, m), 5.02 (2H, s), 4.16 (2H, d), 3.66 (2H, s) ppm.
LRMS (electrospray): m/z [M+H]⁺ 271.

### Preparation 60 : (4-Aminomethyl-benzyl)-benzhydryl-amine hydrochloride

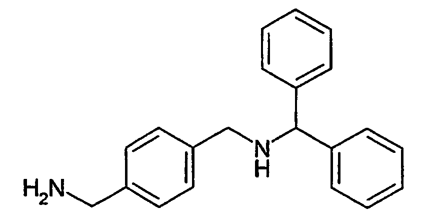

A solution of the nitrile from preparation 61 (10.43 g, 35 mmol) in tetrahydrofuran (100 ml) was added dropwise to a suspension of lithium aluminium-hydride (2.66 g, 70 mmol) in tetrahydrofuran (80 ml) under a nitrogen atmosphere. The resulting suspension was heated to reflux for 5 hours and then cooled to room temperature. Water (2.66 ml) was added followed by 15%w/w aqueous sodium hydroxide (2.66ml), followed by water (7.98 ml). The resulting suspension was filtered and the filtrate reduced *in vacuo* to give a colourless oil. The oil was redissolved in chloroform (200 ml), dried (sodium sulfate) and reduced *in vacuo* to give a yellow oil. The oil was treated with a solution of hydrogen chloride in ether to give the hydrochloride salt which was crystallised (isopropanol/methanol) to give the title compound as a colourless solid.
¹H NMR (60MHz, DMSO_{d6}): δ = 11.00 (1H, m), 9.10 (2H, m), 7.78-7.15 (14H, m), 5.50-5.30 (1H, m), 4.10-3.90 (4H, m) ppm.

### Preparation 61 : 4-[(Benzhydryl-amino)-methyl]-benzonitrile hydrogen chloride

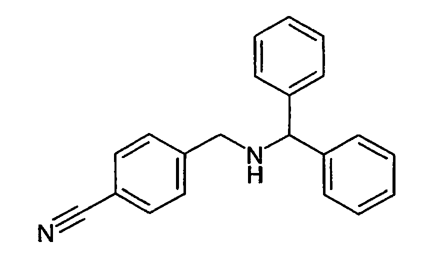

A suspension of 4-cyanobenzylbromide (13.72 g, 70mmol), benzhydrylamine (13.79 g, 75 mmol) and potassium carbonate (16.8 g, 140 mmol) in ethanol (200 ml) was heated to reflux for 5 hours. The reaction mixture was cooled to room temperature and filtered. The filtrate was reduced *in vacuo* and the residue crystallised from a mixture of hexane / isopropanol to give the free base as a colourless solid. The compound was treated with hydrogen chloride in ether and then recrystallised (isopropanol) to give the title compound as a colourless solid.
¹H NMR (60MHz, DMSO_{d6}): δ = 11.30-10.50 (2H, m), 7.82-7.15 (14H, m), 5.10-4.90 (1H, m), 4.20-3.90 (2H, m) ppm.

### Preparation 62 : 2-Benzyloxy-ethylamine

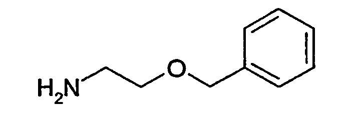

Prepared according to Synthetic Communications 1995, 25(6), 907-913.
¹H NMR (400MHz, CDCl₃): δ = 7.37-7.29 (5H, m), 4.54 (2H, s), 3.52 (2H, t), 2.89 (2H, t), 1.52 (2H, s) ppm.
LRMS (APCI) : m/z [M+H]⁺ 152.

### Preparation 63 : 2-(3-Phenyl-propoxy)-ethylamine

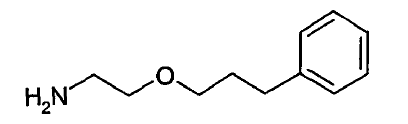

Prepared according to Synthetic Communications 1995, 25(6), 907-913.
¹H NMR (400MHz, CDCl₃): δ = 7.30-7.26 (2H, m), 7.20-7.16 (3H, m), 3.47-3.43 (4H, m), 2.86 (2H, t), 2.68 (2H, t), 1.95-1.88 (2H, m), 1.51 (2H, s) ppm.
LRMS (APCI) : m/z [M+H]⁺ 180.

### Preparation 64 : 2-(Naphthalen-1-ylmethoxy)-ethylamine

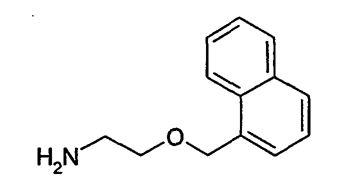

Sodium hydride (1.00 g, 0.025 mmol) was added portionwise to a cooled (0°C) solution of ethanolamine (1.51 ml, 0.025 mmol) in tetrahydrofuran (25 ml). The reaction mixture was then heated to reflux for 30 minutes. Chloromethylnaphthalene (3.4 ml, 0.0225 mmol) was added and the reaction mixture heated at reflux for a further 3 hours. The reaction mixture was cooled to room temperature and the solvent removed *in vacuo.* The residue was partitioned between dichloromethane (50 ml) and 1N aqueous sodium hydroxide (50 ml). The organic phase was separated and the aqueous phase extracted with dichloromethane (2 x 50 ml). The combined organic extracts were reduced *in vacuo* and the residue purified by flash column chromatography eluting with dichloromethane:methanol: 880 ammonia (98:2:0.2 changing to 90:10:1 by volume) to give the title compound as a colourless oil (1.80 g, 40%).
¹H NMR (400MHz, CDCl₃): δ = 8.13 (1H, d), 7.87 (1H, d), 7.82 (1H, d), 7.54-7.42 (4H, m), 4.98 (2H, s), 3.59 (2H, t), 2.89 (2H, t), 1.65 (2H, s) ppm.
LRMS (electrospray) : m/z [M+H]⁺ 202, [M+Na]⁺ 214.

### Preparation 65 : 4-Aminomethyl-N-methyl-benzenesulfonamide

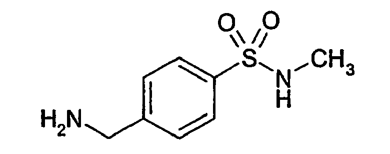

A solution of the nitrile from preparation 74 (1.27 g, 6.47 mmol) in methanol (60 ml) at 0°C was treated with cobalt chloride (1.68 g, 12.9 mmol) and the resulting mixture left to stir for 5 minutes prior to the addition of sodium borohydride (2.40 g, 63.4 mmol). The resulting suspension was allowed to warm gradually to room temperature. After 16 hours 3N hydrochloric acid (10ml) was added and the mixture stirred for 10 minutes. 880 Ammonia solution (20ml). Silica gel was added to the mixture and the solvent removed *in vacuo* to give a free flowing powder which was purified by flash column chromatography eluting with dichloromethane:methanol: 880 ammonia (97:3:0.5 changing to 90:10:1 by volume) to give the title compound as a colourless waxy solid (1.12 g, 86%).
¹H NMR (400MHz, CD₃OD): δ = 7.80 (2H, d), 7.55 (2H, d), 3.85 (2H, s), 2.42 (3H, s) ppm.
LRMS (APCI) : m/z [M+H]⁺ 201.

### Preparation 66 : 4-Aminomethyl-N-ethyl-benzenesulfonamide

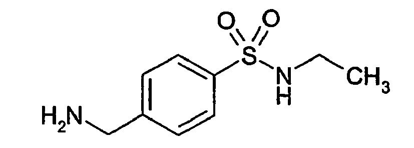

Prepared according to the method for preparation 65 using the nitrile from preparation 75 to give the title compound as a colourless oil.
¹H NMR (400MHz, CD₃OD): δ = 7.79 (2H, d), 7.52 (2H, d), 3.87 (2H, s), 2.86 (2H, q), 1.04 (3H, t) ppm.
LRMS (APCI) : m/z [M+H]⁺ 215.

### Preparation 67: 4-Aminomethyl-N-benzyl-benzenesulfonamide hydrochloride

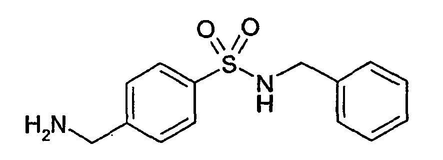

Prepared according to the method for preparation 65 using the nitrile from preparation 76 to give the title compound as a colourless oil.
¹H NMR (400MHz, CD₃OD): δ = 7.78 (2H, d), 7.48 (2H, d), 7.22-7.18 (5H, m), 4.02 (2H, s), 3.82 (2H, s) ppm.
LRMS (APCI) : m/z [M+H]⁺ 277.

### Preparation 68 : 3-Aminomethyl-N-ethyl-benzenesulfonamide

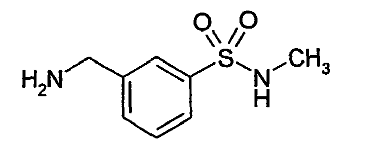

Prepared according to the method for preparation 65 using the nitrile from preparation 71 to give the title compound as a colourless oil.
¹H NMR (400MHz, CD₃OD): δ = 7.83 (1H, s), 7.75 (1H, d), 7.60 (1H, d), 7.55 (1H, m), 3.85 (2H, s), 2.50 (3H, s) ppm.
LRMS (APCI) : m/z [M+H]⁺ 215.

### Preparation 69 : 3-Aminomethyl-N-ethyl-benzenesulfonamide

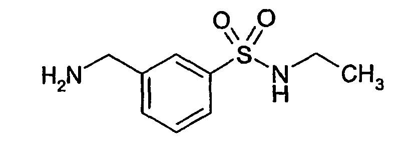

Prepared according to the method for preparation 65 using the nitrile from preparation 72 to give the title compound as a colourless oil.
¹H NMR (400MHz, CD₃OD): δ = 7.83 (1H, s), 7.72 (1H, d), 7.58 (1H, d), 7.52 (1H, m), 3.88 (2H, s), 2.89 (2H, q), 1.05 (3H, t) ppm.
LRMS (APCI) : m/z [M+H]⁺ 215.

### Preparation 70 : 3-Aminomethyl-N-benzyl-benzenesulfonamide

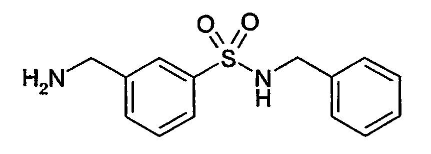

Prepared according to the method for preparation 65 using the nitrile from preparation 73 to give the title compound as colourless oil.
¹H NMR (400MHz, CD₃OD): δ = 7.77 (1H, s), 7.70 (1H, d), 7.54 (1H, d), 7.48 (1H, t), 7.23-7.18 (5H, m), 4.07 (2H, s), 3.82 (2H, s) ppm.
LRMS (APCI) : m/z [M+H]⁺ 277.

### Preparation 71 : 3-Cyano-N-ethyl-benzenesulfonamide

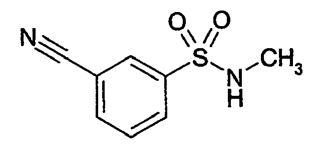

Methylamine hydrochloride (736 mg, 10.9 mmol) was added to a solution of 3-cyano benzene sulfonyl chloride (2.0g, 9.9 mmol) and triethylamine (3.45 ml, 24.7mmol) in tetrahydrofuran (30 ml) and the resulting mixture left to stir at room temperature under a nitrogen atmosphere for 16h hours. The reaction mixture was diluted with water (30 ml) and ethyl acetate (50 ml). The organic phase was separated and dried (sodium sulfate) and reduced *in vacuo* to give the title compound as a colourless solid (1.68g, 86%).
¹H NMR (400MHz, CD₃OD): δ = 8.17 (1H, s), 8.12 (1H, d), 7.98 (1H, d), 7.77 (1H, t), 2.56 (3H, s) ppm.
LRMS (APCl): m/z [M-H]⁻ 195.

### Preparation 72 : 3-Cyano-N-ethyl-benzenesulfonamide

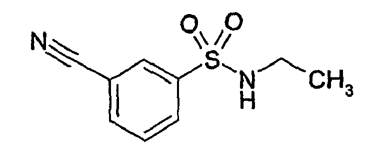

Prepared using the method for preparation 71 using the appropriate amine to give the title compound as a pale yellow solid.
¹H NMR (400MHz, CD₃OD): δ = 8.17 (1H, s), 8.11 (1H, d), 7.98 (1H, d), 7.75 (1H, t), 2.92 (2H, q), 1.06 (3H, t) ppm.
LRMS (APCI) : m/z [M+Na]⁺ 228, [M-H]⁻ 209.

### Preparation 73 : N-Benzyl-3-cyano-benzenesulfonamide

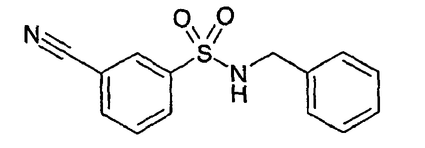

Prepared using the method for preparation 71 using the appropriate amine to give the title compound as a pale orange solid.
¹H NMR (400MHz, CD₃OD): δ = 8.02 (2H, m), 7.88 (1H, d), 7.62 (1H, t), 7.18 (5H, m), 4.17 (2H, s) ppm.
LRMS (APCI) : m/z [M+H]⁺ 273, [M+NH₄]⁺ 290, [M-H]⁻ 271.

### Preparation 74 : 4-Cyano-N-methyl-benzenesulfonamide

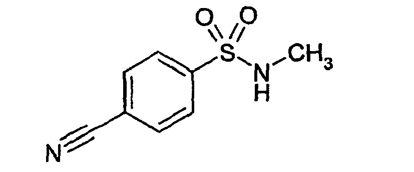

Prepared using the method for preparation 71 using the appropriate amine and sulfonyl chloride to give the title compound as a pale orange solid.
¹H NMR (400MHz, CD₃OD): δ = 7.98-7.95 (4H, m), 2.56 (3H, s) ppm.
LRMS (APCI) : m/z [M+NH₄]⁺ 214, [M-H]⁻ 195.

### Preparation 75 : 4-Cyano-N-ethyl-benzenesulfonamide

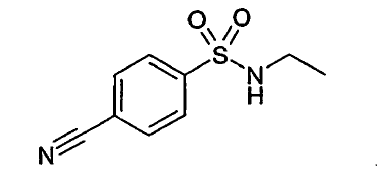

Prepared using the method for preparation 71 using the appropriate amine and sulfonyl chloride to give the title compound as a yellow solid.
¹H NMR (400MHz, CD₃OD): δ = 7.99 (2H, d), 7.93 (2H, d), 2.93 (2H, q), 1.06 (3H, t) ppm.
LRMS (APCI) : m/z [M+NH₄]⁺ 228, [M-H]⁻ 209.

### Preparation 76: N-Benzyl-4-cyano-benzenesulfonamide

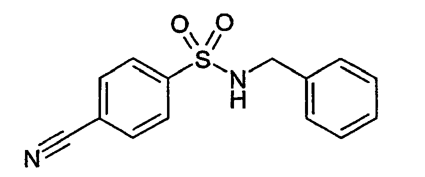

Prepared using the method for preparation 71 using the appropriate amine and sulfonyl chloride to give the title compound as a pale yellow solid.
¹H NMR (400MHz, DMSO_{d6}): δ = 8.35 (1H, bs), 8.03 (2H, d), 7.91 (2H, d), 7.25-7.18 (5H, m), 4.02 (2H, s) ppm.
LRMS (APCl) : m/z [M-H]⁻ 271.

### Preparation 77 : 2-[3-((2R)-2-{(2R)-2-[6-(2,5-Dimethyl-pyrrol-1-yl)-pyridin-3-yl]-2-hydroxy-ethylamino}-propyl)-phenyl]-N-{4-[3-(3-phenoxy-benzyl)-ureidomethyl]-benzyl}-acetamide

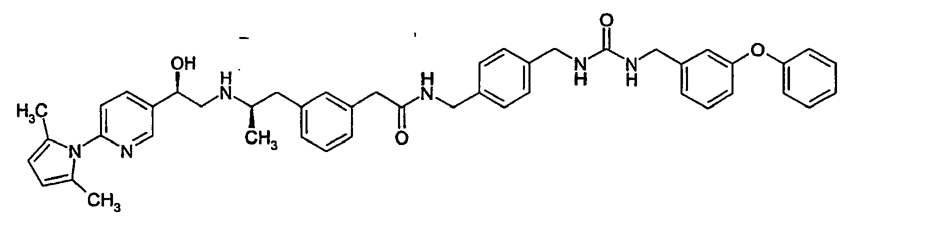

A solution of the acid from preparation 40 (179mg, 0.43mmol), 1-hydroxybenzotriazole hydrate (89 mg, 0.65 mmol), 1-(3-dimethylaminopropyl)-3-ethylcarbodiimide hydrochloride (113 mg, 0.57 mmol), triethylamine (0.18ml, 1.32mmol) and the amine from preparation 82 (210mg, 0.53mmol) in *N,N-*dimethylformamide (2 ml) was left to stir under a nitrogen atmosphere for 24 hours. The reaction mixture was diluted with ethyl acetate (10ml) and water (3ml). The organic phase was separated, dried (sodium sulfate) and the solvent removed *in vacuo.* The residue was purified by flash column chromatography eluting with dichloromethane:methanol:880 ammonia (100:0:0 changing to 94:6:1 by volume) to give the title compound as a pale yellow foam (184mg, 56%).
¹H NMR (400MHz, CD₃OD): δ = 8.50 (1H, s), 7.93 (1H, d), 7.33-7.12 (11H, m), 7.09 (2H, d), 7.02 (1H, d), 6.96 (1H, s), 6.93 (2H, d), 6.84 (1H, d), 5.81 (2H, s), 4.31-4.27 (7H, m), 3.52 (2H, s), 2.98 (1H, m), 2.84 (2H, m), 2.75 (1H, m), 2.59 (1H, m), 2.02 (6H, s), 1.06 (3H, d) ppm.
LRMS (APCI) : m/z [M+H]⁺ 751.

### Preparation 78 : N-{4-[3-(3,4-Dimethoxy-benzyl)-ureidomethyl]-benzyl}-2-[3-((2R)-2-{(2R)-2-[6-(2,5-dimethyl-pyrrol-1-yl)-pyridin-3-yl]-2-hydroxy-ethylamino}-propyl)-phenyl]-acetamide

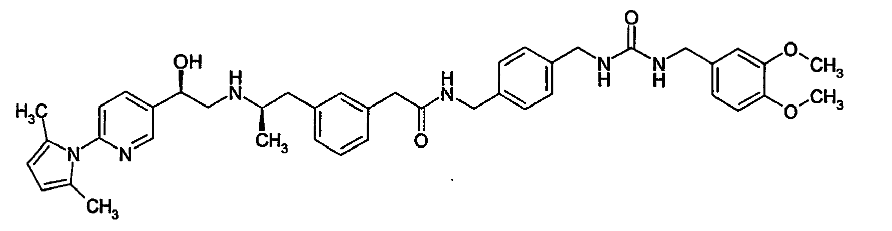

Prepared using the method for preparation 77 using the acid from preparation 40 and the amine from preparation 80 to give the title compound as a pale yellow foam.
¹H NMR (400MHz, CD₃OD): δ = 8.50 (1H, s), 7.94 (1H, d), 7.29-7.10 (9H, m), 6.88 (2H, d), 6.82 (1H, d), 5.81 (2H, s), 4.32-4.25 (7H, m), 3.79 (3H, s), 3.77 (3H, s), 3.52 (2H, s), 2.99 (1H, m), 2.85 (2H, m), 2.75 (1H, m), 2.60 (1H, m), 2.02 (6H, s), 1.06 (3H, d) ppm.
LRMS (APCI) : m/z [M+H]⁺ 719.

### Preparation 79 : 2-[3-(2-{2-[6-(2,5-Dimethyl-pyrrol-1-yl)-pyridin-3-yl]-2-hydroxy-ethylamino}-propyl)-phenyl]-N-{4-[3-(4-phenoxy-benzyl)-ureidomethyl]-benzyl}-acetamide

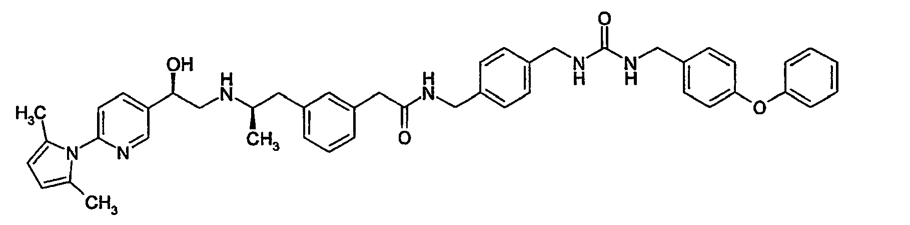

Prepared using the method for preparation 77 using the acid from preparation 40 and the amine from preparation 81 to give the title compound as a pale yellow foam.
¹H NMR (400MHz, CD₃OD): δ = 8.50 (1H, s), 7.93 (1H, d), 7.34-7.08 (15H, m), 6.95-6.90 (3H, m), 5.81 (2H, s), 4.32-4.29 (7H, m), 3.52 (2H, s), 2.98 (1H, m), 2.86 (2H, m), 2.75 (1H, m), 2.60 (1H, m), 2.02 (6H, s), 1.06 (3H, d) ppm.
LRMS (APCI) : m/z [M+H]⁺ 751.

### Preparation 80 : 1-(4-Aminomethyl-benzyl)-3-(3,4-dimethoxy-benzyl)-urea hydrochloride

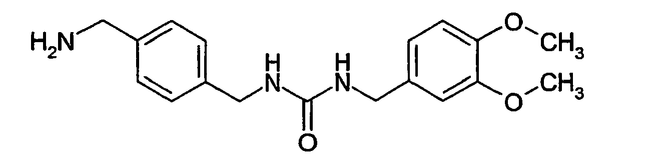

A solution of the urea from preparation 83 (273 mg, 0.64 mmol) in dichloromethane (10 ml) was treated with 1M hydrogen chloride in methanol (10ml) and the resulting solution stirred under a nitrogen atmosphere at room temperature for 16 hours. The solvent was removed *in vacuo* to yield the title compound as a colourless solid (309 mg, 100%).
¹H NMR (400MHz, CD₃OD): δ = 7.42-7.35 (4H, m), 6.89 (2H, d), 6.82 (1H, d), 4.37 (2H, s), 4.26 (2h, s), 4.08 (2H, s), 3.82 (3H, s), 3.81 (3H, s) ppm.
LRMS (APCI) : m/z [M+H]⁺ 330.

### Preparation 81 : 1-(4 Aminomethyl-benzyl)-3-(4-phenoxy-benzyl)-urea hydrochloride

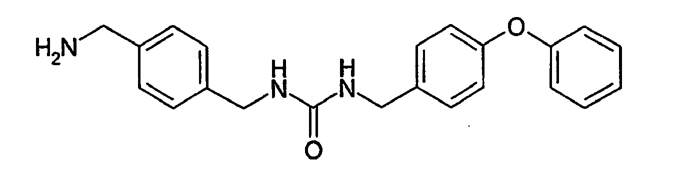

Prepared according to the method for preparation 80 using the urea from preparation 84 to give the title compound as a colourless solid.
¹H NMR (400MHz, CD₃OD): δ = 7.43-7.25 (8H, m), 7.09 (1H, t), 6.95 (4H, m), 4.38 (2H, s), 4.34 (2H, s), 4.09 (2H, s) ppm.
LRMS (APCI) : m/z [M+H]⁺ 362.

### Preparation 82 : 1-(4-Aminomethyl-benzyl)-3-(3-phenoxy-benzyl)-urea hydrochloride

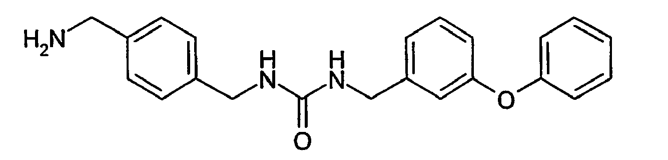

Prepared according to the method for preparation 80 using the urea from preparation 85 to give the title compound as a colourless solid.
¹H NMR (400MHz, CD₃OD): δ = 7.95 (2H, d), 7.90 (1H, s), 7.82 (1H, d), 7.42-7.23 (7H, m), 7.08 (1H, t), 7.02 (1H, d), 4.35 (2H, s), 4.30 (2H, s), 4.06 (2H, s) ppm.
LRMS (APCl) : m/z [M+H]⁺ 362.

### Preparation 83 : {4-[3-(3,4-Dimethoxy-benzyl)-ureidomethyl]-benzyl}-carbamic acid tert-butyl ester

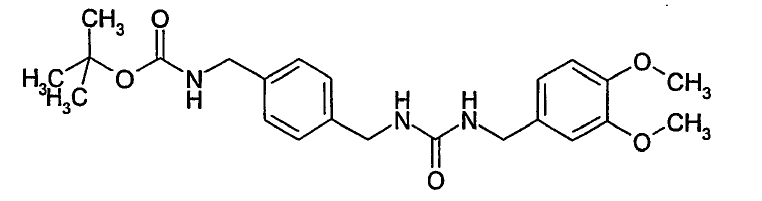

A solution of the imidazole from preparation 86 (300 mg, 0.9mmol) and 3,4-dimethoxybenzylamine (0.142ml, 0.95mmol) in toluene (5 ml) was heated to 60°C under a nitrogen atmosphere for 2 hours. The solvent was removed *in vacuo* and the residue purified by strong cation exchange resin eluting with methanol to give the title compound as a colourless solid (273mg, 70%).
¹H NMR (400MHz, DMSO): δ = 7.34 (1H, t), 7.16-7.13 (4H, m), 6.84 (2H, m), 6.75 (1H, d), 6.34 (1H, t), 6.30 (1H, t), 4.18 (2H, d), 4.12 (2H, d), 4.07 (2H, d), 3.70 (3H, s), 3.69 (3H, s), 1.37 (9H, s) ppm.
LRMS (APCI) : m/z [M+H]⁺ 430, [M+NH₄]⁺ 447.

### Preparation 84 : {4-[3-(4-Phenoxy-benzyl)-ureidomethyl]-benzyl}-carbamic acid tert-butyl ester

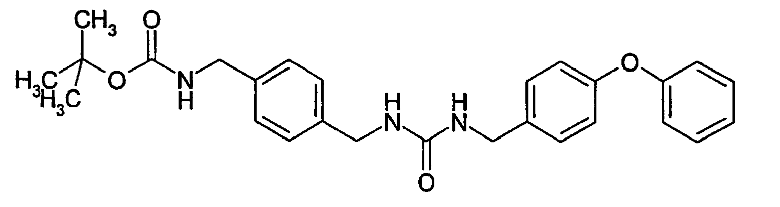

Prepared according to the method for preparation 83 using the appropriate amine to give the title compound as a colourless solid.
¹H NMR (400MHz, DMSO): δ = 7.35 (3H, m), 7.25 (2H, d), 7.17-7.09 (5H, m), 6.93 (4H, d), 6.69 (2H, m), 4.18 (4H, m), 4.07 (2H, d), 1.37 (9H, s) ppm.
LRMS (APCl) : m/z [M+H]⁺ 462, [M+NH₄]⁺ 479.

### Preparation 85 : {4-[3-(3-Phenoxy-benzyl)-ureidomethyl]-benzyl}-carbamic acid tert-butyl ester

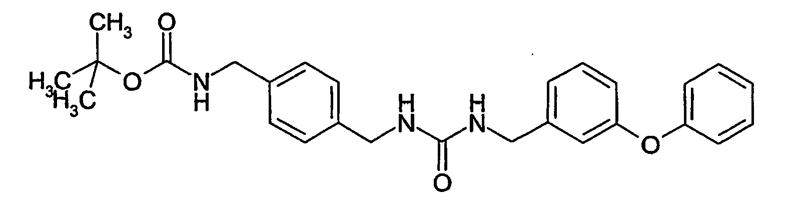

Prepared according to the method for preparation 83 using the appropriate amine to give the title compound as a colourless solid which was used without further purification.

### Preparation 86: (4-{[(Imidazole-1-carbonyl)-amino]-methyl}-benzyl)-carbamic acid tert-butyl ester

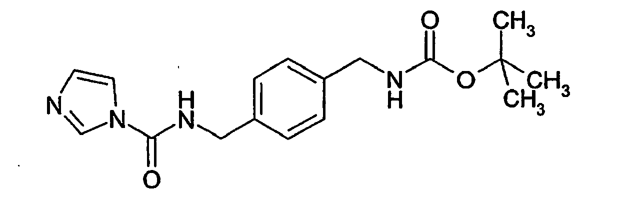

A suspension of the amine from preparation 87 (2.0 g, 8.46 mmol) in tetrahydrofuran (30 ml) was added to a solution of carbonyl diimidazole (1.51 g, 9.31 mmol) in tetrahydrofuran (30 ml) and the resulting mixture left to stir at room temperature under a nitrogen atmosphere for 16 hours. The reaction mixture was diluted with dichloromethane (50ml) and washed with water (30 ml), saturated aqueous sodium chloride (30 ml). The organic phase was dried (sodium sulfate) and reduced *in vacuo* to give a white solid. Purification by flash column chromatography eluting with dichloromethane:methanol: 880 ammonia (100:0:0 changing to 94:6:1 by volume) gave the title compound as a colourless oil (1.38 g, 49%).
¹H NMR (400MHz, CD₃OD): δ = 8.42 (1H, s), 7.62 (1H, s), 7.32 (2H, d), 7.25 (2H, d), 7.05 (1H, s), 4.50 (2H, s), 4.20 (2H, s), 1.42 (9H, s) ppm.
LRMS (APCI) : m/z [M+H]⁺ 331.

### Preparation 87 : (4-Aminomethyl-benzyl)-carbamic acid tert-butyl ester

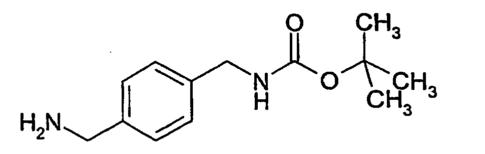

A solution of di-*tert*-butyl dicarbonate (19.0g, 87 mmol) in dichloromethane (50 ml) was added dropwise to a cooled (0°C) suspension of *para*-xylylenediamine (44.0 g, 323 mmol) in dichloromethane (200ml) over a period of 30 minutes. The resulting suspension was then allowed to warm to room temperature over a period of 2 hours. The reaction mixture was washed with 1N aqueous sodium hydroxide (300 ml) and the organic phase separated, dried (sodium sulfate) and reduced *in vacuo* to give a colourless solid. Purification by flash column chromatography eluting with dichloromethane:methanol:880 ammonia (97:3:0 changing to 94:6:1 by volume) gave the title compound as a colourless solid (9.61 g, 47%).
¹H NMR (400MHz, CD₃OD): δ = 7.30-7.20 (4H, m), 4.21 (2H, s), 3.75 (2H, s), 1.43 (9H, s) ppm.
LRMS (APCI) : m/z [M+H]⁺ 237.

### In vitro activity of the compounds of formula (1)

The ability of the compounds of the formula (1) to act as potent β2 agonists therefore mediating smooth muscle relaxation may be determined by the measure of the effect of beta-2 adrenergic receptor stimulation on electrical field stimulated-contraction of guinea pig trachea strips.

### Guinea-pig trachea

Male, Dunkin-Hartley guinea pigs (475-525g) are killed by CO₂ asphyxiation and exsanguination from the femoral artery and the trachea is isolated. Four preparations are obtained from each animal, starting the dissection immediately below the larynx and taking 2.5 cm length of trachea. The piece of trachea is opened by cutting the cartilage opposite the trachealis muscle, then transverse sections, 3-4 cartilage rings wide, are cut. The resulting strip preparations are suspended in 5 ml organ baths using cotton threads tied through the upper and lower cartilage bands. The strips are equilibrated, un-tensioned, for 20 minutes in a modified Krebs Ringer buffer (Sigma K0507) containing 3 µM Indomethacin (Sigma 17378), 10 µM Guanethidine (Sigma G8520) and 10 µM Atenolol (Sigma A7655), heated at 37°C and gassed with 95% O₂/5% CO₂, before applying an initial tension of 1 g. The preparations are allowed to equilibrate for a further 30-45 minutes, during which time they are re-tensioned (to 1 g) twice at 15-minute intervals. Changes in tension are recorded and monitored via standard isometric transducers coupled to a data-collection system (custom-designed at Pfizer). Following the tensioning equilibration, the tissues are subjected to electrical field stimulation (EFS) using the following parameters : 10 s trains every 2 minutes, 0.1 ms pulse width, 10 Hz and just-maximal voltage (25 Volts) continuously throughout the length of the experiment. EFS of post-ganglionic cholinergic nerves in the trachea results in monophasic contractions of the smooth muscle and twitch height is recorded. The organ baths are constantly perfused with the above-described Krebs Ringer buffer by means of a peristaltic pump system (pump flow rate 7.5 ml / minute) throughout the experiment, with the exception of when a beta-2 agonist according to the present invention is added, the pump is then stopped for the time of the cumulative dosing to the bath and started again after maximal response is reached for the wash-out period.

### Experimental protocol for assessment of potency and efficacy

Following equilibration to EFS, the peristaltic pump is stopped and the preparations 'primed' with a single dose of 300 nM isoprenaline (Sigma 15627) to establish a maximal response in terms of inhibition of the contractile EFS response. The isoprenaline is then washed out over a period of 40 minutes. Following the priming and wash-out recovery, a standard curve to isoprenaline is carried out on all tissues (Isoprenaline Curve 1) by means of cumulative, bolus addition to the bath using half log increments in concentration. The concentration range used is 1^{e-9} to 1^{e}/3^{e-6} M. At the end of the isoprenaline curve the preparations are washed again for 40 minutes before commencing a second curve, either to isoprenaline (as internal control) or a beta-2 agonist according to the present invention. Beta-2 agonist responses are expressed as percentage inhibition of the EFS response. Data for beta-2 agonist are normalised by expressing inhibition as a percentage of the maximal inhibition induced by isoprenaline in Curve 1. The EC₅₀ value for beta-2 agonist according to the present invention refers to the concentration of compound required to produce half maximal effect. Data for beta-2 agonists according to the present invention are then expressed as relative potency to isoprenaline defined by the ratio (EC₅₀ beta-2 agonist)/(EC50 Isoprenaline).

### Confirmation of beta-2 mediated functional activity

Beta-2 agonist activity of test compounds is confirmed using the protocol above, however, prior to constructing the curve to beta-2 agonist according to the present invention, the preparations are pre-incubated (for a minimum of 45 minutes) with 300 nM ICI 118551 (a selective β₂ antagonist) which results in the case of a beta-2 mediated effect in a rightward-shift of the test compound dose response curve.

It has thus been found that the compounds of formula (1) according to the present invention that have been tested show a relative potency to Isoprenaline which is comprised between 0.008 and 2.0.

According to another alternative, the agonist potency for the β2 receptor of the compounds of the formula (1) may also be determined by the measure of the concentration of compound according to the present invention required to produce half maximal effect (EC₅₀) for the β2 receptor.

### Compound Preparation

10 mM/100% DMSO (dimethylsulfoxide) stock of compound is diluted to required top dose in 4 % DMSO. This top dose is used to construct a 10-point semi-log dilution curve, all in 4 % DMSO. Isoprenaline (Sigma, 1-5627) was used as a standard in every experiment and for control wells on each plate. Data was expressed as % Isoprenaline response.

### Cell Culture

CHO (Chinese Hamster Ovary) cells recombinantly expressing the human β2 adrenergic receptor (from Kobilka et al., PNAS 84: 46-50, 1987 and Bouvier et al., Mol Pharmacol 33: 133-139 1988 CHOhβ2) were grown in Dulbeccos MEM/NUT MIX F12 (Gibco, 21331-020) supplemented with 10 % foetal bovine serum (Sigma, F4135, Lot 90K8404 Exp 09/04), 2 mM glutamine (Sigma, G7513), 500 µg/ml geneticin (Sigma, G7034) and 10 µg/ml puromycin (Sigma, P8833). Cells were seeded to give about 90 % confluency for testing.

### Assay Method

25 µl /well each dose of compound was transferred into a cAMP- Flashplate® (NEN, SMP004B), with 1% DMSO as basal controls and 100 nM Isoprenaline as max controls. This was diluted 1:2 by the addition of 25 µl /well PBS. Cells were trypsinised (0.25% Sigma, T4049), washed with PBS (Gibco, 14040-174) and resuspended in stimulation buffer (NEN, SMP004B) to give 1x10⁶ cells / ml CHOhB2. Compounds were incubated with 50 µl / well cells for 1 hour. Cells were then lysed by the addition of 100 µl / well detection buffer (NEN, SMP004B) containing 0.18 µCi / ml¹²⁵l-cAMP (NEN, NEX-130) and plates were incubated at room temperature for a further 2 hours. The amount of ¹²⁵l-cAMP bound to the Flashplate® was quantified using a Topcount NXT (Packard), normal counting efficiency for 1 minute. Dose-response data was expressed as % Isoprenaline activity and fitted using a four parameter sigmoid fit.

It has thus been found that the compounds of formula (1) according to the present invention that are illustrated in examples 1 to 36 above show a β2 cAMP EC₅₀ between 0.01 nM and 2 nM.

## Claims

1. A compound of the formula (1): 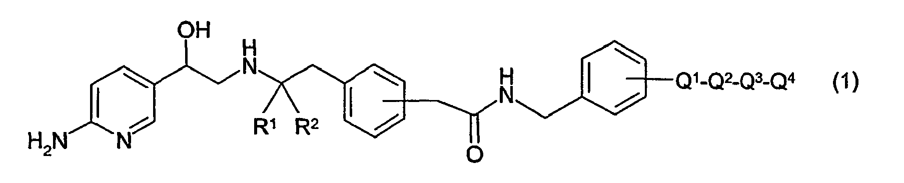 wherein the CH₂-C(=O)NH-benzyl-Q¹-Q²-Q³-Q⁴ group is in the meta or para position, and
R¹ and R² are independently selected from H and C₁-C₄ alkyl;
Q¹ is (CH₂)ₙ wherein n is an integer selected from 0 and 1;
Q² is a group selected from NH, -C(=O)NH-, -NHC(=O)-, -NH-C(O)-NH- and-SO₂NH-;
Q³ is a single bond, a C₁-C₄ alkylene optionally substituted with OH or a group (CH₂)ₘ-O-(CH₂)ₚ wherein m and p are integers independently selected from 1, 2 or 3;
Q⁴ is selected from 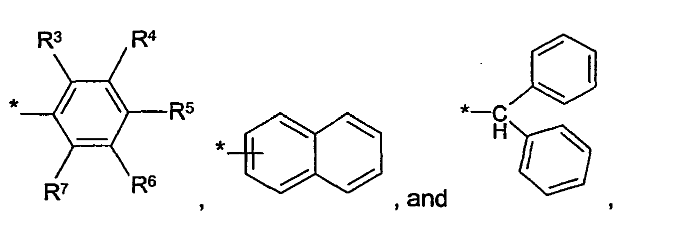 wherein * represents the attachment point to Q³ and R³, R⁴, R⁵, R⁶ and R⁷ are independently selected from H, phenoxy, C₁-C₄ alkyl, OR⁹, SR⁹, halo, CF₃, OCF₃, COOR⁹, SO₂NR⁸R⁹, CONR⁸R⁹, NHR⁸R⁹, NHCOR⁹, CH₂-NHC(=O)NH-R⁹ provided at least 2 of R³ to R⁷ are equal to H;
wherein R⁸ is selected from H or C₁-C₄ alkyl and R⁹ is selected from H, C₁-C₄ alkyl, benzyl optionally substituted with 1, 2, 3 or 4 C₁-C₄ alkoxy, and a group 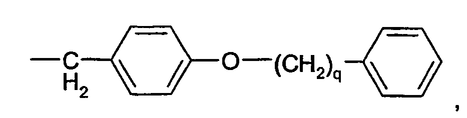 wherein q is an integer selected from 0, 1, 2 and 3;
or, if appropriate, their pharmaceutically acceptable salts and/or isomers, tautomers, solvates or isotopic variations thereof.

2. A compound according to claim 1 wherein R¹ is H and R² is CH₃.

3. A compound according to claim 1 wherein R¹ is CH₃ and R² is CH₃.

4. A compound according to any one of claims 1 to 3, wherein n is 0 and Q² is-C(=O)NH- or SO₂NH.

5. A compound according to any one of claims 1 to 3, wherein n is 1 and Q² is-NH-C(=O)- or -NH-C(O)-NH-.

6. A compound according to any one of claims 1 to 5, wherein Q³ is selected from single bond, CH₂, CH(-CH₃)CH₂OH-, CH(CH₃)-, -CH(CH₂OH)CH₂-,-(CH₂)₂-, -(CH₂)₂-OCH₂- and -(CH₂)₂-O-(CH₂)₃-.

7. A compound according to any one of claims 1 to 6, wherein Q⁴ is selected from 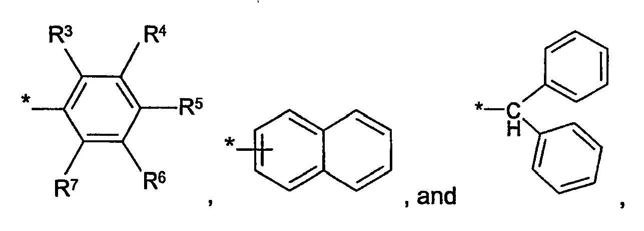 wherein * represents the attachment point to Q³ and R³, R⁴, R⁵, R⁶ and R⁷ are independently selected from H,
- OR⁹, provided at least 2 of R³ to R⁷ are equal to H, or
- SO₂NR⁸R⁹ or CH₂-NHC(=O)NH-R⁹ provided at least 4 of R³ to R⁷ are equal to H,
wherein R⁸ is selected from H or C₁-C₄ alkyl and k⁹ is selected from H, C₁-C₄ alkyl, benzyl optionally substituted with 1, 2, 3 or 4 C₁-C₄ alkoxy, and a group 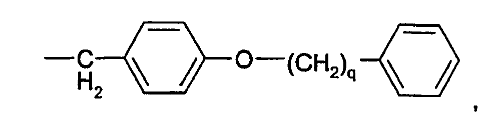 wherein q is an integer selected from 0, 1, 2 and 3.

8. A compound according to any one of claims 1 to 6, wherein Q⁴ is selected from 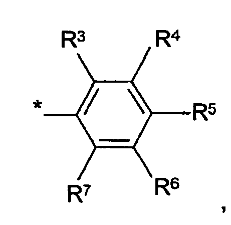 wherein * represents the attachment point to Q³ and R³, R⁴, R⁵, R⁶ and R⁷ are independently selected from H,
- OR⁹, provided at least 2 of R³ to R⁷ are equal to H, or
- SO₂NR⁸R⁹ or CH₂-NHC(=O)NH-R⁹ provided at least 4 of R³ to R⁷ are equal to H,
wherein R⁸ is selected from H or C₁-C₄ alkyl and R⁹ is selected from H, C₁-C₄ alkyl, benzyl optionally substituted with 1, 2, 3 or 4 C₁-C₄ alkoxy, and a group 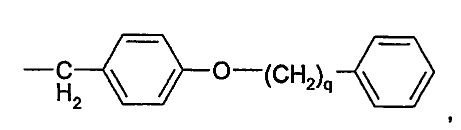 wherein q is an integer selected from 0, 1, 2 and 3.

9. A compound according to claim 1, said compound being of formula (1a) 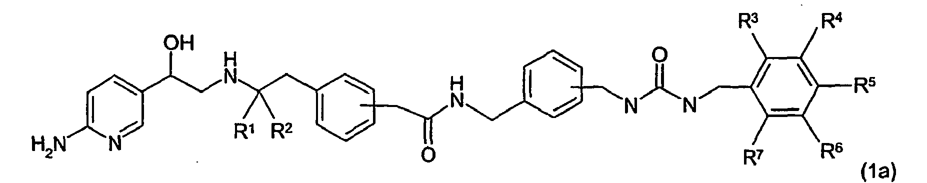 wherein the CH₂-C(=O)NH-benzyl and the CH₂-NHC(=O)NH-benzyl-Q¹-Q²-Q³-Q⁴ groups are independently in para or meta position and,
wherein, R³, R⁴, R⁵, R⁶ and R⁷ are independently selected from H, C₁-C₄ alkyl, OR⁹, phenoxy, SR⁹, halo, CF₃, OCF₃, COOR⁹, SO₂NR⁸R⁹, CONR⁸R⁹, NHR⁹R¹⁰ and NHCOR⁹, provided at least 2 of R³ to R⁷ are equal to H;
wherein R⁸ is selected from H or C₁-C₄ alkyl and R⁹ is selected from H, C₁-C₄ alkyl and benzyl optionally substituted with 1, 2, 3 or 4 C₁-C₄ alkoxy.

10. A compound according to claim 9 wherein R¹ is CH₃ and R² is H, and,
R³, R⁴, R⁵, R⁶ and R⁷ are independently selected from H,
- OR⁹, phenoxy provided at least 2 of R³ to R⁷ are equal to H, or
- SO₂NR⁸R⁹ provided at least 4 of R³ to R⁷ are equal to H,
wherein R⁸ is selected from H or C₁-C₄ alkyl and R⁹ is selected from H, C₁-C₄ alkyl, phenyl and benzyl optionally substituted with 1, 2, 3 or 4 C₁-C₄ alkoxy.

11. A compound according to claim 9 wherein R¹ is CH₃ and R² is H, and
R³ R⁴, R⁵, R⁶ and R⁷ are independently selected from H phenoxy and OR⁹, provided at least 2 of R³ to R⁷ are equal to H, wherein R⁹ is selected from H and C₁-C₄ alkyl.

12. The (*R,R*)-stereoisomer of a compound according to any one of claims 1 to 11, wherein R¹ is hydrogen and R² is C₁-C₄ alkyl.

13. A compound according to any one of claims 1 to 12 wherein the CH₂-C(=O)NH-benzyl-Q¹-Q²-Q³-Q⁴ group is in position meta.

14. A compound according to claim 1 selected from the group consisting of
4-{[2-(3-{2-[(2*R*)-2-(6-Amino-pyridin-3-yl)-2-hydroxy-ethylamino]-propyl}-phenyl)-acetylamino]-methyl}-*N*-(3,4-dimethoxy-benzyl)-benzamide,
4-{[2-(3-{2-[(2*R*)-2-(6-Amino-pyridin-3-yl)-2-hydroxy-ethylamino]-propyl}-phenyl)-acetylamino]-methyl}-*N*-(4-sulfamoyl-benzyl)-benzamide,
4-{[2-(3-{2-[(2*R*)-2-(6-Amino-pyridin-3-yl)-2-hydroxy-ethylamino]-propyl}-phenyl)-acetylamino]-methyl}-*N*-(2-ethoxy-benzyl)-benzamide,
4-{[2-(3-{2-[(2*R*)-2-(6-Amino-pyridin-3-yl)-2-hydroxy-ethylamino]-propyl}-phenyl)-acetylamino]-methyl}-*N*-[(1*R*)-1-(4-methoxy-phenyl)-ethyl]-benzamide.
4-{[2-(3-{2-[(2*R*)-2-(6-Amino-pyridin-3-yl)-2-hydroxy-ethylamino]-propyl}-phenyl)-acetylamino]-methyl}-*N*-((1*R*)-1-hydroxymethyl-2-phenyl-ethyl)-benzamide,
4-{[2-(3-{2-[(2*R*)-2-(6-Amino-pyridin-3-yl)-2-hydroxy-ethylamino]-propyl}-phenyl)-acetylamino]-methyl}-*N*-((1*R*, 2*S*)-2-hydroxy-1-methyl-2-phenyl-ethyl)-benzamide,
4-{[2-(3-{2-[(2*R*)-2-(6-Amino-pyridin-3-yl)-2-hydroxy-ethylamino]-propyl}-phenyl)-acetylamino]-methyl}-*N*-((1*S*, 2*R*)-2-hydroxy-1-methyl-2-phenyl-ethyl)-benzamide,
4-{[2-(3-{2-[(2*R*)-2-(6-Amino-pyridin-3-yl)-2-hydroxy-ethylamino]-propyl}-phenyl)-acetylamino]-methyl}-*N-*[2-(3-methoxy-phenyl)-ethyl]-benzamide,
4-{[2-(3-{(2*R*)-2-[(2*R*)-2-(6-Amino-pyridin-3-yl)-2-hydroxy-ethylamino]-propyl}-phenyl)-acetylamino]-methyl}-*N*-(3,4-dimethoxy-benzyl)-benzamide,
4-{[2-(3-{(2*R*)-2-[(2*R*)-2-(6-Amino-pyridin-3-yl)-2-hydroxy-ethylamino]-propyl}-phenyl)-acetylamino]-methyl}-*N-*(2-ethoxy-benzyl)-benzamide,
4-{[2-(3-{(2*R*)-2-[(2*R*)-2-(6-Amino-pyridin-3-yl)-2-hydroxy-ethylamino]-propyl}-phenyl)-acetylamino]-methyl}-*N*-(4-hydroxy-3-methoxy-benzyl)-benzamide,
4-{[2-(3-{(2*R*)-2-[(2*R*)-2-(6-Amino-pyridin-3-yl)-2-hydroxy-ethylamino]-propyl}-phenyl)-acetylamino]-methyl}-*N*-(4-sulfamoyl-benzyl)-benzamide,
4-{[2-(3-{(2*R*)-2-[(2*R*)-2-(6-Amino-pyridin-3-yl)-2-hydroxy-ethylamino]-propyl}-phenyl)-acetylamino]-methyl}-*N*-((1*R*, 2*S*)-2-hydroxy-1-methyl-2-phenyl-ethyl)-benzamide,
N-(4-{[2-(3-{(2*R*)-2-[(2*R*)-2-(6-Amino-pyridin-3-yi)-2-hydroxy-ethylamino]-propyl}-phenyl)-acetylamino]-methyl}-benzyl)-2,2-diphenyl-acetamide,
2-(3-{(2*R*)-2-[(2*R*)-2-(6-Amino-pyridin-3-yl)-2-hydroxy-ethylamino]-propyl}-phenyl)-*N*-{4-[(benzhydryl-amino)-methyl]-benzyl}-acetamide,
4-{[2-(3-{(2*R*)-2-[(2*R*)-2-(6-Amino-pyridin-3-yl)-2-hydroxy-ethylamino]-propyl}-phenyl)-acetylamino]-methyl}-*N-*(2-benzyloxy-ethyl)-benzamide,
4-{[2-(3-{(2*R*)-2-[(2*R*)-2-(6-Amino-pyridin-3-yl)-2-hydroxy-ethylamino]-propyl}-phenyl)-acetylamino]-methyl}-*N-*[2-(3-phenyl-propoxy)-ethyl]-benzamide,
4-{[2-(3-{(2*R*)-2-[(2*R*)-2-(6-Amino-pyridin-3-yl)-2-hydroxy-ethylamino]-propyl}-phenyl)-acetylamino]-methyl}-*N*-[2-(naphthalen-1-ylmethoxy)-ethyl]-benzamide,
4-{[2-(3-{(2*R*)-2-[(2*R*)-2-(6-Amino-pyridin-3-yl)-2-hydroxy-ethylamino]-propyl}-phenyl)-acetylamino]-methyl}-*N-*(3-benzylsulfamoyl-benzyl)-benzamide,
4-{[2-(3-{(2*R*)-2-[(2*R*)-2-(6-Amino-pyridin-3-ylr2-hydroxy-ethylamino]-propyl}-phenyl)-acetylamino]-methyl}-*N*-(4-methylsulfamoyl-benzyl)-benzamide,
4-{[2-(3-{(2*R*)-2-[(2*R*)-2-(6-Amino-pyridin-3-yl)-2-hydroxy-ethylamino]-propyl}-phenyl)-acetylamino]-methyl}-*N-*(4-ethylsulfamoyl-benzyl)-benzamide,
4-{[2-(3-{(2*R*)-2-[(2*R*)-2-(6-Amino-pyridin-3-yl)-2-hydroxy-ethylamino]-propyl}-phenyl)-acetylamino]-methyl}-*N-*(3-benzylsulfamoyl-benzyl)-benzamide,
4-{[2-(3-{(2*R*)-2-[(2*R*)-2-(6-Amino-pyridin-3-yl)-2-hydroxy-ethylamino]-propyl}-phenyl)-acetylamino]-methyl}-*N*-(3-methylsulfamoyl-benzyl)-benzamide,
4-{[2-(3-{(2*R*)-2-[(2*R*)-2-(6-Amino-pyridin-3-yl)-2-hydroxy-ethylamino]-propyl}-phenyl)-acetylamino]-methyl}-*N-*(3-ethylsulfamoyl-benzyl)-benzamide,
2-(3-{(2*R*)-2-[(2*R*)-2-(6-Amino-pyridin-3-yl)-2-hydroxy-ethylamino]-propyl}-phenyl)-*N-*(4-benzylsulfamoyl-benzyl)-acetamide hydrochloride,
2-(3-{(2*R*)-2-[(2*R*)-2-(6-Amino-pyridin-3-yl)-2-hydroxy-ethylamino]-propyl}-phenyl)-*N*-(3-benzylsulfamoyl-benzyl)-acetamide hydrochloride,
4-{[2-(4-{2-[(2*R*)-2-(6-Amino-pyridin-3-yl)-2-hydroxy-ethylamino]-propyl}-phenyl)-acetylamino]-methyl}-*N*-(3,4-dimethoxy-benzyl)-benzamide,
4-{[2-(4-{2-[(2*R*)-2-(6-Amino-pyridin-3-yl)-2-hydroxy-ethylamino]-propyl}-phenyl)-acetylamino]-methyl}-*N*-(2-ethoxy-benzyl)-benzamide,
4-{[2-(4-{2-[(2*R*)-2-(6-Amino-pyridin-3-yl)-2-hydroxy-ethylamino]-propyl}-phenyl)-acetylamino]-methyl}-*N*-[(1*R*)-1-(4-methoxy-phenyl)-ethyl]-benzamide,
4-{[2-(4-{2-[(2*R*)-2-(6-Amino-pyridin-3-yl)-2-hydroxy-ethylamino]-propyl}-phenyl)-acetylamino]-methyl}-*N*-((1*R*)-1-benzyl-2-hydroxy-ethyl)-benzamide,
4-{[2-(4-{2-[(2*R*)-2-(6-Amino-pyridin-3-yl)-2-hydroxy-ethylamino]-propyl}-phenyl)-acetylamino]-methyl}-*N*-((1*R*, 2*S*)-2-hydroxy-1-methyl-2-phenyl-ethyl)-benzamide,
4-{[2-(4-{2-[(2*R*)-2-(6-Amino-pyridin-3-yl)-2-hydroxy-ethylamino]-propyl}-phenyl)-acetylamino]-methyl}-*N*-((1*S*, 2*R*)-2-hydroxy-1-methyl-2-phenyl-ethyl)-benzamide,
4-{[2-(4-{2-[(2*R*)-2-(6-Amino-pyridin-3-yl)-2-hydroxy-ethylamino]-propyl}-phenyl)-acetylamino]-methyl}-*N*-[2-(3-methoxy-phenyl)-ethyl]-benzamide,
4-{[2-(3-{(2*R*)-2-[(2*R*)-2-(6-Amino-pyridin-3-yl)-2-hydroxy-ethylamino]-propyl}-phenyl)-acetylamino]-methyl]-*N*-[4-(3-benzyl-ureidomethyl)-benzyl]-benzamide,
4-{[2-(3-{(2*R*)-2-[(2*R*)-2-(6-Amino-pyridin-3-yl)-2-hydroxy-ethylamino]-propyl}-phenyl)-acetylamino]-methyl}-*N*-{4-[3-(3,4-dimethoxy-benzyl)-ureidomethyl]-benzyl}-benzamide,
4-{[2-(3-{(2*R*)-2-[(2*R*)-2-(6-Amino-pyridin-3-yl)-2-hydroxy-ethylamino]-propyl}-phenyl)-acetylamino]-methyl}-*N*-{4-[3-(4-phenoxy-benzyl)-ureidomethyl]-benzyl}-benzamide,
4-{[2-(3-{(2*R*)-2-[(2*R*)2-(6-Amino-pyridin-3-yl)-2-hydroxy-ethylamino]-propyl}-phenyl)-acetylamino]-methyl}-*N*-{4-[3-(4-benzyloxy-benzyl)-ureidomethyl]-benzyl}-benzamide,
2-(3-{(2*R*)-2-[(2*R*)-2-(6-Amino-pyridin-3-yl)-2-hydroxy-ethylamino]-propyl}-phenyl)-*N*-{4-[3-(3-phenoxy-benzyl)-ureidomethyl]-benzyl}-acetamide,
2-(3-{(2*R*)-2-[(2*R*)-2-(6-Amino-pyridin-3-yl)-2-hydroxy-ethylamino]-propyl}-phenyl)-*N*-{4-[3-(3,4-dimethoxy-benzyl)-ureidomethyl]-benzyl}-acetamide, and,
2-(3-{(2*R*)-2-[(2*R*)-2-(6-Amino-pyridin-3-yl)-2-hydroxy-ethylamino]-propyl}-phenyl)-*N*-{4-[3-(4-phenoxy-benzyl)-ureidomethyl]-benzyl}-acetamide.

15. A process for the preparation of a compound of the formula (1) as described in any one of claims 1 to 14 or a pharmaceutically acceptable salt or derived form thereof **characterized in that** it comprises the step of
(a) coupling an acid of formula (3) : 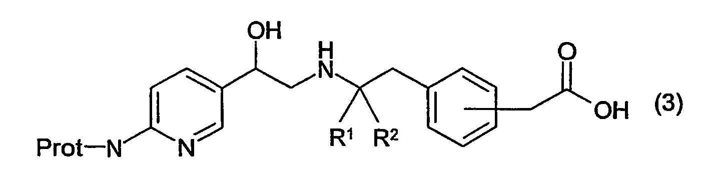 wherein Prot is a protecting group, R¹ and R² are as defined in claim 1,
with an amine of formula (4) : 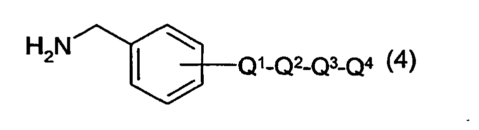 wherein Q¹, Q², Q³ and Q⁴ are as defined in claim 1,
(b) removing the protecting group "Prot" from the compound of formula (2), 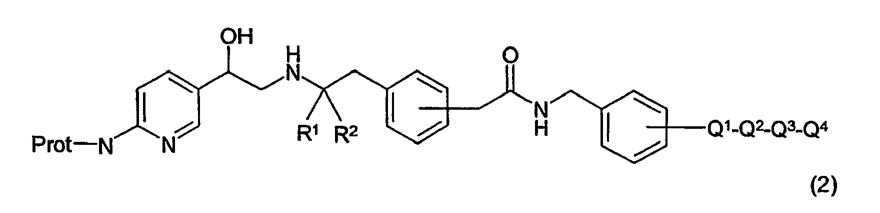
(c) isolating said compound of formula (1).

16. A pharmaceutical composition including a compound of the formula (1) as described in any one of claims 1 to 14 or a pharmaceutically acceptable salt or derived form thereof, together with customary pharmaceutically innocuous excipients and/or additives.

17. A compound of the formula (1) as described in any one of claims 1 to 14 or a pharmaceutically acceptable salt, derived form or composition thereof, for use as a medicament.

18. A compound of the formula (1) as described in any one of claims 1 to 14 or a pharmaceutically acceptable salt, derived form or composition thereof, for use in the treatment of diseases, disorders, and conditions in which the β2 receptor is involved.

19. A compound of the formula (1) as described in any one of claims 1 to 14 or a pharmaceutically acceptable salt, derived form or composition thereof, for use in the treatment of diseases, disorders, and conditions selected from the group consisting of :
• asthma of whatever type, etiology, or pathogenesis, in particular asthma that is a member selected from the group consisting of atopic asthma, non-atopic asthma, allergic asthma, atopic bronchial IgE-mediated asthma, bronchial asthma, essential asthma, true asthma, intrinsic asthma caused by pathophysiologic disturbances, extrinsic asthma caused by environmental factors, essential asthma of unknown or inapparent cause, non-atopic asthma, bronchitic asthma, emphysematous asthma, exercise-induced asthma, allergen induced asthma, cold air induced asthma, occupational asthma, infective asthma caused by bacterial, fungal, protozoal, or viral infection, non-allergic asthma, incipient asthma, wheezy infant syndrome and bronchiolytis,
• chronic or acute bronchoconstriction, chronic bronchitis, small airways obstruction, and emphysema,
• obstructive or inflammatory airways diseases of whatever type, etiology, or pathogenesis, in particular an obstructive or inflammatory airways disease that is a member selected from the group consisting of chronic eosinophilic pneumonia, chronic obstructive pulmonary disease (COPD), COPD that includes chronic bronchitis, pulmonary emphysema or dyspnea associated or not associated with COPD, COPD that is **characterized by** irreversible, progressive airways obstruction, adult respiratory distress syndrome (ARDS), exacerbation of airways hyper-reactivity consequent to other drug therapy and airways disease that is associated with pulmonary hypertension,
• bronchitis of whatever type, etiology, or pathogenesis, in particular bronchitis that is a member selected from the group consisting of acute bronchitis, acute laryngotracheal bronchitis, arachidic bronchitis, catarrhal bronchitis, croupus bronchitis, dry bronchitis, infectious asthmatic bronchitis, productive bronchitis, staphylococcus or streptococcal bronchitis and vesicular bronchitis,
• bronchiectasis of whatever type, etiology, or pathogenesis, in particular bronchiectasis that is a member selected from the group consisting of cylindric bronchiectasis, sacculated bronchiectasis, fusiform bronchiectasis, capillary bronchiectasis, cystic bronchiectasis, dry bronchiectasis and follicular bronchiectasis.

20. The use of a compound of the formula (1) as described in any one of claims 1 to 14 or of a pharmaceutically acceptable salt derived form or composition thereof, for the manufacture of a drug having a β2 agonist activity.

21. The use of a compound of the formula (1) as described in any one of claims 1 to 14 or of a pharmaceutically acceptable salt, solvate or composition thereof, for the manufacture of a drug for the treatment of diseases, disorders, and conditions selected from the group as described in claim 19.

22. Combination of a compound according to any one of claims 1 to 14 with other therapeutic agent(s) selected from:
(a) 5-Lipoxygenase (5-LO) inhibitors or 5-lipoxygenase activating protein (FLAP) antagonists,
(b) Leukotriene antagonists (LTRAs) including antagonists of LTB₄, LTC₄, LTD₄, and LTE₄,
(c) Histamine receptor antagonists including H1 and H3 antagonists,
(d) α₁- and α₂-adrenoceptor agonist vasoconstrictor sympathomimetic agents for decongestant use,
(e) muscarinic M3 receptor antagonists or anticholinergic agents,
(f) PDE inhibitors, e.g. PDE3, PDE4 and PDE5 inhibitors,
(g) Theophylline,
(h) Sodium cromoglycate,
(i) COX Inhibitors both non-selective and selective COX-1 or COX-2 inhibitors (NSAIDs),
(j) Oral and inhaled glucocorticosteroids,
(k) Monoclonal antibodies active against endogenous inflammatory entities,
(l) Anti-tumor necrosis factor (anti-TNF-α) agents,
(m) Adhesion molecule inhibitors including VLA-4 antagonists,
(n) Kinin-B₁ - and B₂ -receptor antagonists,
(o) Immunosuppressive agents,
(p) Inhibitors of matrix metalloproteases (MMPs),
(q) Tachykinin NK₁, NK₂ and NK₃ receptor antagonists,
(r) Elastase inhibitors,
(s) Adenosine A2a receptor agonists,
(t) Inhibitors of urokinase,
(u) Compounds that act on dopamine receptors, e.g. D2 agonists,
(v) Modulators of the NFκβ pathway, e.g. IKK inhibitors,
(w)modulators of cytokine signalling pathyways such as p38 MAP kinase or syk kinase,
(x) Agents that can be classed as mucolytics or anti-tussive, and
(y) Antibiotics.

## Patentansprüche

1. Verbindung der Formel (1): 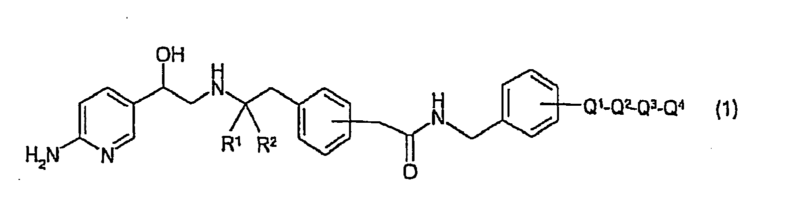 worin die CH₂-C(=O)NH-Benzyl-Q¹-Q²-Q³-Q⁴-Gruppe in der meta- oder para-Stellung ist und
R¹ und R² unabhängig ausgewählt sind aus H und C₁-C₄-Alkyl;
Q¹ (CH₂)ₙ ist, worin n eine ganze Zahl, ausgewählt aus 0 und 1, ist;
Q² eine Gruppe ist, ausgewählt aus NH, -C(=O)NH-, -NHC(=O)-, -NH-C(O)-NH- and -SO₂NH-;
Q₃ eine Einfachbindung, ein C₁-C₄-Alkylen, gegebenenfalls substituiert mit OH, oder eine Gruppe (CH₂)ₘ-O-(CH₂)ₚ ist, worin m und p ganze Zahlen, unabhängig ausgewählt aus 1, 2 oder 3, sind;
Q₄ ausgewählt ist aus 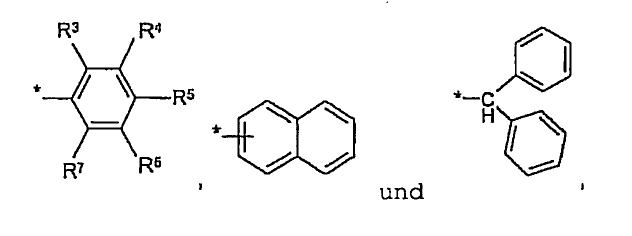 worin * den Anheftungspunkt an Q³ darstellt und R³, R⁴, R⁵, R⁶ und R⁷ unabhängig ausgewählt sind aus H, Phenoxy, C₁-C₄-Alkyl, OR⁹, SR⁹ , Halogen, CF₃, OCF₃, COOR⁹ , SO₂NR⁸R⁹, CONR⁸R⁹, NHR⁸R⁹, NHCOR⁹, CH₂-NHC(=O)NH-R⁹, mit der Maßgabe, dass wenigstens zwei von R³ bis R⁷ gleich H sind;
worin R⁸ aus H oder C₁-C₄-Alkyl ausgewählt ist und R⁹ aus H, C₁-C₄-Alkyl, Benzyl, gegebenenfalls substituiert mit 1, 2, 3 oder 4 C₁-C₄-Alkoxy, und einer Gruppe 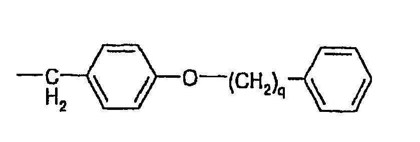 ausgewählt ist, worin q eine ganze Zahl, ausgewählt aus 0, 1, 2 und 3, ist;
oder, sofern zweckdienlich, deren pharmazeutisch verträgliche Salze und/oder Isomere, Tautomere, Solvate oder isotopische Variationen davon.

2. Verbindung nach Anspruch 1, worin R¹ H ist und R² CH₃ ist.

3. Verbindung nach Anspruch 1, worin R¹ CH₃ ist und R² CH₃ ist.

4. Verbindung nach einem der Ansprüche 1 bis 3, worin n 0 ist und Q² -C(=O)NH- oder SO₂NH- ist.

5. Verbindung nach einem der Ansprüche 1 bis 3, worin n 1 ist und Q² -NH-C(=O)- oder -NH-C(O)-NH- ist.

6. Verbindung nach einem der Ansprüche 1 bis 5, worin Q³ ausgewählt ist aus einer Einfachbindung, CH₂, CH(-CH₃)CH₂OH-, CH(CH₃)-, -CH(CH₂OH)CH₂-, -(CH₂)₂-, -(CH₂)₂-OCH₂- und (CH₂)₂-O-(CH₂)₃- ist.

7. Verbindung nach einem der Ansprüche 1 bis 6, worin Q⁴ ausgewählt ist aus 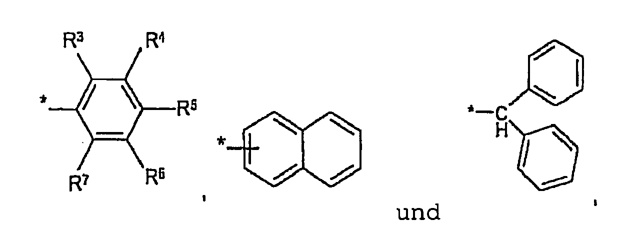 worin * den Anheftungspunkt an Q³ darstellt und R³, R⁴, R⁵, R⁶ und R⁷ unabhängig ausgewählt sind aus H,
- OR⁹, mit der Maßgabe, dass wenigstens 2 von R³ bis R⁷ gleich H sind, oder
- SO₂NR⁸R⁹ oder CH₂-NHC(=O)NH-R⁹, mit der Maßgabe, dass wenigstens 4 von R³ bis R⁷ gleich H sind,
worin R⁸ ausgewählt ist aus H oder C₁-C₄-Alkyl und R⁹ ausgewählt ist aus H, C₁-C₄-Alkyl, Benzyl, gegebenenfalls substituiert mit 1, 2, 3 oder 4 C₁-C₄-Alkoxy, und einer Gruppe 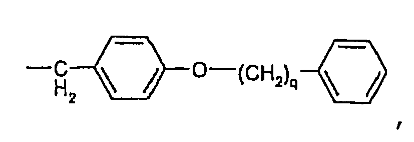 worin q eine ganze Zahl, ausgewählt aus 0, 1, 2 und 3, ist.

8. Verbindung nach einem der Ansprüche 1 bis 6, worin Q⁴ ausgewählt ist aus 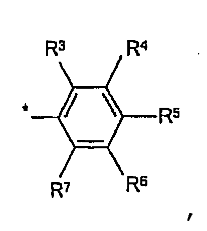 worin * den Anheftungspunkt an Q³ darstellt und R³, R⁴, R⁵, R⁶ und R⁷ unabhängig ausgewählt sind aus H,
- OR⁹, mit der Maßgabe, dass wenigstens 2 von R³ bis R⁷ gleich H sind, oder
- SO₂NR⁸R⁹ oder CH₂-NHC(=O)NH-R⁹, mit der Maßgabe, dass wenigstens 4 von R³ bis R⁷ gleich H sind,
worin R⁸ ausgewählt ist aus H oder C₁-C₄-Alkyl und R⁹ ausgewählt ist aus H, C₁-C₄-Alkyl, Benzyl, gegebenenfalls substituiert mit 1, 2, 3 oder 4 C₁-C₄-Alkoxy, und einer Gruppe 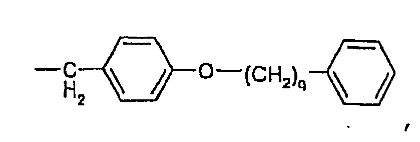 worin q eine ganze Zahl, ausgewählt aus 0, 1, 2 und 3, ist.

9. Verbindung nach Anspruch 1, wobei die Verbindung die Formel (1a) hat 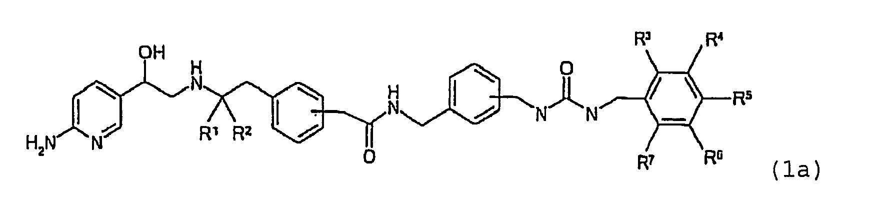 worin die CH₂-C(=O)NH-Benzyl- und die CH₂-NHC(=O)NH-Benzyl-Q¹-Q²-Q³-Q⁴-Gruppen unabhängig in para- oder meta-Stellung sind und
worin R³, R⁴, R⁵, R⁶ und R⁷ unabhängig ausgewählt sind aus H, C₁-C₄-Alkyl, OR⁹ Phenoxy, SR⁹, Halogen, CF₃, OCF₃, COOR⁹, SO₂NR⁸R⁹, CONR⁸R⁹, NHR⁹R¹⁰ und NHCOR⁹, mit der Maßgabe, dass wenigstens 2 von R³ bis R⁷ gleich H sind;
worin R⁸ ausgewählt ist aus H oder C₁-C₄-Alkyl und R⁹ ausgewählt ist aus H, C₁-C₄-Alkyl und Benzyl, gegebenenfalls substituiert mit 1, 2, 3 oder 4 C₁-C₄-Alkoxy.

10. Verbindung nach Anspruch 9, worin R¹ CH₃ ist und R² H ist und R³, R⁴, R⁵ , R⁶ und R⁷ unabhängig ausgewählt sind aus H,
- OR⁹, Phenoxy, mit der Maßgabe, dass wenigstens 2 von R³ bis R⁷ gleich H sind, oder
- SO₂NR⁸R⁹ mit der Maßgabe, dass wenigstens 4 von R³ bis R⁷ gleich H sind,
worin R⁸ ausgewählt ist aus H oder C₁-C₄-Alkyl und R⁹ ausgewählt ist aus H, C₁-C₄-Alkyl, Phenyl und Benzyl, gegebenenfalls substituiert mit 1, 2, 3 oder 4 C₁-C₄-Alkoxy.

11. Verbindung nach Anspruch 9, worin R¹ CH₃ ist und R² H ist und
R³, R⁴, R⁵, R⁶ und R⁷ unabhängig ausgewählt sind aus H, Phenoxy und OR⁹, mit der Maßgabe, dass wenigstens 2 von R³ bis R⁷ gleich H sind, worin R⁹ ausgewählt ist aus H und C₁-C₄-Alkyl.

12. (*R,R*)-Stereoisomer einer Verbindung nach einem der Ansprüche 1 bis 11, worin R¹ Wasserstoff ist und R² C₁-C₄-Alkyl ist.

13. Verbindung nach einem der Ansprüche 1 bis 12, worin die CH₂-C(=O)NH-Benzyl-Q¹-Q²-Q³-Q⁴-Gruppe in meta-Stellung ist.

14. Verbindung nach Anspruch 1, ausgewählt aus der Gruppe, bestehend aus
4-{[2-(3-{2-[(2*R*)-2-(6-Aminopyridin-3-yl)-2-hydroxyethylamino]propyl}phenyl)acetylamino]methyl}-*N*-(3,4-dimethoxybenzyl)benzamid,
4-{[2-(3-{2-[(2*R*)-2-(6-Aminopyridin-3-yl)-2-hydroxyethylamino]propyl}phenyl)acetylamino]methyl}-*N*-(4-sulfamoylbenzyl)benzamid,
4-{[2-(3-{2-[(2*R*)-2-(6-Aminopyridin-3-yl)-2-hydroxyethylamino]propyl}phenyl)acetylamino]methyl}-*N*-(2-ethoxybenzyl)benzamid,
4-{[2-(3-{2-[(2*R*)-2-(6-Aminopyridin-3-yl)-2-hydroxyethylamino]propyl}phenyl)acetylamino]methyl}-*N*-[(1*R*)-1-(4-methoxyphenyl)ethyl]benzamid,
4-{[2-(3-{2-[(2*R*)-2-(6-Aminopyridin-3-yl)-2-hydroxyethylamino]propyl}phenyl)acetylamino]methyl}-*N*-((1*R*)-1-hydroxymethyl-2-phenylethyl)benzamid,
4-{[2-(3-{2-[(2*R*)-2-(6-Aminopyridin-3-yl)-2-hydroxyethylamino]propyl}phenyl)acetylaminol]methyl}-*N*-((1*R*, 2*S*)-2-hydroxy-1-methyl-2-phenylethyl)benzamid,
4-{[2-(3-{2-[(2*R*)-2-(6-Aminopyridin-3-yl)-2-hydroxyethylamino]propyl}phenyl)acetylamino]methyl}-*N*-((1*S*, 2*R*)-2-hydroxy-1-methyl-2-phenylethyl)benzamid,
4-{[2-(3-{2-[(2*R*)-2-(6-Aminopyridin-3-yl)-2-hydroxyethylamino]propyl}phenyl)acetylamino]methyl}-*N*-[2-(3-methoxyphenyl)ethyl]benzamid,
4-{[2-(3-{(2*R*)-2-[(2*R)*-2-(6-Aminopyridin-3-yl)-2-hydroxyethylamino]propyl}phenyl)acetylamino]methyl}-*N*-(3,4-dimethoxybenzyl)benzamid,
4-{[2-(3-{(2*R*)-2-[(2*R*)-2-(6-Aminopyridin-3-yl)-2-hydroxyethylamino]propyl}phenyl)acetylamino]methyl}-*N*-(2-ethoxybenzyl)benzamid,
4-{[2-(3-{(2*R*)-2-[(2*R*)-2-(6-Aminopyridin-3-yl)-2-hydroxyethylamino]propyl}phenyl)acetylamino]methyl}-*N*-(4-hydroxy-3-methoxybenzyl)benzamid,
4-{[2-(3-{(2*R*)-2-[(2*R*)-2-(6-Aminopyridin-3-yl)-2-hydroxyethylamino]propyl}phenyl)acetylamino]methyl}-*N*-(4-sulfamoylbenzyl)benzamid,
4-{[2-(3-{(2*R*)-2-[(2*R*)-2-(6-Aminopyridin-3-yl)-2-hydroxyethylamino]propyl}phenyl)acetylamino]methyl}-*N*-((1*R*, 2*S*)-2-hydroxy-1-methyl-2-phenylethyl)benzamid,
N-(4-{[2-(3-{(2*R*)-2-[(2*R*)-2-(6-Aminopyridin-3-yl)-2-hydroxyethylamino]propyl}phenyl)acetylamino]methyl}-benzyl)-2,2-diphenylacetamid,
2-(3-{(2*R*)-2-[(2*R*)-2-(6-Aminopyridin-3-yl)-2-hydroxyethylamino]propyl}phenyl)-*N*-{4-[(benzhydrylamino)methyl]benzyl}acetamid,
4-{[2-(3-{(2*R*)-2-[(2*R*)-2-(6-Aminopyridin-3-yl)-2-hydroxyethylamino]propyl}phenyl)acetylamino]methyl}-*N*-(2-benzyloxyethyl)benzamid,
4-{[2-(3-{(2*R*)-2-[(2*R*)-2-(6-Aminopyridin-3-yl)-2-hydroxyethylamino]propyl}phenyl)acetylamino]methyl}-*N*-[2-(3-phenylpropoxy)ethyl]benzamid,
4-{[2-(3-{(2*R*)-2-[(2*R*)-2-(6-Aminopyridin-3-yl)-2-hydroxyethylamino]propyl}phenyl)acetylamino]methyl}-*N*-[2-(naphthalen-1-ylmethoxy)ethyl]benzamid,
4-{[2-(3-{(2*R*)-2-[(2*R*)-2-(6-Aminopyridin-3-yl)-2-hydroxyethylamino]propyl}phenyl)acetylamino]methyl}-*N*-(3-benzylsulfamoylbenzyl)benzamid,
4-{[2-(3-{(2*R*)-2-[(2*R*)-2-(6-Aminopyridin-3-yl)-2-hydroxyethylamino]propyl}phenyl)acetylamino]methyl}*-N*-(4-methylsulfamoylbenzyl)benzamid,
4-{[2-(3-{(2*R*)-2-[(2*R*)-2-(6-Aminopyridin-3-yl)-2-hydroxyethylamino]propyl}phenyl)acetylamino]methyl}-*N*-(4-ethylsulfamoylbenzyl)benzamid,
4-{[2-(3-{(2*R*)-2-[(2*R*)-2-(6-Aminopyridin-3-yl)-2-hydroxyethylamino]propyl}phenyl)acetylamino]methyl}-*N*-(3-benzylsulfamoylbenzyl)benzamid,
4-{[2-(3-{(2*R*)-2-[(2*R*)-2-(6-Aminopyridin-3-yl)-2-hydroxyethylamino]propyl}phenyl)acetylamino]methyl}-*N*-(3-methylsulfamoylbenzyl)benzamid,
4-{[2-(3-{(2*R*)-2-[(2*R*)-2-(6-Aminopyridin-3-yl)-2-hydroxyethylamino]propyl}phenyl)acetylamino]methyl}-*N*-(3-ethylsulfamoylbenzyl)benzamid,
2-(3-{(2*R*)-2-[(2*R*)-2-(6-Aminopyridin-3-yl)-2-hydroxyethylamino]propyl}phenyl)-*N*-(4-benzylsulfamoylbenzyl)acetamid-Hydrochlorid,
2-(3-{(2*R*)-2-[(2*R*)-2-(6-Aminopyridin-3-yl)-2-hydroxyethylamino]propyl}phenyl)-*N*-(3-benzylsulfamoylbenzyl)acetamid-Hydrochlorid,
4-{[2-(4-{2-[(2*R*)-2-(6-Aminopyridin-3-yl)-2-hydroxyethylamino]propyl}phenyl)acetylamino]methyl}-*N*-(3,4-dimethoxybenzyl)benzamid,
4-{[2-(4-{2-[(2*R*)-2-(6-Aminopyridin-3-yl)-2-hydroxyethylamino]propyl}phenyl)acetylamino]methyl}-*N*-(2-ethoxybenzyl)benzamid,
4-{[2-(4-{2-[(2*R*)-2-(6-Aminopyridin-3-yl)-2-hydroxyethylamino]propyl}phenyl)acetylamino]methyl}-*N*-[(1*R*)-1-(4-methoxyphenyl)ethyl]benzamid,
4-{[2-(4-{2-[(2*R*)-2-(6-Aminopyridin-3-yl)-2-hydroxyethylamino]propyl}phenyl)acetylamino]methyl}-*N*-((1*R*)-1-benzyl-2-hydroxyethyl)benzamid,
4-{[2-(4-{2-[(2*R*)-2-(6-Aminopyridin-3-yl)-2-hydroxyethylamino]propyl}phenyl)acetylamino]methyl}-*N-*((1*R*,2*S*)-2-hydroxy-1-methyl-2-phenylethyl)benzamid,
4-{[2-(4-{2-[(2*R*)-2-(6-Aminopyridin-3-yl)-2-hydroxyethylamino]propyl}phenyl)acetylamino]methyl}-N-((1S, 2R)-2-hydroxy-1-methyl-2-phenylkethyl)benzamid,
4-{[2-(4-{2-[(2*R*)-2-(6-Aminopyridin-3-yl)-2-hydroxyethylamino]propyl}phenyl)acetylamino]methyl}-*N*-[2-(3-methoxyphenyl)ethyl]benzamid,
4-{[2-(3-{(2*R*)-2-[(2*R*)-2-(6-Aminopyridin-3-yl)-2-hydroxyethylamino]propyl}phenyl)acetylamino]methyl}-*N*-[4-(3-benzylureidomethyl)benzyl]benzamid,
4-{[2-(3-{(2*R*)-2-[(2*R*)-2-(6-Aminopyridin-3-yl)-2-hydroxyethylamino]propyl}phenyl)acetylamino]methyl}-*N*-{4-[3-(3,4-dimethoxybenzyl)ureidomethyl]benzyl}benzamid,
4-{[2-(3-{(2*R*)-2-[(2*R*)-2-(6-Aminopyridin-3-yl)-2-hydroxyethylamino]propyl}phenyl)acetylamino]methyl}-*N*-{4-[3-(4-phenoxybenzyl)ureidomethyl]benzyl}benzamid,
4-{[2-(3-{(2*R*)-2-[(2*R*)-2-(6-Aminopyridin-3-yl)-2-hydroxyethylamino]propyl}phenyl)acetylamino]methyl}-*N*-{4-[3-(4-benzyloxybenzyl)ureidomethyl]benzyl}benzamid,
2-(3-{(2*R*)-2-[(2*R*)-2-(6-Aminopyridin-3-yl)-2-hydroxyethylamino]propyl}phenyl)-*N*-{4-[3-(3-phenoxybenzyl)ureidomethyl]benzyl}acetamid,
2-(3-{(2*R*)-2-[(2*R*)-2-(6-Aminopyridin-3-yl)-2-hydroxyethylamino]propyl}phenyl)-*N*-{4-[3-(3,4-dimethoxybenzyl)ureidomethyl]benzyl}acetamid und
2-(3-{(2*R*)-2-[(2*R*)-2-(6-Aminopyridin-3-yl)-2-hydroxyethylamino]propyl}phenyl)-*N*-{4-[3-(4-phenoxybenzyl)ureidomethyl]benzyl}acetamid.

15. Verfahren zur Herstellung einer Verbindung der Formel (1), wie in einem der Ansprüche 1 bis 14 beschrieben, oder eines pharmazeutisch verträglichen Salzes oder einer abgeleiteten Form davon, **dadurch gekennzeichnet, dass** es die Stufe der
(a) Kupplung einer Säure der Formel (3): 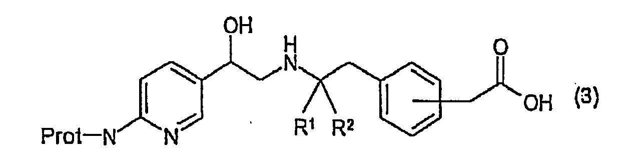 worin Prot eine Schutzgruppe ist, R¹ und R² wie in Anspruch 1 definiert sind,
mit einem Amin der Formel (4): 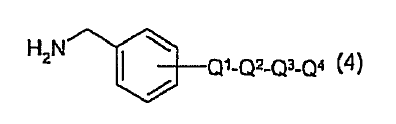 worin Q¹, Q², Q³ und Q⁴ wie in Anspruch 1 definiert sind,
(b) Entfernen der Schutzgruppe "Prot" aus der Verbindung der Formel (2), 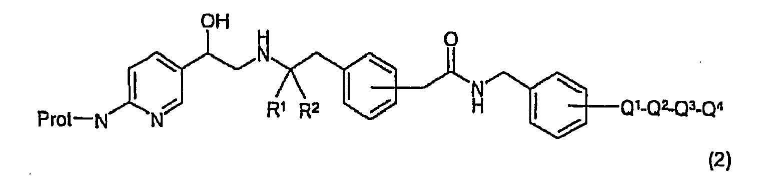
(c) Isolieren der Verbindung der Formel (1), umfasst.

16. Pharmazeutische Zusammensetzung, umfassend eine Verbindung der Formel (1), wie sie in einem der Ansprüche 1 bis 14 beschrieben ist, oder ein pharmazeutisch verträgliches Salz oder eine abgeleitete Form davon, zusammen mit herkömmlichen pharmazeutisch unschädlichen Exzipienzien und/oder Additiven.

17. Verbindung der Formel (1), wie sie in einem der Ansprüche 1 bis 14 beschrieben ist, oder ein pharmazeutisch verträgliches Salz, eine abgeleitete Form oder Zusammensetzung davon zur Verwendung als Medikament.

18. Verbindung der Formel (1), wie sie in einem der Ansprüche 1 bis 14 beschrieben ist, oder ein pharmazeutisch verträgliches Salz, eine abgeleitete Form oder Zusammensetzung davon zur Verwendung in der Behandlung von Erkrankungen, Störungen und Zuständen, an denen der β2-Rezeptor beteiligt ist.

19. Verbindung der Formel (1), wie sie in einem der Ansprüche 1 bis 14 beschrieben ist, oder ein pharmazeutisch verträgliches Salz, eine abgeleitete Form oder Zusammensetzung davon zur Verwendung in der Behandlung von Erkrankungen, Störungen und Zuständen, ausgewählt aus der Gruppe, bestehend aus:
• Asthma von beliebigem Typ, von beliebiger Ätiologie oder Pathogenese, insbesondere Asthma, das ein Mitglied ist, ausgewählt aus der Gruppe, bestehend aus atopischem Asthma, nicht-atopischem Asthma, allergischem Asthma, atopischem bronchialem IgE-vermitteltem Asthma, Bronchialasthma, essentiellem bzw. primärem Asthma, echtem Asthma, intrinsischem Asthma, verursacht durch pathophysiologische Störungen, extrinsischem Asthma, verursacht durch Umweltfaktoren, essentiellem Asthma unbekannter oder nicht offensichtlicher Ursache, nicht-atopischem Asthma, bronchitischem Asthma, emphysematösem Asthma, belastungsinduziertem Asthma, allergeninduziertem Asthma, durch kalte Luft induziertem Asthma, berufsbedingtem Asthma ("occupational asthma"), infektionsbedingtem Asthma, verursacht durch Bakterien-, Pilz-, Protozoen- oder Virusinfektion, nicht-allergischem Asthma, beginnendem Asthma, "wheezy infant"-Syndrom und Bronchiolytis,
• chronischer oder akuter Bronchokonstriktion, chronischer Bronchitis, Obstruktion der kleinen Luftwege und Emphysem,
• obstruktiven oder entzündlichen Luftwegserkrankungen von beliebigem Typ, beliebiger Ätiologie oder Pathogenese, insbesondere einer obstruktiven oder entzündlichen Luftwegserkrankung, die ein Mitglied ist, ausgewählt aus der Gruppe, bestehend aus chronischer eosinophiler Pneumonie, chronischer obstruktiver Lungenerkrankung (COPD), COPD, die chronische Bronchitis umfasst, Lungenemphysem oder Dyspnoe, assoziiert mit COPD oder nicht, COPD die **gekennzeichnet ist durch** irreversible progressive Luftwegsobstruktion, Atemnotsyndrom bei Erwachsenen (ARDS), Exazerbation der Hyperreaktivität von Luftwegen, folgend auf eine andere Arzneimitteltherapie, und Luftwegserkrankung, die mit pulmonaler Hypertonie assoziiert ist,
• Bronchitis von beliebigem Typ, beliebiger Ätiologie oder Pathogenese, insbesondere Bronchitis, die ein Mitglied ist, ausgewählt aus der Gruppe, bestehend aus akuter Bronchitis, akuter laryngotrachealer Bronchitis, erdnussverursachter Bronchitis ("arachidic bronchitis"), katharralischer Bronchitis, kruppöser Bronchitis, trockener Bronchitis, infektionsbedingter asthmatischer Bronchitis, produktiver Bronchitis, Staphylokokken- oder Streptokokkenbronchitis und Bläschenbronchitis,
• Bronchiektasie von beliebigem Typ, beliebiger Ätiologie oder Pathogenese, insbesondere Bronchiektasie, die ein Mitglied ist, ausgewählt aus der Gruppe, bestehend aus zylinderförmiger Bronchiektasie, sackförmiger Bronchiektasie, fusiformer Bronchiektasie, Bronchiolektasie ("a-pillary bronchiectasis"), zystischer Bronchiektasie, trockener Bronchiektasie und follikulärer Bronchiektasie ("follicular bronchiectasis").

20. Verwendung einer Verbindung der Formel (1), wie sie in einem der Ansprüche 1 bis 14 beschrieben ist, oder eines/einer pharmazeutisch verträglichen Salzes, abgeleiteten Form oder Zusammensetzung davon zur Herstellung eines Arzneimittels mit β2-Agonistenaktivität.

21. Verwendung einer Verbindung der Formel (1), wie sie in einem der Ansprüche 1 bis 14 beschrieben ist, oder eines/einer pharmazeutisch verträglichen Salzes, Solvats oder Zusammensetzung davon zur Herstellung eines Arzneimittels zur Behandlung von Erkrankungen, Störungen und Zuständen, ausgewählt aus der Gruppe, wie sie in Anspruch 19 beschrieben ist.

22. Kombination einer Verbindung nach einem der Ansprüche 1 bis 14 mit (einem) anderen therapeutischen Mittel(n), ausgewählt aus:
(a) 5-Lipoxygenase (5-LO)-Inhibitoren oder 5-Lipoxygenaseaktivierendes Protein (FLAP)-Antagonisten,
(b) Leukotrienantagonisten (LTRAs) einschließlich Antagonisten von LTB4, LTC4, LTD4 und LTE4,
(c) Histaminrezeptorantagonisten einschließlich H1- und H3-Antagonisten,
(d) α₁- and α₂-Adrenozeptoragonist-Vasokonstriktor-Sympathomimetika zur Verwendung als Entstauungsmittel,
(e) muscarinischen M3-Rezeptorantagonisten oder anticholinergen Mitteln,
(f) PDE-Inhibitoren, z. B. PDE3-, PDE4- und PDES-Inhibitoren,
(g) Theophyllin,
(h) Natriumcromoglycat,
(i) COX-Inhibitoren, sowohl nicht-selektive als auch selektive COX-1- oder COX-2-Inhibitoren (NSAIDs),
(j) Oralen und inhalierten Glucocorticosteroiden,
(k) monoklonalen Antikörpern, die gegen endogene Entzündungsfunktionseinheiten wirksam sind,
(l) Anti-Tumornekrosefaktor (anti-TNF-α)-Mitteln,
(m) Adhäsionsmolekül-Inhibitoren einschließlich VLA-4-Antagonisten,
(n) Kinin B1- und B2-Rezeptorantagonisten,
(o) Immunosuppressiven Mitteln,
(p) Inhibitoren von Matrix-Metalloproteasen (MMPs),
(q) Tachykinin NK1-, NK2- und NK3-Rezeptor-Antagonisten,
(r) Elastaseinhibitoren,
(s) Adenosin A2a-Rezeptoragonisten,
(t) Inhibitoren der Urokinase,
(u) Verbindungen, die auf Dopaminrezeptoren wirken, z. B. D2-Agonisten,
(v) Modulatoren des NFκβ-Wegs, z. B. IKK-Inhibitoren,
(w) Modulatoren des Cytokin-Signalwegs, wie p38-MAP-Kinase oder Syk-Kinase,
(x) Mitteln, die als Mukolytika oder Antitussiva klassifiziert werden können und
(y) Antibiotika.

## Revendications

1. Composé de formule (1) : 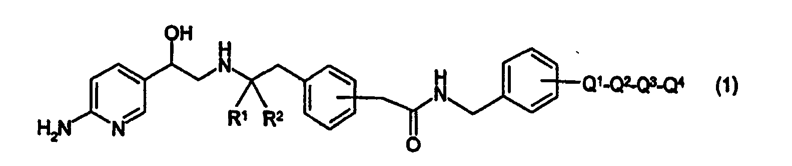 dans laquelle le groupe CH₂-C(=O)NH-benzyl-Q¹-Q²-Q³-Q⁴ est en position méta ou para, et
R¹ et R² sont choisis indépendamment entre H et un groupe alkyle en C₁ à C₄ ;
Q¹ représente un groupe (CH₂)ₙ dans lequel n représente le nombre entier 0 ou 1 ;
Q² représente un groupe choisi entre des groupes NH, -C(=O)NH-, -NHC(=O)-, -NH-C(O)-NH- et -SO₂NH- ;
Q³ représente une liaison simple, un groupe alkylène en C₁ à C₄ facultativement substitué avec un substituant OH ou un groupe (CH₂)ₘ-O-(CH₂)ₚ dans lequel m et p représentent des nombres entiers choisis indépendamment entre 1, 2 et 3 ;
Q⁴ est choisi entre des groupes 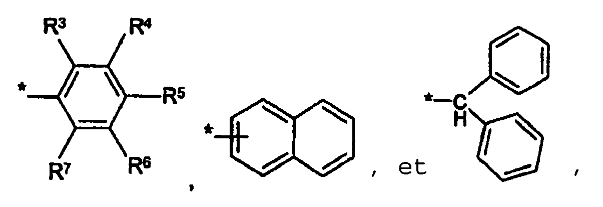 dans lesquels * représente le point de fixation à Q³ et R³, R⁴, R⁵, R⁶ et R⁷ sont choisis indépendamment entre H, des groupes phénoxy, alkyle en C₁ à C₄, OR⁹, SR⁹, halogéno, CF₃, OCF₃, COOR⁹, SO₂NR⁸R⁹ , CONR⁸R⁹, NHR⁸R⁹, NHCOR⁹, CH₂-NHC(=O)NH-R⁹, sous réserve qu'au moins deux des groupes R³ à R⁷ représentent H ;
dans lesquels R⁸ est choisi entre H et un groupe alkyle en C₁ à C₄ et R⁹ est choisi entre H, un groupe alkyle en C₁ à C₄, un groupe benzyle facultativement substitué avec 1, 2, 3 ou 4 substituants alkoxy en C₁ à C₄, et un groupe 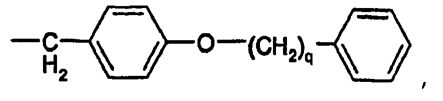 dans lequel q représente un nombre entier choisi entre 0, 1, 2 et 3 ;
ou, si cela est approprié, ses sels et/ou isomères, formes tautomères, produits de solvatation ou variantes isotopiques pharmaceutiquement acceptables.

2. Composé suivant la revendication 1, dans lequel R¹ représente H et R² représente un groupe CH₃.

3. Composé suivant la revendication 1, dans lequel R¹ représente un groupe CH₃ et R² représente un groupe CH₃.

4. Composé suivant l'une quelconque des revendications 1 à 3, dans lequel n est égal à 0 et Q² représente un groupe -C(=O)NH- ou SO₂NH-.

5. Composé suivant l'une quelconque des revendications 1 à 3, dans lequel n est égal à 1 et Q² représente un groupe -NH-C(=O)- ou -NH-C(O)-NH-.

6. Composé suivant l'une quelconque des revendications 1 à 5, dans lequel Q³ est choisi entre une liaison simple, des groupes CH₂, CH(-CH₃)CH₂OH-, CH(CH₃)-, -CH(CH₂OH)CH₂-, -(CH₂)₂-, -(CH₂)₂-OCH₂- et -(CH₂)₂-O-(CH₂)₃-.

7. Composé suivant l'une quelconque des revendications 1 à 6, dans lequel Q⁴, est choisi entre des groupes 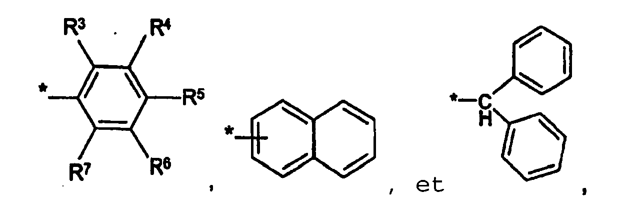 dans lesquels * représente le point de fixation à Q³ et R³, R⁴, R⁵, R⁶ et R⁷ sont choisis indépendamment entre H,
- un groupe OR⁹, sous réserve qu'au moins 2 des groupes R³ à R⁷ représentent H, ou
- un groupe SO₂NR⁸R⁹ ou CH₂-NHC(=O)NH-R⁹, sous réserve qu'au moins 4 des groupes R³ à R⁷ représentent H,
dans lesquels R⁸ est choisi entre H et un groupe alkyle en C₁ à C₄ et R⁹ est choisi entre H, un groupe alkyle en C₁ à C₄, un groupe benzyle facultativement substitué avec 1, 2, 3 ou 4 groupes alkoxy en C₁ à C₄, et un groupe 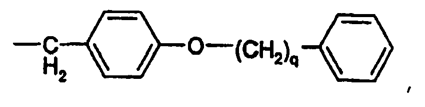 dans lequel q représente un nombre entier choisi entre 0, 1, 2 et 3.

8. Composé suivant l'une quelconque des revendications 1 à 6, dans lequel Q⁴ est choisi entre un groupe 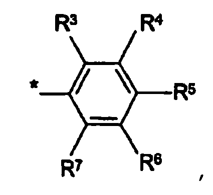 dans lequel * représente le point de fixation à Q³ et R³, R⁴, R⁵ R⁶ et R⁷sont choisis indépendamment entre H,
- un groupe OR⁹ sous réserve qu'au moins 2 des groupes R³ à R⁷ représentent H, ou
- un groupe SO₂NR⁸R⁹ ou CH₂-NHC(=O)NH-R⁹, sous réserve qu'au moins 4 des groupes R³ à R⁷ représentent H,
dans lesquels R⁹ est choisi entre H et un groupe alkyle en C₁ à C₄ et R⁹ est choisi entre H, un groupe alkyle en C₁ à C₄, un groupe benzyle facultativement substitué avec 1, 2, 3 ou 4 groupes alkoxy en C₁ à C₄, et un groupe 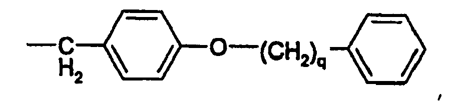 dans lequel q représente un nombre entier choisi entre 0, 1, 2 et 3.

9. Composé suivant la revendication 1, ledit composé répondant à la formule (1a) 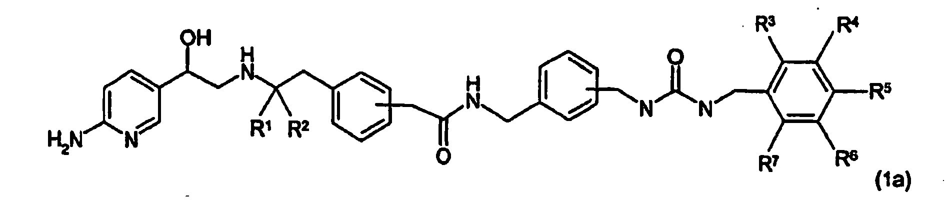 dans laquelle les groupes CH₂-C(=O)NH-benzyle et CH₂-NHC(=O)NH-benzyl-Q¹-Q²-Q³-Q⁴ sont indépendamment en position para ou méta, et
dans laquelle R³, R⁴ R⁵ R⁶ et R⁷ sont choisis indépendamment entre H, des groupes alkyle en C₁ à C₄, OR⁹, phénoxy, SR⁹, halogène, CF₃, OCF₃, COOR⁹ , SO₂NR⁸R⁹, CONR⁸R⁹, NHR⁹R¹⁰ et NHCOR⁹, sous réserve qu'au moins 2 des groupes R³ à R⁷ représentent H ;
dans laquelle R⁸ est choisi entre H et un groupe alkyle en C₁ à C₄ et R⁹ est choisi entre H, un groupe alkyle en C₁ à C₄ et un groupe benzyle facultativement substitué avec 1, 2, 3 ou 4 groupes alkoxy en C₁ à C₄.

10. Composé suivant la revendication 9, dans lequel R¹ représente un groupe CH₃ et R² représente H, et
R³ R⁴ ,R⁵, R⁶ et R⁷ sont choisis indépendamment entre H,
- un groupe OR⁹, phénoxy, sous réserve qu'au moins 2 des groupes R³ à R⁷ représentent H, ou
- un groupe SO₂NR⁸R⁹ , sous réserve qu'au moins 4 des groupes R³ à R⁷ représentent H,
dans lesquels R⁸ est choisi entre H et un groupe alkyle en C₁ à C₄ et R⁹ est choisi entre H, un groupe alkyle en C₁ à C₄, phényle, benzyle facultativement substitué avec 1, 2, 3 ou 4. groupes alkoxy en C₁ à C₄.

11. Composé suivant la revendication 9, dans lequel R¹ représente un groupe CH₃ et R² représente H, et
R³, R⁴ , R⁵ , R⁶ et R⁷ sont choisis indépendamment entre H, un groupe phénoxy et un groupe OR⁹, sous réserve qu'au moins 2 des groupes R³ à R⁷ représentent H, dans lequel R⁹ est choisi entre H et un groupe alkyle en C₁ à C₄.

12. Stéréoisomère (*R,R*) d'un composé suivant l'une quelconque des revendications 1 à 11, dans lequel R¹ représente un atome d'hydrogène et R² représente un groupe alkyle en C₁ à C₄.

13. Composé suivant l'une quelconque des revendications 1 à 12, dans lequel le groupe CH₂-C(=O)NH-benzyl-Q¹-Q²-Q³-Q⁴ est en position méta.

14. Composé suivant la revendication 1, choisi dans le groupe consistant en
4-{[2-(3-{2-[(2*R*)-2-(6-amino-pyridine-3-yl)-2-hydroxy-éthylamino]-propyl}-phényl)-acétylamino]-méthyl)-*N*-(3,4-diméthoxy-benzyl)-benzamide,
4-{[2-(3-{2-[(2*R*)-2-(6-amino-pyridine-3-yl)-2-hydroxy-éthylamino]-propyl}-phényl)-acétylamino]-méthyl}-*N*-(4-sulfamoyl-benzyl)-benzamide,
4-{[2-(3-{2-[(2*R*)-2-(6-amino-pyridine-3-yl)-2-hydroxy-éthylamino]-propyl}-phényl)-acétylamino]-méthyl}-*N*-(2-éthoxy-benzyl)-benzamide,
4-{[2-(3-{2-[(2*R*)-2-(6-amino-pyridine-3-yl)-2-hydroxy-éthylamino]-propyl}-phényl)-acétylamino]-méthyl}-*N*-[(1*R*)-1-(4-méthoxy-phényl)-éthyl]-benzamide,
4-{[2-(3-{2-[(2*R*)-2-(6-amino-pyridine-3-yl)-2-hydroxy-éthylamino]-propyl}-phényl)-acétylamino]-méthyl}-*N*-((1*R*)-1-hydroxyméthyl-2-phényl-éthyl)-benzamide,
4-{[2-(3-{2-[(2*R*)-2-(6-amino-pyridine-3-yl)-2-hydroxy-éthylamino]-propyl}-phényl)-acétylamino]-méthyl}-*N-*((1*R*,2*S*)-2-hydroxy-1-méthyl-2-phényl-éthyl)-benzamide,
4-{[2-(3-{2-[(2*R*)-2-(6-amino-pyridine-3-yl)-2-hydroxy-éthylamino]-propyl}-phényl)-acétylamino]-méthyl}-*N-*((1*S*,2*R*)-2-hydroxy-1-méthyl-2-phényl-éthyl)-benzamide,
4-{[2-(3-{2-[(2*R*)-2-(6-amino-pyridine-3-yl)-2-hydroxy-éthylamino]-propyl}-phényl)-acétylamino]-méthyl}-*N*-[2-(3-méthoxy-phényl)-éthyl]-benzamide,
4-{[2-(3-{(2*R*)-2-[(2*R*)-2-(6-amino-pyridine-3-yl)-2-hydroxy-éthylamino]-propyl}-phényl)-acétylamino]-méthyl}-*N*-(3,4-diméthoxy-benzyl)-benzamide,
4-{[2-(3-{(2*R*)-2-[(2*R*)-2-(6-amino-pyridine-3-yl)-2-hydroxy-éthylamino]-propyl}-phényl)-acétylamino]-méthyl}-*N*-(2-éthoxy-benzyl)-benzamide,
4-{[2-(3-{(2*R*)-2-[(2*R*)-2-(6-amino-pyridine-3-yl)-2-hydroxy-éthylamino-propyl}-phényl)-acétylamino]-méthyl}-*N*-(4-hydroxy-3-méthoxy-benzyl)-benzamide,
4-{[2-(3-{(2*R*)-2-[(2*R*)-2-(6-amino-pyridine-3-yl)-2-hydroxy-éthylamino]-propyl}-phényl)-acétylamino]-méthyl}-*N*-(sulfamoyl-benzyl)-benzamide,
4-{[2-(3-{(2*R*)-2-[(2*R*)-2-(6-amino-pyridine-3-yl)-2-hydroxy-éthylamino]-propyl}-phényl)-acétylamino]-méthyl}-*N*-((1*R*, 2*S*)-2-hydroxy-1-méthyl-2-phényl-éthyl)-benzamide,
N-(4-{[2-(3-{(2*R*)-2-[(2*R*)-2-(6-amino-pyridine-3-yl)-2-hydroxy-éthylamino]-propyl}-phényl)-acétylamino]-méthyl}-benzyl)-2,2-diphényl-acétamide,
2-(3-{(2*R*)-2-[(2*R*)-2-(6-amino-pyridine-3-yl)-2-hydroxy-éthylamino]-propyl}-phényl)-*N*-{4-[(benzhydryl-amino)-méthyl]-benzyl}-acétamide,
4-{[2-(3-{(2*R*)-2-[(2*R*)-2-(6-amino-pyridine-3-yl)-2-hydroxy-éthylamino]-propyl}-phényl)-acétylamino]-méthyl}-*N*-(2-benzyloxy-éthyl)-benzamide,
4-{[2-(3-{(2*R*)-2-[(2*R*)-2-(6-amino-pyridine-3-yl)-2-hydroxy-éthylamino]-propyl}-phényl)-acétylamino]-méthyl}-*N*-[2-(3-phényl-propoxy)-éthyl]-benzamide,
4-{[2-(3-{(2*R*)-2-[(2*R*)-2-(6-amino-pyridine-3-yl)-2-hydroxy-éthylamino]-propyl}-phényl)-acétylamino]-méthyl}-*N*-[2-(naphtalène-1-ylméthoxy)-éthyl]-benzamide,
4-{[2-(3-{(2*R*)-2-[(2*R*)-2-(6-amino-pyridine-3-yl)-2-hydroxy-éthylamino]-propyl}-phényl)-acétylamino]-méthyl}-*N*-(3-benzylsulfamoyl-benzyl)-benzamide,
4-{[2-(3-{(2*R*)-2-[(2*R*)-2-(6-amino-pyridine-3-yl)-2-hydroxy-éthylamino]-propyl}-phényl)-acétylamino]-méthyl}-*N*-(4-méthylsulfamoyl-benzyl)-benzamide,
4-{[2-(3-{(2*R*)-2-[(2*R*)-2-(6-amino-pyridine-3-yl)-2-hydroxy-éthylamino]-propyl}-phényl)-acétylamino]-méthyl}-*N*-(4-éthylsulfamoyl-benzyl)-benzamide,
4-{[2-(3-{(2*R*)-2-[(2*R*)-2-(6-amino-pyridine-3-yl)-2-hydroxy-éthylamino]-propyl}-phényl)-acétylamino]-méthyl}-*N*-(3-benzyl-sulfamoyl-benzyl)-benzamide,
4-{[2-(3-{(2*R*)-2-[(2*R*)-2-(6-amino-pyridine-3-yl)-2-hydroxy-éthylamino]-propyl}-phényl)-acétylamino]-méthyl}-*N*-(3-méthyl-sulfamoyl-benzyl)-benzamide,
4-{[2-(3-{(2*R*)-2-[(2*R*)-2-(6-amino-pyridine-3-yl)-2-hydroxy-éthylamino]-propyl}-phényl)-acétylamino]-méthyl}-*N*-(3-éthyl-sulfamoyl-benzyl)-benzamide,
chlorhydrate de 2-(3-{(2*R*)-2-[(2*R*)-2-(6-amino-pyridine-3-yl)-2-hydroxy-éthylamino]-propyl}-phényl)-*N*-(4-benzylsulfamoyl-benzyl)-acétamide,
chlorhydrate de 2-(3-{(2*R*)-2-[(2*R*)-2-(6-amino-pyridine-3-yl)-2-hydroxy-éthylamino]-propyl}-phényl)-*N*-(3-benzylsulfamoyl-benzyl)-acétamide,
4-{[2-(4-{2-[(2*R*)-2-(6-amino-pyridine-3-yl)-2-hydroxy-éthylamino]-propyl}-phényl)-acétylamino]-méthyl}-*N*-(3,4-diméthoxy-benzyl)-benzamide,
4-{[2-(4-{2-[(2*R*)-2-(6-amino-pyridine-3-yl)-2-hydroxy-éthylamino]-propyl}-phényl)-acétylamino]-méthyl*-N-*(2-éthoxy-benzyl)-benzamide,
4-{[2-(4-{2-[(2*R*)-2-(6-amino-pyridine-3-yl)-2-hydroxy-éthylamino]-propyl}-phényl)-acétylamino]-méthyl}-*N*-[(1*R*)-1-(4-méthoxy-phényl)-éthyl]-benzamide,
4-{[2-(4-{2-[(2*R*)-2-(6-amino-pyridine-3-yl)-2-hydroxy-éthylamino]-propyl}-phényl)-acétylamino]-méthyl}-*N*-((1*R*)-1-benzyl-2-hydroxy-éthyl)-benzamide,
4-{[2-(4-{2-[(2*R*)-2-(6-amino-pyridine-3-yl)-2-hydroxy-éthylamino]-propyl}-phényl)-acétylamino]-méthyl}-*N*-((1*R*, 2*S*)-2-hydroxy-1-méthyl-2-phényl-éthyl)-benzamide,
4-{[2-(4-{2-[(2*R*)-2-(6-amino-pyridine-3-yl)-2-hydroxy-éthylamino]-propyl}-phényl)-acétylamino]-méthyl}-*N-*((1*S,*2*R*)-2-hydroxy-1-méthyl-2-phényl-éthyl)-benzamide,
4-{[2-(4-{2-[(2*R*)-2-(6-amino-pyridine-3-yl)-2-hydroxy-éthylamino]-propyl}-phényl)-acétylamino]-méthyl}-*N*-[2-(3-méthoxy-phényl)-éthyl]-benzamide,
4-{[2-(3-{(2*R*)-2-[(2*R*)-2-(6-amino-pyridine-3-yl)-2-hydroxy-éthylamino]-propyl}-phényl)-acétylamino]-méthyl}-*N-*[4-(3-benzyl-uréidométhyl)-benzyl]-benzamide,
4-{[2-(3-{(2*R*)-2-[(2*R*)-2-(6-amino-pyridine-3-yl)-2-hydroxy-éthylamino]-propyl}-phényl)-acétylamino]-méthyl}-*N*-{4-[3-(3,4-diméthoxy-benzyl)-uréidométhyl]-benzyl}-benzamide,
4-{[2-(3-{(2*R*)-2-[(2*R*)-2-(6-amino-pyridine-3-yl)-2-hydroxy-éthylamino]-propyl}-phényl)-acétylamino]-méthyl}-*N*-{4-[3-(4-phénoxy-benzyl)-uréidométhyl]-benzyl}-benzamide,
4-{[2-(3-{(2*R*)-2-[(2*R*)-2-(6-amino-pyridine-3-yl)-2-hydroxy-éthylamino]-propyl}-phényl)-acétylamino]-méthyl}-*N*-{4-[3-(4-benzyloxy-benzyl)-uréidométhyl]-benzyl}-benzamide,
2-(3-{(2*R*)-2-[(2*R*)-2-(6-amino-pyridine-3-yl)-2-hydroxy-éthylamino]-propyl}-phényl)-*N*-{4-[3-(3-phénoxy-benzyl)-uréidométhyl]-benzyl}-acétamide,
2-(3-{(2*R*)-2-[(2*R*)-2-(6-amino-pyridine-3-yl)-2-hydroxy-éthylamino]-propyl}-phényl)-*N*-{4-[3-(3,4-diméthoxy-benzyl)-uréidométhyl]-benzyl}-acétamide, et
2-(3-{(2*R*)-2-[(2*R*)-2-(6-amino-pyridine-3-yl)-2-hydroxy-éthylamino]-propyl}-phényl)-*N*-{4-[3-(4-phénoxy-benzyl)-uréidométhyl]-benzyl}-acétamide.

15. Procédé pour la préparation d'un composé de formule (1) suivant l'une quelconque des revendications 1 à 14 ou d'un de ses sels ou d'une de ses formes dérivées pharmaceutiquement acceptables, **caractérisé en ce qu'**il comprend les étapes
(a) de couplage d'un acide de formule (3) : 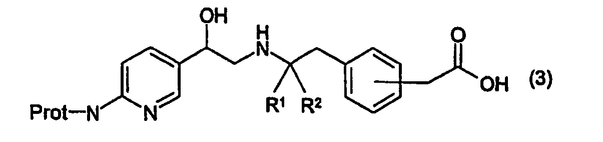 dans laquelle Prot représente un groupe protecteur, R¹ et R² sont tels que définis dans la revendication 1, avec une amine de formule (4) : 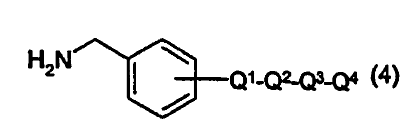 dans laquelle Q¹, Q², Q³ et Q⁴ sont tels que définis dans la revendication 1 ;
(b) d'élimination du groupe protecteur Prot du composé de formule (2) : 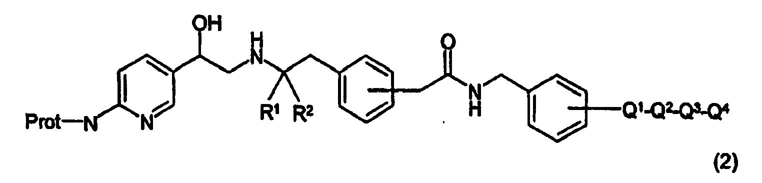
(c) d'isolement du composé de formule (1).

16. Composition pharmaceutique comprenant un composé de formule (1) suivant l'une quelconque des revendications 1 à 14 ou un de ses sels ou une de ses formes dérivées pharmaceutiquement acceptables, conjointement avec des excipients et/ou additifs usuels pharmaceutiquement doués d'innocuité.

17. Composé de formule (1) suivant l'une quelconque des revendications 1 à 14 ou un de ses sels ou une de ses formes dérivées pharmaceutiquement acceptables ou une composition le contenant, destiné à être utilisé comme médicament.

18. Composé de formule (1) suivant l'une quelconque des revendications 1 à 14 ou un de ses sels ou une de ses formes dérivées pharmaceutiquement acceptables ou une composition le contenant, destiné à être utilisé dans le traitement de maladies, de troubles et d'affections dans lesquels le récepteur β2 est impliqué.

19. Composé de formule (1) suivant l'une quelconque des revendications 1 à 14 ou un de ses sels ou une de ses formes dérivées pharmaceutiquement acceptables ou une composition le contenant, destiné à être utilisé dans le traitement de maladies, de troubles et d'affections choisis dans le groupe consistant en :
• l'asthme de n'importe quel type, étiologie ou pathogenèse, en particulier l'asthme qui est un membre choisi dans le groupe consistant en l'asthme atopique, l'asthme non atopique, l'asthme allergique, l'asthme bronchique atopique à médiation par IgE, l'asthme bronchique, l'asthme essentiel, l'asthme vrai, l'asthme intrinsèque provoqué par des perturbations physiopathologiques, l'asthme extrinsèque provoqué par des facteurs ambiants, l'asthme essentiel de cause inconnue ou inapparente, l'asthme non atopique, l'asthme bronchitique, l'asthme emphysémateux, l'asthme induit par l'effort, l'asthme induit par un allergène, l'asthme induit par l'air froid, l'asthme professionnel, l'asthme infectieux provoqué par une infection par des bactéries, des champignons, des protozoaires ou des virus, l'asthme non allergique, l'asthme naissant, le syndrome de respiration sifflante du nourrisson et la bronchiolite,
• la bronchoconstriction chronique ou aiguë, la bronchite chronique, une obstruction des petites voies aériennes et l'emphysème,
• des maladies obstructives ou inflammatoires des voies aériennes de n'importe quel type, étiologie ou pathogenèse, en particulier une maladie obstructive ou inflammatoire des voies aériennes qui est un membre choisi dans le groupe consistant en la pneumonie éosinophile chronique, la maladie pulmonaire obstructive chronique (COPD), la COPD qui comprend la bronchite chronique, l'emphysème pulmonaire ou la dyspnée associée ou non associée à la COPD, la COPD qui est **caractérisée par** une obstruction progressive irréversible des voies aériennes, le syndrome de détresse respiratoire de l'adulte (ARDS), l'exacerbation de l'hyperréactivité des voies aériennes consécutive à une autre pharmacothérapie et une maladie des voies aériennes qui est associée à l'hypertension pulmonaire,
• la bronchite de n'importe quel type, étiologie ou pathogenèse, en particulier la bronchite qui est un membre choisi dans le groupe consistant en la bronchite aiguë, la bronchite laryngotrachéale aiguë, la bronchite arachidique, la bronchite catarrhale, la bronchite croupeuse, la bronchite sèche, la bronchite asthmatique infectieuse, la bronchite productive, la bronchite staphylococcique ou streptococcique et la bronchite vésiculaire,
• la bronchiectasie de n'importe quel type, étiologie ou pathogenèse, en particulier la bronchiectasie qui est un membre choisi dans le groupe consistant en la bronchiectasie cylindrique, la bronchiectasie sacciforme, la bronchiectasie fusiforme, la bronchiectasie capillaire, la bronchiectasie kystique, la bronchiectasie sèche et la bronchiectasie folliculaire.

20. Utilisation d'un composé de formule (1) suivant l'une quelconque des revendications 1 à 14 ou d'un de ses sels ou d'une de ses formes dérivées pharmaceutiquement acceptables ou d'une composition le contenant, pour la production d'un médicament ayant une activité agoniste de β2.

21. Utilisation d'un composé de formule (1) suivant l'une quelconque des revendications 1 à 14 ou d'un de ses sels ou produits de solvatation pharmaceutiquement acceptables ou d'une composition le contenant, pour la production d'un médicament destiné au traitement de maladies, de troubles et d'affections choisis dans le groupe tel que décrit dans la revendication 19.

22. Association d'un composé suivant l'une quelconque des revendications 1 à 14 avec un ou plusieurs autres agents thérapeutiques choisis entre :
(a) des inhibiteurs de 5-lipoxygénase (5-LO) ou des antagonistes de la protéine activatrice de 5-lipoxygénase (FLAP),
(b) des antagonistes de leucotriène (LTRA), comprenant des antagonistes de LTB₄, LTC₄, LTD₄ et LTE₄,
(c) des antagonistes du récepteur d'histamine, comprenant des antagonistes de H1 et H3,
(d) des agents sympathomimétiques vasoconstricteurs agonistes des adrénorécepteurs α₁ et α₂ destinés à être utilisés comme décongestionnants,
(e) des antagonistes des récepteurs muscariniques M3 ou agents anticholinergiques,
(f) des inhibiteurs de PDE, par exemple des inhibiteurs de PDE3, PDE4 et PDE5,
(g) la théophylline,
(h) le cromoglycate de sodium,
(i) des inhibiteurs de COX, consistant en inhibiteurs non sélectifs et sélectifs de COX-1 ou COX-2 (MAINS),
(j) des glucocorticoïdes oraux et glucocorticoïdes inhalés,
(k) des anticorps monoclonaux dirigés contre des entités inflammatoires endogènes,
(l) des agents anti-facteur de nécrose de tumeur (anti-TNF-α),
(m) des inhibiteurs de molécules d'adhésion, comprenant des antagonistes de VLA-4,
(n) des antagonistes des récepteurs de kinines B₁ et B₂,
(o) des agents immunosuppresseurs,
(p) des inhibiteurs de métalloprotéases de matrice (MMP),
(q) des antagonistes des récepteurs de tachykinines NK₁, NK₂ et NK₃,
(r) des inhibiteurs d'élastase,
(s) des agonistes du récepteur d'adénosine A2a,
(t) des inhibiteurs d'urokinase,
(u) des composés qui agissent sur les récepteurs de dopamine, par exemple des agonistes D2,
(v) des modulateurs de la voie de NFκβ, par exemple des inhibiteurs de IKK,
(w) des modulateurs de voies de transmission de cytokines, telles que la kinase MAP p38 ou la kinase syk,
(x) des agents qui peuvent être classés dans les agents mucolytiques ou les agents antitussifs, et
(y) des antibiotiques.
